(19) Europäisches Patentamt / European Patent Office / Office européen des brevets

(11) EP 2 848 697 B1

(12) **EUROPEAN PATENT SPECIFICATION**

(45) Date of publication and mention of the grant of the patent:
**03.01.2018 Bulletin 2018/01**

(21) Application number: **13787593.6**

(22) Date of filing: **30.04.2013**

(51) Int Cl.:
***C12Q 1/68*** *(2018.01)* ***C12N 15/09*** *(2006.01)*

(86) International application number:
**PCT/JP2013/062650**

(87) International publication number:
**WO 2013/168644 (14.11.2013 Gazette 2013/46)**

(54) **METHOD FOR PREDICTING PROGNOSIS OF RENAL CELL CARCINOMA**

VERFAHREN ZUR VORHERSAGE EINER PROGNOSE EINES NIERENZELLKARZINOMS

PROCÉDÉ POUR LA PRÉDICTION DU PRONOSTIC D'UN NÉPHROCARCINOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.05.2012 US 201261646044 P**

(43) Date of publication of application:
**18.03.2015 Bulletin 2015/12**

(73) Proprietor: **National Cancer Center**
**Tokyo 1040045 (JP)**

(72) Inventors:
- **KANAI, Yae**
  **Tokyo 104-0045 (JP)**
- **ARAI, Eri**
  **Tokyo 104-0045 (JP)**
- **TIAN, Ying**
  **Tokyo 104-0045 (JP)**

(74) Representative: **J A Kemp**
**14 South Square**
**Gray's Inn**
**London WC1R 5JJ (GB)**

(56) References cited:
**WO-A1-2011/032721**
**WO-A1-2011/148983**
**WO-A1-2012/031329**
**JP-A- 2008 535 853**
**JP-A- 2009 519 039**
**JP-A- 2010 063 413**
**JP-A- 2011 525 114**

- **SARAH DEDEURWAERDER ET AL: "Evaluation of the Infinium Methylation 450K technology", EPIGENOMICS, vol. 3, no. 6, 1 December 2011 (2011-12-01), pages 771-784, XP055259694, United Kingdom ISSN: 1750-1911, DOI: 10.2217/epi.11.105**
- **M R MORRIS ET AL: "Identification of candidate tumour suppressor genes frequently methylated in renal cell carcinoma", ONCOGENE, vol. 29, no. 14, 15 February 2010 (2010-02-15), pages 2104-2117, XP055100662, GB ISSN: 0950-9232, DOI: 10.1038/onc.2009.493**
- **E. ARAI ET AL: "Genetic Clustering of Clear Cell Renal Cell Carcinoma Based on Array-Comparative Genomic Hybridization: Its Association with DNA Methylation Alteration and Patient Outcome", CLINICAL CANCER RESEARCH, vol. 14, no. 17, 1 September 2008 (2008-09-01), pages 5531-5539, XP055113052, ISSN: 1078-0432, DOI: 10.1158/1078-0432.CCR-08-0443**
- **ERI ARAI ET AL: "Regional DNA hypermethylation and DNA methyltransferase (DNMT) 1 protein overexpression in both renal tumors and corresponding nontumorous renal tissues", INTERNATIONAL JOURNAL OF CANCER, vol. 119, no. 2, 1 January 2006 (2006-01-01), pages 288-296, XP055259483, US ISSN: 0020-7136, DOI: 10.1002/ijc.21807**
- **ARAI, E. ET AL.: 'Single-CpG-resolution methylome analysis identifies clinicopathologically aggressive CpG island methylator phenotype clear cell renal cell carcinomas.' CARCINOGENESIS vol. 33, no. 8, August 2012, pages 1487 - 1493, XP055177040**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KAWAI, Y. ET AL.: 'Methylation level of the RASSF1A promoter is an independent prognostic factor for clear-cell renal cell carcinoma.' ANNALS OF ONCOLOGY vol. 21, no. 8, August 2010, pages 1612 - 1617, XP055177042
- VAN VLODROP, I.J.H. ET AL.: 'Prognostic significance of Gremlinl (GREM1) promoter CpG island hypermethylation in clear cell renal cell carcinoma.' THE AMERICAN JOURNAL OF PATHOLOGY vol. 176, no. 2, February 2010, pages 575 - 584, XP055177044
- ARAI, E. ET AL.: 'Genetic and epigenetic alterations during renal carcinogenesis.' INTERNATIONAL JOURNAL OF CLINICAL AND EXPERIMENTAL PATHOLOGY vol. 4, no. 1, 13 December 2010, pages 58 - 73, XP055177047

## Description

[Technical Field]

The present invention relates to a method for detecting an unfavorable prognostic risk of renal cell carcinoma, the method comprising detecting a DNA methylation level. Moreover, the present invention relates to an oligonucleotide used in the method.

[Background Art]

Renal cell carcinoma (RCC) often occurs in the working population at the maturity stage. While there are many case groups who are curable by nephrectomy, there are also apparently case groups who develop a distant metastasis rapidly. The two greatly differ in clinical course. Further, there is known a case for which an immunotherapy, molecularly targeted therapeutic drug, or the like is effective even if a metastasis occurs. Cases who are highly likely to have a recurrence should be subjected to a close follow-up observation to diagnose a recurrence at an early stage, and if an additional after-treatment is performed, there is a possibility that the prognosis can be improved. However, cases are experienced, who belong to histopathologically low grade and the most common histological type, clear cell RCC, and rapidly develop a distant metastasis . It is difficult to predict a prognosis utilizing existing clinicopathological parameters and the like.

It is well known that clear cell RCCs are characterized by inactivation of the VHL tumor-suppressor gene. Moreover, systematic resequencing and exome analysis of RCCs are performed by The Cancer Genome Atlas, The Cancer Genome Project, and other international efforts. Then, such efforts have revealed that renal carcinogenesis involves inactivation of histone-modifying genes, such as SETD2, a histone H3 lysine 36 methyltransferase; JARID1C (KDM5C), a histone H3 lysine 4 demethylase; UTX (KDM6A), a histone H3 lysine 27 demethylase; and PBRM1, a SWI/SNF chromatin remodeling complex (NPLs 1 to 3). Furthermore, non-synonymous mutations of the NF2 gene and truncating mutations of the MLL2 gene in RCC have also been reported (NPL 1). However, such gene mutations cannot fully explain the aforementioned difference in RCC clinical course and the like (clinicopathological diversity).

Not only genetic but also epigenetic events are observed during carcinogenesis, and these two events reflect the clinicopathological diversity in various tissues in association with each other. In addition, DNA methylation alternation is believed to be one of major epigenetic changes in human cancers. In renal cell carcinoma the methylation of BNC1, COL14A1 and SFRP1 was each associated with significantly poorer patient survival (NPL 14). In fact, on the basis of the analyses of RCCs by methylation-specific PCR (MSP), COBRA (combined bisulfite restriction enzyme analysis), and bacterial artificial chromosome (BAC) array-based methylated CpG island amplification (BAMCA), the present inventors have demonstrated that a non-cancerous renal cortex tissue obtained from RCC patients is already at the precancerous stage associated with DNA methylation alterations (PLT 1 and NPLs 4 to 7). Further, the inventors have revealed by the genome-wide analysis using BAMCA that the DNA methylation alternation status in a non-cancerous renal cortex tissue at the precancerous stage is inherited by the corresponding RCC in the same patient, and successfully developed a method for predicting a prognosis of an RCC case (PLT 1 and NPL 6).

However, the technique of evaluating a DNA methylation status using BAMCA is complex. In addition, in predicting a prognosis of an RCC case using such BAMCA, the region of chromosomes that can be covered by BAC clones was quite limited at the time of the invention. Hence, a methylated CpG site having a truly high diagnostic ability has not been identified.

Moreover, regarding the DNA methylation in cancers, the existence of a cancer phenotype, CpG island methylator phenotype (CIMP), showing that DNA hypermethylation accumulates on CpG islands in a manner correlated with clinicopathological parameters of cases has been revealed in colorectal cancer, stomach cancer, and the like (NPLs 8 to 11).

Nevertheless, regarding renal cell carcinomas, it has been considered that an association between the CIMP-positive phenotype and renal cell carcinomas has not been revealed yet (NPL 12). In fact, on the basis of a finding that the distribution of the number of methylated CpGs in individual tumors was shown to differ from the expected Poisson distribution, a possibility has suggested that a subset of renal cell carcinomas exhibit CIMP. However, the existence of CIMP-positive renal cell carcinomas in kidneys has not been verified, and no distinct CpG site that could become a hallmark for CIMP has been identified (NPL 13) .

From such circumstances, desired are methods capable of indicating, in renal cell carcinomas also, the existence of a phenotype (CIMP) showing that DNA methylation accumulates on CpG islands in a manner strongly correlated with clinicopathological RCC parameters, identifying a CpG site serving as a CIMP marker, and predicting a prognosis of RCC easily with quite high sensitivity and specificity. However, such methods are not put into practical use at present.

[Citation List]

[Patent Literature]

[PLT 1] Japanese Unexamined Patent Application Publication No. 2010-63413

[Non Patent Literature]

[NPL 1] Dalgliesh, G. L. et al., Nature, 2010, vol. 463, pp. 360 to 363
[NPL 2] van Haaften, G. et al., Nat. Genet., 2009, vol. 41, pp. 521 to 523
[NPL 3] Varela, I. et al., Nature, 2011, vol. 469, pp. 539 to 542
[NPL 4] Arai, E. et al., Clin. Cancer Res., 2008, vol. 14, pp. 5531 to 5539
[NPL 5] Arai, E. et al., Int. J. Cancer, 2006, vol. 119, pp. 288 to 296
[NPL 6] Arai, E. et al., Carcinogenesis, 2009, vol. 3 0, pp. 214 to 221
[NPL 7] Arai, E. et al., Pathobiology, 2011, vol. 78, pp. 1 to 9
[NPL 8] Issa, J. P., Nat. Rev. Cancer, 2004, vol. 4, pp. 988 to 993
[NPL 9] Toyota, M. et al., Proc. Natl. Acad. Sci. USA, 1999, vol. 96, pp. 8681 to 8686
[NPL 10] Shen, L. et al., Proc. Natl. Acad. Sci. USA, 2007, vol. 104, pp. 18654 to 18659
[NPL 11] Toyota, M. et al., Cancer Res., 1999, vol. 5 9, pp. 5438 to 5442
[NPL 12] Morris, M. R. et al., Genome Med., 2010, 2 (9) : 59
[NPL 13] McRonald, F. E. et al., Mol. Cancer, 2009, 8: 31
[NPL 14] Morris, M.R. et al., Oncogene, 2010, 29:2104-2117

[Summary of Invention]

[Technical Problem]

An object is to provide a method for determining an unfavorable prognostic risk of renal cell carcinoma easily with quite high sensitivity and specificity.

[Solution to Problem]

In order to achieve the above object, the present inventors have performed a methylome analysis using a single CpG resolution Infinium array on 29 normal renal cortex tissue (C) samples, and 107 non-cancerous renal cortex tissue (N) samples and 109 tumor tissue (T) samples obtained from patients with clear cell renal cell carcinomas (clear cell RCCs). The result revealed that the DNA methylation level of the N samples was already altered at 4830 CpG sites in comparison with the C samples. Further, DNA methylation alternations occurred in the N samples, and 801 CpG sites where the alternations were inherited by and strengthened in the T samples were identified. An unsupervised hierarchical clustering analysis was performed based on the DNA methylation levels at the 801 CpG sites. As a result, it was found out that renal cell carcinomas was grouped into Cluster A (n=90) and Cluster B (n=14). Then, it was found out that clinicopathologically aggressive tumors were accumulated in this Cluster B, and also that the cancer-free survival rate (recurrence-free survival rate) and overall survival rate of patients belonging to this Cluster B were significantly lower than those of patients belonging to Cluster A. Specifically, it was revealed that renal cell carcinomas belonging to Cluster B were characterized by accumulation of DNA hypermethylation on CpG islands and were CpG island methylator phenotype (CIMP)-positive cancers.

Further, it was also found out for the first time that DNA hypermethylations at CpG sites of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2 genes were hallmarks of CIMP in renal cell carcinomas.

Note that none of the CpG sites of the 17 genes identified this time were included in the renal cell carcinoma-associated regions (70 BAC clones) having been identified as being effective in predicting a prognosis of renal cell carcinoma, by examining the presence or absence of the DNA methylation described in PLT 1 and NPL 6.

Moreover, it was also verified that it was possible to detect the hypermethylation status at the CpG sites of these 17 genes by methods other than the analysis using the Infinium array (a pyrosequencing method and a DNA methylation analysis method using a mass spectrometer) . These have led to the completion of the present invention. More specifically, the present invention is as follows.

<1> A method for detecting an unfavorable prognostic risk of renal cell carcinoma, the method comprising the following steps (a) to (c):

(a) a step of preparing a genomic DNA derived from a kidney tissue of a subject;
(b) a step of detecting a DNA methylation level of at least one CpG site of a gene selected from the gene group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2 in the genomic DNA prepared in the step (a); and
(c) a step of determining whether or not the subject is classified into an unfavorable prognosis group according to the DNA methylation level detected in the step (b).

<2> The method according to <1>, wherein the step (b) is a step of treating the genomic DNA prepared in the step (a) with bisulfite and detecting a DNA methylation level of the CpG site.
<3> An oligonucleotide according to any one of the following (a) and (b), which have a length of at least 12 bases, for use in the method according to any one of <1> and <2>:

(a) an oligonucleotide that is a pair of primers designed to flank at least one CpG site of a gene selected from the gene group; and
(b) an oligonucleotide that is any one of a primer and a probe capable of hybridizing to a nucleotide comprising at least one CpG site of a gene selected from the gene group.

[Advantageous Effects of Invention]

It is made possible to determine an unfavorable prognostic risk of renal cell carcinoma easily with quite high sensitivity and specificity.

[Brief Description of Drawings]

[Fig. 1] Fig. 1 shows micrographs for illustrating a histological difference between a non-cancerous renal cortex tissue (N) and a tumorous tissue (T) derived from a patient with clear cell renal cell carcinoma. Specifically, N consists mainly of proximal renal tubules. On the other hand, T shows alveolar structures. Moreover, the cytoplasm of tumor cells is filled with lipids and glycogen and surrounded by a distinct cell membrane. Further, the micrograph shows that the nuclei of the tumor cells tend to be round with finely granular, evenly distributed chromatins.
[Fig. 2] Fig. 2 is a graph for illustrating a correlation between the DNA methylation level ($\beta$ value) at a CpG site of a ZFP42 gene detected by an Infinium assay and the DNA methylation level detected by pyrosequencing.
[Fig. 3] Fig. 3 is a graph for illustrating a correlation between the DNA methylation level ($\beta$ value) at a CpG site of a ZFP154 gene detected by the Infinium assay and the DNA methylation level detected by pyrosequencing.
[Fig. 4] Fig. 4 is a graph for illustrating a correlation between the DNA methylation level ($\beta$ value) at a CpG site of a ZFF540 gene detected by the Infinium assay and the DNA methylation level detected by pyrosequencing.
[Fig. 5] Fig. 5 is a map for illustrating that unsupervised hierarchical clustering subclustered differences ($\Delta\beta_{T-N}$) of DNA methylation levels on 801 probes (CpG sites) between tumor tissues (T) and non-cancerous tissues (N) from 104 patients with clear cell renal cell carcinomas into Cluster A (n=90) and Cluster B (n=14). Note that the DNA methylation status at the 801 probes was altered at the precancerous stage, which was presumably involved in the renal carcinogenesis.
[Fig. 6] Fig. 6 is a graph for illustrating a change over time in a recurrence-free survival rate after surgery of patients with clear cell renal cell carcinomas (patients belonging to Cluster A and patients belonging to Cluster B) .
[Fig. 7] Fig. 7 is a graph for illustrating a change over time in an overall survival rate after the surgery of the patients with clear cell renal cell carcinomas (patients belonging to Cluster A and patients belonging to Cluster B) .
[Fig. 8] Fig. 8 is a graph for illustrating proportions of probes showing a difference in DNA methylation level (absolute value of $\Delta\beta_{T-N}$) by 0.1 or more between non-cancerous tissues (N samples) of patients with clear cell renal cell carcinomas and tumor tissues (T samples) of the patients, relative to all 26454 probes as the detection target of the Infinium assay. In the figure, the term "all cases" shows the result of all the analyzed patients with clear cell renal cell carcinomas, "A" shows that of patients with clear cell renal cell carcinomas belonging to Cluster A among the analyzed patients with clear cell renal cell carcinomas, and "B" shows that of patients with clear cell renal cell carcinomas belonging to Cluster B among the analyzed patients with clear cell renal cell carcinomas. A bar represents SD (standard deviation), and "NS" indicates that no significant difference is observed (the same applies to Figs. 9 to 12) .

[Fig. 9] Fig. 9 is a graph for illustrating proportions of probes showing a difference in DNA methylation level (absolute value of $\Delta\beta_{T-N}$) by 0.2 or more between the N samples and the T samples, relative to all the 26454 probes as the detection target of the Infinium assay.

[Fig. 10] Fig. 10 is a graph for illustrating proportions of probes showing a difference in DNA methylation level (absolute value of $\Delta\beta_{T-N}$) by 0.3 or more between the N samples and the T samples, relative to all the 26454 probes as the detection target of the Infinium assay.

[Fig. 11] Fig. 11 is a graph for illustrating proport ions of probes showing a difference in DNA methylation level (absolute value of $\Delta\beta_{T-N}$) by 0.4 or more between t he N samples and the T samples, relative to all the 264 54 probes as the detection target of the Infinium assay

[Fig. 12] Fig. 12 is a graph for illustrating proportions of probes showing a difference in DNA methylation level (absolute value of $\Delta\beta_{T-N}$) by 0 .5 or more between the N samples and the T samples, relative to all the 26454 probes as the detection target of the Infinium assay.

[Fig. 13] Fig. 13 shows scattergrams for illustrating the result of associating DNA methylation levels (β values) in renal cell carcinoma tissues (T samples) with those in non-cancerous renal tissues (N samples) from representative patients with clear cell renal cell carcinomas belonging to Cluster A (cases 1 to 4).

[Fig. 14] Fig. 14 shows scattergrams for illustrating the result of associating DNA methylation levels (β values) in renal cell carcinoma tissues (T samples) with those in non-cancerous renal tissues (N samples) from representative patients with clear cell renal cell carcinomas belonging to Cluster B (cases 5 to 8). In the figure, sections marked by circles each represent a distribution of probes for which DNA methylation levels were low in the N samples and for which the degree of DNA hypermethylation in the T samples relative to the corresponding N samples was prominent.

[Fig. 15] Fig. 15 is a representation for illustrating an association between the patients with clear cell renal cell carcinomas belonging to Cluster A or B and DNA methylation levels of 16 probes (16 CpG sites), shown in Table 14, serving as hallmarks of CpG island methylator phenotype (CIMP). In the figure, a section filled with black indicates that $\Delta\beta_{T-N}$ exceeds 0.4.

[Fig. 16] Fig. 16 is a graph for illustrating the result of performing random forest analysis using 869 probes on which DNA methylation levels ($\Delta\beta_{T-N}$) differed markedly between Clusters A and B (FDR [q=0.01]). In the figure, polygonal lines represent spam (3), out-of-bag (OOB), and non-spam (1) in this order from the top. The horizontal axis represents the number of trees, and the vertical axis represents prediction error (Error).

[Fig. 17] Fig. 17 is a plot graph for illustrating the result of performing random forest analysis using 869 probes on which DNA methylation levels ($\Delta\beta_{T-N}$) differed markedly between Clusters A and B (FDR [q=0.01]). In the figure, the horizontal axis represents the mean of Gini index (MeanDecreaseGini), and the vertical axis represents probes (CpG sites) used in the Infinium assay.

[Fig. 18] Fig. 18 is a graph for illustrating the result of analyzing by MassARRAY the DNA methylation level on a CpG island of a SLC13A5 gene in patients with clear cell renal cell carcinomas belonging to Cluster A or B. Note that, in the figure, SLC13A5_10 "CpG_40" is a CpG site (probe ID: cg22040627, position: 6617030 on chromosome 17 on NCBI database Genome Build 37) detected at a high DNA methylation level in Cluster B by the Infinium assay also.

[Fig. 19] Fig. 19 is a graph for illustrating the result of analyzing by MassARRAY the DNA methylation level on a CpG island of a RIMS4 gene in the patients with clear cell renal cell carcinomas belonging to Cluster A or B.

[Fig. 20] Fig. 20 is a graph for illustrating the result of analyzing by MassARRAY the DNA methylation level on a CpG island of a PCDHAC1 gene in the patients with clear cell renal cell carcinomas belonging to Cluster A or B.

[Fig. 21] Fig. 21 is a graph for illustrating the result of analyzing by MassARRAY the DNA methylation level on a CpG island of a ZNF540 gene in the patients with clear cell renal cell carcinomas belonging to Cluster A or B.

[Fig. 22] Fig. 22 is a graph for illustrating the result of analyzing by MassARRAY the DNA methylation level on a CpG island of a TRH gene in the patients with clear cell renal cell carcinomas belonging to Cluster A or B.

[Fig. 23] Fig. 23 is a graph for illustrating the result of analyzing by MassARRAY the DNA methylation level on a CpG island of a PRAC gene in the patients with clear cell renal cell carcinomas belonging to Cluster A or B.

[Fig. 24] Fig. 24 is a graph for illustrating the result of classifying patients with clear cell renal cell carcinomas into Cluster A or B according to the number of CpG sites satisfying a cutoff value (diagnostic threshold) . As to the cutoff value, see Tables 19 to 27. Moreover, the CpG sites used as the indicator in this classification are 23 CpG units having an AUC larger than 0.95 shown in Tables 19 to 27 (32 CpG sites).

[Description of Embodiments]

The present invention provides a method for detecting an unfavorable prognostic risk of renal cell carcinoma, the method comprising the following steps (a) to (c):

(a) a step of preparing a genomic DNA derived from a kidney tissue of a subject;
(b) a step of detecting a DNA methylation level of at least one CpG site of a gene selected from the gene group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2 in the genomic DNA prepared in the step (a); and
(c) a step of determining whether or not the subject is classified into an unfavorable prognosis group according to the DNA methylation level detected in the step (b).

In the present invention, the term "renal cell carcinoma" refers to a cancer originated from the renal tubular epithelial cells in the kidney. According to the pathological features, the cancer is classified into clear cell type, granular cell type, chromophobe type, spindle type, cyst-associated type, cyst-originating type, cystic type, or papillary type. Moreover, examples of the "subject" according to the present invention include patients who have been treated for renal cell carcinomas by nephrectomy or the like.

An example of the "unfavorable prognostic risk of renal cell carcinoma" according to the present invention includes a low survival rate in a prognosis (after nephrectomy or the like) of a subject. More specifically, the examples include a recurrence-free survival rate (cancer-free survival rate) of 50% or less after 500 days from the surgery as illustrated later in Fig. 6, and an overall survival rate of 70% or less after 1500 days from the surgery as illustrated later in Fig. 7.

In the present invention, the term "CpG site" means a site where cytosine (C) is linked to guanine (G) with a phosphodiester bond (p), and the term "DNA methylation" means a state where carbon at position 5 of cytosine is methylated at the CpG site. The term "DNA methylation level" means a ratio of the methylation at a particular CpG site to be detected, and can be expressed, for example, as a ratio of the number of methylated cytosines relative to the number of all cytosines (methylated cytosines and unmethylated cytosines) at a particular CpG site to be detected.

The "preparation of a genomic DNA derived from a kidney tissue" according to the present invention is not particularly limited. A known procedure such as a phenol-chloroform treatment method can be appropriately selected and used for the preparation.

Examples of a kidney tissue from which a genomic DNA is prepared by such a method include an intact kidney tissue sampled in nephrectomy or the like, a kidney tissue frozen after sampled in nephrectomy or the like, and a kidney tissue fixed in formalin and embedded in paraffin after sampled at the time of nephrectomy or the like. Among these kidney tissues, a frozen kidney tissue is desirably used from the viewpoints that degradation of a genomic DNA in the kidney tissue and the like are suppressed until the kidney tissue is subjected to the detection method of the present invention, and that a bisulfite treatment, PCR, and so on can be performed more efficiently in the step of detecting a DNA methylation level described later.

Additionally, as described in Examples later, the present inventors have revealed by an Infinium assay that it is possible to clearly distinguish between renal cell carcinomas of unfavorable prognosis (CIMP-positive renal cell carcinomas) and relatively favorable renal cell carcinomas by detecting DNA methylation levels of 18 CpG sites of 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2). Further, the inventors have revealed a DNA methylation analysis method using a mass spectrometer that the hypermethylation status in the renal cell carcinomas of unfavorable prognosis continues in all regions of CpG islands comprising the CpG sites also.

Thus, the "CpG site" according to the present invention means CpG sites located at positions closer to at least one gene in the 17-gene group than to the other genes, and is preferably at least one CpG site within a CpG island located at the position closer to the gene than to the other genes, more preferably at least one CpG site located in promoter regions of the 17-gene group, and particularly preferably at least one CpG site at a position on a reference human genome sequence NCBI database Genome Build 37, the position being indicated by the chromosomal number and the position on the chromosome shown in Tables 1 to 4.

[Table 1]

| Gene symbol | Chromosomal number | Position on the chromosome |
|---|---|---|
| FAM150A | 8 | 53478309 |
| | | 53478316, 53478323 |
| | | 53478361, 53478363, 53478366 |
| | | 53478396, 53478403 |
| | | 53478426, 53478428 |
| | | 53478454 |
| | | 53478477 |
| | | 53478496, 53478499 |
| | | 53478504 |
| | | 53478511 |
| | | 53478536 |
| | | 53478585, 53478588, 53478592 |
| | | 53478624, 53478626 |
| GRM6 | 5 | 178422244 |
| | | 178422320, 178422324 |
| | | 178422375, 178422380 |
| ZNF540 | 19 | 38042472, 38042474 |
| | | 38042496 |
| | | 38042518 |
| | | 38042530, 38042532 |
| | | 38042544, 38042552 |
| | | 38042576 |
| | | 38042800, 38042802 |
| | | 38042816 |

[Table 2]

| Gene symbol | Chromosomal number | Position on the chromosome |
|---|---|---|
| ZFP42 | 4 | 188916867 |
| | | 188916875 |
| | | 188916899 |
| | | 188916913 |
| | | 188916982, 188916984 |
| ZNF154 | 19 | 58220494 |
| | | 58220567 |
| | | 58220627 |
| | | 58220657, 58220662 |
| | | 58220706 |
| | | 58220766, 58220773 |

(continued)

| Gene symbol | Chromosomal number | Position on the chromosome |
|---|---|---|
| RIMS4 | 20 | 43438576 |
| | | 43438621 |
| | | 43438865 |
| PCDHAC1 | 5 | 140306458 |
| KHDRBS2 | 6 | 62995963 |
| ASCL2 | 11 | 2292004 |
| | | 2292542, 2292544 |
| KCNQ1 | 11 | 2466409 |
| PRAC | 17 | 46799640 |
| | | 46799645, 46799648 |
| | | 46799654 |
| | | 46799745 |
| | | 46799755 |

[Table 3]

| Gene symbol | Chromosomal number | Position on the chromosome |
|---|---|---|
| WNT3A | 1 | 228194448 |
| | | 228195688 |
| | | 228195722 |
| | | 228195779 |
| TRH | 3 | 129693350, 129693352, 129693355, 129693358 |
| | | 129693406, 129693412 |
| | | 129693425 |
| | | 129693500 |
| | | 129693518, 129693521, 129693528 |
| | | 129693540, 129693543 |
| | | 129693563 |
| | | 129693570, 129693574 |
| | | 129693586 |
| | | 129693607 |
| | | 129693613 |
| | | 129693628 |
| | | 129693635 |
| | | 129693672 |
| FAM78A | 9 | 134152531 |

(continued)

| Gene symbol | Chromosomal number | Position on the chromosome |
|---|---|---|
| ZNF671 | 19 | 58238740 |
| | | 58238780 |
| | | 58238810 |
| | | 58238850 |
| | | 58238928 |
| | | 58238954 |
| | | 58238987 |
| | | 58239012 |
| | | 58239027 |

[Table 4]

| Gene symbol | Chromosomal number | Position on the chromosome |
|---|---|---|
| SLC13A5 | 17 | 6616653, 6616655, 6616657 |
| | | 6616702, 6616705, 6616707 |
| | | 6616733 |
| | | 6616751 |
| | | 6616763, 6616768 |
| | | 6616812 |
| | | 6616826, 6616828 |
| | | 6616851, 6616854, 6616857 |
| | | 6616927, 6616929 |
| | | 6616968, 6616973 |
| | | 6617030, 6617038, 6617040, 6617044 |
| | | 6617077 |
| | | 6617124 |
| | | 6617251, 6617255 |
| | | 6617287, 6617291 |
| | | 6617300, 6617305 |
| | | 6617382 |
| | | 6617421, 6617423 |
| | | 6617456 |
| | | 6617466, 6617470 |
| | | 6617382 |
| | | 6617398, 6617402, 6617405 |
| | | 6617415 |
| | | 6617421, 6617423 |
| | | 6617466, 6617470 |

(continued)

| Gene symbol | Chromosomal number | Position on the chromosome |
|---|---|---|
| | | 6617595, 6617597 |
| NKX6-2 | 10 | 134599860 |

Moreover, in the present invention, typically, FAM150A is a gene encoding a protein specified under RefSeq ID: NP_997296, GRM6 is a gene encoding a protein specified under RefSeq ID: NP_000834, ZNF540 is a gene encoding a protein specified under RefSeq ID: NP_689819, ZFP42 is a gene encoding a protein specified under RefSeq ID: NP_777560, ZNF154 is a gene encoding a protein specified under RefSeq ID: NP_001078853, RIMS4 is a gene encoding a protein specified under RefSeq ID: NP_892015, PCDHAC1 is a gene encoding a protein specified under RefSeq ID: NP_061721, KHDRBS2 is a gene encoding a protein specified under RefSeq ID: NP_689901, ASCL2 is a gene encoding a protein specified under RefSeq ID: NP_005161, KCNQ1 is a gene encoding a protein specified under RefSeq ID: NP_000209, PRAC is a gene encoding a protein specified under RefSeq ID: NP_115767, WNT3A is a gene encoding a protein specified under RefSeq ID: NP_149122, TRH is a gene encoding a protein specified under RefSeq ID: NP_009048, FAM78A is a gene encoding a protein specified under RefSeq ID: NP_203745, ZNF671 is a gene encoding a protein specified under RefSeq ID: NP_079109, SLC13A5 is a gene encoding a protein specified under RefSeq ID: NP_808218, and NKX6-2 a gene encoding a protein specified under RefSeq ID: NP_796374.

In the present invention, the "method for detecting a DNA methylation level" may be any method capable of quantifying a DNA methylation level at a particular CpG site. A known method can be appropriately selected for the detection. Examples of such a known method include first to seventh methods described below.

The first method is a method based on the following principle. First, the genomic DNA is treated with bisulfite. Note that this bisulfite treatment converts unmethylated cytosine residues to uracil, but does not convert methylated cytosine residues (see Clark SJ et al., Nucleic Acids Res, 1994, vol. 22, pp. 2990 to 7). Then, using the bisulfite-treated genomic DNA as a template, the full genome is amplified, enzymatically fragmented (normally fragmented into approximately 300 to 600 bp), and dissociated into single strands.

Moreover, in the first method, a probe is prepared which is capable of hybridizing to the genomic DNA converted by the bisulfite treatment, the base at the 3' end of the probe being a base complementary to cytosine of the CpG site. Specifically, in a case where the CpG site is methylated, the base at the 3' end of the probe is guanine; meanwhile, in a case where the CpG site is not methylated, the base at the 3' end of the probe is adenine.

Then, two types of such probes differing from each other only in the base at the 3' end complementary to the CpG site are hybridized to the fragmented genomic DNA, and a single-base extension reaction is carried out in the presence of a fluorescence-labeled base. As a result, in the case where the CpG site is methylated, the fluorescence-labeled base is incorporated into the probe having guanine as the base at the 3' end (probe for detecting methylation). On the other hand, in the case where the CpG site is not methylated, the fluorescence-labeled base is incorporated into the probe having adenine as the base at the 3' end (probe for detecting unmethylation) . Hence, the DNA methylation level can be calculated from an intensity of fluorescence emitted by the probe for detecting methylation and/or the probe for detecting unmethylation.

Further, as another embodiment of the first method, instead of the above-described probe for detecting methylation and probe for detecting unmethylation, a probe may be used which is capable of hybridizing to the genomic DNA converted by the bisulfite treatment, the base at the 3' end of the probe being a base complementary to guanine of the CpG site. Then, the probe is hybridized to the fragmented genomic DNA, and a single-base extension reaction is carried out in the presence of guanine labeled with a fluorescent substance and/or adenine labeled with a fluorescent dye different from the fluorescent substance. As a result, in the case where the CpG site is methylated, the fluorescence-labeled guanine is incorporated into the probe. On the other hand, in the case where the CpG site is not methylated, the fluorescence-labeled adenine is incorporated into the probe. Hence, the DNA methylation level can be calculated from an intensity of fluorescence emitted by each fluorescent substance incorporated in the probe.

An example of the first method includes a bead array method (for example, Infinium(registered trademark) assay).

Furthermore, in the first method, the CpG site as the target of the DNA methylation level detection is preferably at least one CpG site located at a position on the reference human genome sequence NCBI database Genome Build 37, the position being selected from the group consisting of position 53,478,454 on chromosome 8, position 178,422,244 on chromosome 5, position 38,042,472 on chromosome 19, position 188,916,867 on chromosome 4, position 58, 220, 662 on chromosome 19, position 43, 438, 865 on chromosome 20, position 140,306,458 on chromosome 5, position 62,995,963 on chromosome 6, position 2,292,004 on chromosome 11, position 2,466,409 on chromosome 11, position 46, 799, 640 on chromosome 17, position 58, 220, 494 on chromosome 19, position 228,194,448 on chromosome 1, position 129, 693, 613 on chromosome 3, position 134, 152, 531 on chromosome 9, position 58,238,928 on chromosome 19, position 6,617,030 on chromosome 17, and position 134, 599, 860 on chromosome 10. Additionally, in the first method according to the present invention, it is preferable to detect the DNA methylation level at at least one site among the 18 CpG sites. Nevertheless, from the viewpoint that the sensitivity or specificity in detecting an unfavorable prognostic risk can be further improved, the target of the DNA methylation level detection is more preferably multiple CpG sites (for example, 2 sites, 5 sites, 10 sites, 15 sites), and the target of the DNA methylationlevel detection is particularly preferably all of the 18 CpG sites.

The second method is a method based on the following principle. First, the genomic DNA is treated with bisulfite. Then, using the bisulfite-treated genomic DNA as a template, a DNA comprising at least one of the CpG sites is amplified with a primer to which a T7 promoter is added. Subsequently, the resultant is transcribed into RNA, and a base-specific cleavage reaction is carried out with an RNAse. Thereafter, the cleavage reaction product is subjected to a mass measurement with a mass spectrometer.

After that, the mass of the methylated cytosine residues (the mass of cytosine) and the mass of the unmethylated cytosine residues (the mass of uracil), which are obtained by the mass measurement, are compared with each other to calculate the DNA methylation level at the CpG site.

An example of the second method includes a DNA methylation analysis method using a mass spectrometer (for example, MassARRAY(registered trademark), see Jurinke C et al., Mutat Res, 2005, vol. 573, pp. 83 to 95).

Additionally, in the second method, the CpG site as the target of the DNA methylation level detection is preferably at least one CpG site contained in base sequences of SEQ ID NOs: 1 to 16. From the viewpoint that the sensitivity or specificity in detecting an unfavorable prognostic risk can be further improved, the CpG site is more preferably at least one CpG site among a CpG site group shown in Tables 5 to 8 below and having an area under the ROC curve (AUC) to be described later larger than 0.90, and further preferably at least one CpG site among a CpG site group having an AUC larger than 0.95 shown in Tables 5 to 8 below. The target of the DNA methylation level detection is particularly preferably all among the CpG site group having an AUC larger than 0.95.

[Table 5]

| Gene symbol | Chromosomal number | Target gene name primer set name CpG site | Position on the chromosome | AUC value | Cutoff value | Specificity | Sensitivity | 1-specificity |
|---|---|---|---|---|---|---|---|---|
| FAM150A | 8 | FAM150A_MA_14_CpG_8 | 53478309 | 0.936 | 0.108 | 0.833 | 0.941 | 0.059 |
| | | FAM150A_MA_14_CpG_9.10 | 53478316, 53478323 | 0.947 | 0.074 | 0.917 | 0.838 | 0.162 |
| | | FAM150A_MA_14_CpG_131415 | 53478361, 53478363, 53478366 | 0.912 | 0.108 | 0.833 | 0.853 | 0.147 |
| | | FAM150A_MA_14_CpG_18,19 | 53478396, 53478403 | 0.945 | 0.183 | 1.000 | 0.838 | 0.162 |
| | | FAM150A_MA_14_CpG_21.22 | 53478426, 53478428 | 0.934 | 0.338 | 0.917 | 0.912 | 0.088 |
| | | FAM150A_MA_14_CpG_26 | 53478477 | 0.968 | 0.307 | 0.833 | 0.985 | 0.015 |
| | | FAM150A_MA_14_CpG_27.28 | 53478496, 53478499 | 0.939 | 0.255 | 0.917 | 0.941 | 0059 |
| | | FAM150A_MA_14_CpG_29 | 53478504 | 0.911 | 0.055 | 0.917 | 0.926 | 0.074 |
| | | FAM150A_MA_14_CpG_30 | 53478511 | 0.968 | 0.307 | 0.833 | 0.985 | 0.015 |
| | | FAM150A_MA_14_CpG_31 | 53478536 | 0.925 | 0.072 | 0.833 | 0.941 | 0.059 |
| | | FAM150A_MA_14_CpG_37.38.39 | 53478585, 53478588, 53478592 | 0.912 | 0.227 | 0.150 | 0.971 | 0.029 |
| | | FAM150A_MA_14_CpG_41.42 | 53478624, 53478626 | 0.939 | 0.255 | 0.917 | 0.941 | 0 059 |
| GRM6 | 5 | GRM6_MA_8_CpG_12 | 178422320, 178422324 | 0.903 | 0.232 | 0.786 | 0.932 | 0.068 |
| | | GRM6_MA_8_CpG_4.5 | 178422375, 178422380 | 0.931 | 0.115 | 0.929 | 0.83 | 0.17 |
| ZFP42 | 4 | ZFP42_MA_2_CoG_3 | 188916875 | 0.917 | 0.202 | 0.786 | 0.943 | 0.057 |
| | | ZFP42_MA_2_CpG_4 | 188916899 | 0.933 | 0.255 | 0.929 | 0.841 | 0.159 |
| | | ZFP42_MA_2_CpG_5 | 188916913 | 0.928 | 0.133 | 0.929 | 0 886 | 0114 |
| | | ZFP42_MA_2_CpG_7.8 | 188916982, 188916984 | 0.932 | 0.345 | 0.857 | 0.909 | 0.091 |

(continued)

| Gene symbol | Chromosomal number | Target gene name primer set name CpG site | Position on the chromosome | AUC value | Cutoff value | Specificity | Sensitivity | 1-specificity |
|---|---|---|---|---|---|---|---|---|
| ZNP540 | 19 | ZNP540_MA_17_CpG_3.4 | 38042472, 38042474 | 0.928 | 0.222 | 0.833 | 0.897 | 0.103 |
| | | ZNF540_MA_17_CpG_6 | 38042496 | 0.983 | 0.41 | 1 | 0.983 | 0.017 |
| | | ZNF540_MA_17_CpG_9 | 38042518 | 0.96 | 0.357 | 1 | 0.931 | 0.069 |
| | | ZNF540_MA_17_CpG_10.11 | 38042530, 38042532 | 0.991 | 0.364 | 1 | 0966 | 0.034 |
| | | ZNP640_MA_17_CpG_12.13 | 38042544, 38042552 | 0.927 | 0.477 | 1 | 0.81 | 0.19 |
| | | ZNF540_MA_17_CpG_15 | 38042576 | 0.92 | 0.282 | 1 | 0.81 | 0.19 |
| | | ZNF540_MA_17_CpG_24.25 | 38042800, 38042802 | 0.941 | 0.502 | 0.833 | 0.966 | 0.034 |
| | | ZNFS40_MA_17_CoG_26 | 38042816 | 0.928 | 0.378 | 0.833 | 0.897 | 0.103 |

[Table 6]

| Gene symbol | Chromosomal number | Target gene name_primer set name CpG site | Position on the chromosome | AUC value | Cutoff value | Specificity | Sensitivity | 1 - specificity |
|---|---|---|---|---|---|---|---|---|
| ZNF154 | 19 | ZNF154_MA_5_CpG_1 | 58220567 | 0.956 | 0.133 | 0.929 | 0.909 | 0.091 |
| | | ZNF154_MA_5_CpG_4 | 58220627 | 0.966 | 0.148 | 0.857 | 0.955 | 0.045 |
| | | ZNF154_MA_5_CpG_5.6 | 58220657, 58220662 | 0.959 | 0.222 | 0929 | 0.955 | 0.045 |
| | | ZNF154_MA_5_CpG_8 | 58220706 | 0.912 | 0.118 | 1 | 0.75 | 0.25 |
| | | ZNF154_MA_5_CpG_11.12 | 58220766, 58220773 | 0.917 | 0.368 | 0.929 | 0.184 | 0.216 |
| RIMS4 | 20 | RIMS4_MA_9_CpG_15 | 43438576 | 0.913 | 0.102 | 0.833 | 0.877 | 0.123 |
| | | RIMS4_MA_9_CpG_17 | 43438621 | 0.914 | 0.135 | 0.833 | 0.864 | 0.136 |
| PRAC | 17 | PRAC_MA_2_CoG_2.3 | 46799645, 46799648 | 0.943 | 0.415 | 0.857 | 0.943 | 0.057 |
| | | PRAC_MA_2_CpG_4 | 46799654 | 0.915 | 0.393 | 0.786 | 0.932 | 0.068 |
| | | PRAC_MA_2_CpG_7 | 46199745 | 0.944 | 0.35 | 0.929 | 0.864 | 0.136 |
| | | PRAC_MA_2_CpG_8 | 46799755 | 0.957 | 0.407 | 0.929 | 0.898 | 0.102 |
| TRH | 3 | TRH_MA_8_CpG_2.3.4.5 | 129693350, 129693352, 129693355, 129693358 | 0.903 | 0.158 | 0.846 | 0.795 | 0.205 |
| | | TRH_MA_8_CpG_11.12 | 129693406, 129693412 | 0.973 | 0308 | 1 | 0.886 | 0.114 |
| | | TRH_MA_8_CoG_13 | 129693425 | 0.917 | 0.172 | 0.255 | 0.841 | 0.159 |
| | | TRH_MA_8_CpG_25 | 129693500 | 0902 | 0.21 | 0.846 | 0.898 | 0.102 |
| | | TRH_MA_8_CpG_272829 | 129693518, 129693521, 129693528 | 095 | 0.258 | 0.846 | 0.932 | 0.068 |
| | | TRH_MA_8_CpG_30,31 | 129693540, 129693543 | 0 943 | 0.175 | 0.923 | 0.909 | 0.091 |
| | | TRH_MA_8_CpG_32 | 129693563 | 0.902 | 0.175 | 0.846 | 0.932 | 0.068 |
| | | TRH_MA_8_CpG_3334 | 129693570, 129693574 | 0.935 | 0.173 | 0.923 | 0.82 | 0.148 |
| | | TRH_MA_8_CpG_35 | 129693586 | 0.952 | 0.11 | 0.923 | 0.92 | 0.08 |
| | | TRH_MA_8_CpG_36 | 129693607 | 0.917 | 0.172 | 0.846 | 0.841 | 0.159 |
| | | TRH_MA_8_CpG_37 | 129693613 | 0.921 | 0.055 | 1 | 0.761 | 0.239 |
| | | TRH_MA_8_CpG_39 | 129693628 | 0.943 | 0.115 | 1 | 0.886 | 0.114 |
| | | TRH_MA_8_CpG_40 | 129693635 | 0.967 | 0.066 | 1 | 0.875 | 0.125 |

(continued)

| Gene symbol | Chromosomal number | Target gene name_primer set name CpG site | Position on the chromosome | AUC value | Cutoff value | Specificity | Sensitivity | 1 - specificity |
|---|---|---|---|---|---|---|---|---|
| | | TRH_MA_8_CpG_41 | 129693672 | 0.925 | 0.187 | 0.846 | 0.92 | 0.08 |

[Table 7]

| Gene symbol | Chromosomal number | Target gene name_primer set name CpG site | Position on the chromosome | AUC value | Cutoff value | Specificity | Sensitivity | 1-specificity |
|---|---|---|---|---|---|---|---|---|
| SLC13A5 | 17 | SLC13A5_MA_10_CpG_3.4.5 | 6616653, 6616855, 6616657 | 0.94 | 0.243 | 0929 | 0.83 | 0.17 |
| | | SLC13A5_MA_10_CpG_9.10.11 | 6616702, 6616705, 6616707 | 0.906 | 0.145 | 0.857 | 0.875 | 0.125 |
| | | SLC13A5_MA_10_CpG_12 | 6616733 | 0.983 | 0.075 | 0.929 | 0.966 | 0.034 |
| | | SLC13A5_MA_10_CpG_13 | 6616751 | 0.928 | 0.04 | 0.929 | 0.875 | 0.125 |
| | | SLC13A5_MA_10_CpG_1415 | 6616763, 6616768 | 0.946 | 0.205 | 0.857 | 0.898 | 0.102 |
| | | SLC13A5_MA_10_CpG_21 | 6616812 | 0.983 | 0.185 | 1 | 0.943 | 0057 |
| | | SLC13A5_MA_10_CpG_2223 | 6616826, 6616828 | 0.951 | 0.233 | 1 | 0.886 | 0.114 |
| | | SLC13A5_MA_10_CpG_2425.26 | 6616851, 6616854, 6616857 | 0.954 | 0.148 | 1 | 0.875 | 0.125 |
| | | SLC13A5_MA_10_CpG_30,31 | 6616927, 6616929 | 0.951 | 0.233 | 1 | 0.886 | 0.114 |
| | | SLC13A5_MA_10_CpG_34.35 | 6616968, 6616973 | 0.927 | 0.144 | 0.929 | 0.818 | 0.182 |
| | | SLC13A5_MA_10_CpG_40.41.42.43 | 6617030, 6617038, 6617040, 6617044 | 0.942 | 0.258 | 1 | 0.83 | 0.17 |
| | | SLC13A5_MA_10_CpG_44 | 6611077 | 0.949 | 0.138 | 0.857 | 0.955 | 0.045 |
| | | SLC13A5_MA_13_CpG_1 | 6617077 | 0.927 | 0.155 | 0.8 | 0.977 | 0.023 |
| | | SLG13A5_MA_13_CpG_2 | 6617124 | 0.93 | 0.318 | 1 | 0.864 | 0.136 |
| | | SLC13A5_MA_13_CpG_15.16 | 6617251, 6617255 | 0.916 | 0.278 | 08 | 0.898 | 0.102 |
| | | SLC13A5_MA_13_CpG_17.18 | 6617287, 6617291 | 0.931 | 0.267 | 1 | 0.795 | 0.205 |
| | | SLC13A6_MA_13_CoG_19,20 | 6617300, 6617305 | 0.93 | 0.328 | 1 | 0.864 | 0.136 |
| | | SLC13A5_MA_13_CpG_26 | 6617382 | 0.944 | 0.228 | 1 | 0.852 | 0.148 |
| | | SLC13A5_MA_13_CpG_32_33 | 6617421, 6617423 | 0.914 | 0.288 | 1 | 0.739 | 0.261 |
| | | SLC13A5_MA_13_CpG_35 | 6617456 | 0.913 | 0.392 | 0.9 | 0.898 | 0.102 |
| | | SLC13A5_MA_13_CpG_36.37 | 6617466, 6617470 | 0.934 | 0.238 | 1 | 0.773 | 0.227 |
| | | SLC13A5_MA_15_CpG_3 | 6617382 | 0.942 | 0.222 | 1 | 0.866 | 0134 |
| | | SLC13A5_MA_15_CpG_5.6.7 | 6617398, 6617402, 6617405 | 0.936 | 0.3 | 0.778 | 1 | 0.255 |
| | | SLC13A5_MA_15_CpG_8 | 6617415 | 0.908 | 0.388 | 0.889 | 0.896 | 0.104 |

(continued)

| Gene symbol | Chromosomal number | Target gene name_primer set name CpG site | Position on the chromosome | AUC value | Cutoff value | Specificity | Sensitivity | 1-specificity |
|---|---|---|---|---|---|---|---|---|
| | | SLC13A5_MA_15_CpG_9.10 | 6617421, 6617423 | 0.927 | 0.377 | 0.889 | 0.896 | 0.104 |
| | | SLC13A5_MA_15_CpG_13.14 | 6617466, 6617470 | 0.935 | 0.284 | 0.889 | 0.896 | 0.104 |
| | | SLC13AS_MA_15_CpG_20,21 | 6617595, 6617597 | 0.942 | 0.685 | 0.889 | 0.881 | 0.119 |

[Table 8]

| Gene symbol | Chromosomal number | Target gene name_primer set name CpG site | Position on the chromosome | AUC value | Cutoff value | Specificity | Sensitivity | 1 - specificity |
|---|---|---|---|---|---|---|---|---|
| ZNF671 | 19 | ZNF671_MA_8_CpG_4 | 58238740 | 0.906 | 0.048 | 0.929 | 0. 713 | 0.287 |
| | | ZNF671_MA_8_CpG_10 | 58238780 | 0.954 | 0.152 | 0.857 | 0.897 | 0.103 |
| | | ZNF671_MA_8_CpG_14 | 58238810 | 0.926 | 0.062 | 1 | 0.747 | 0.253 |
| | | ZNF671_MA_8_CpG_20 | 58238850 | 0.927 | 0.105 | 0.929 | 0.759 | 0.241 |
| | | ZNF671_MA_8_CpG_26 | 58238928 | 0.965 | 0.105 | 1 | 0.885 | 0,115 |
| | | ZNF671_MA_8_CpG_28 | 58238954 | 0.954 | 0.152 | 0.857 | 0.897 | 0.103 |
| | | ZNF671_MA_8_CpG_29 | 58238987 | 0.954 | 0.152 | 0.857 | 0.897 | 0.103 |
| | | ZNF671_MA_8_CpG_31 | 58239012 | 0.951 | 0.105 | 0.857 | 0.92 | 0.08 |
| | | ZNF671_MA_8_CpG_33 | 58239027 | 0.91 | 0.11 | 0.786 | 0.92 | 0.08 |
| WNT3A | 1 | WNT3A_MA_9_CpG_7 | 228195688 | 0.943 | 0.225 | 0.857 | 0.886 | 0.114 |
| | | WNT3A_MA_9_CpG_8 | 228195722 | 0.943 | 0.225 | 0.857 | 0.886 | 0.114 |
| | | WNT3A_MA_9_CpG_9 | 228195779 | 0.943 | 0.225 | 0.857 | 0.886 | 0.114 |
| ASCL2 | 11 | ASCL2_MA_8_CpG_9.10 | 2292542, 2292544 | 0.907 | 0.3 | 0.929 | 0.821 | 0.179 |

Note that "chromosomal number" and "position on chromosome" shown in Tables 5 to 8 indicate a position on the reference human genome sequence NCBI database Genome Build 37. "Target gene name_primer set name_CpG site" indicates the order of CpG sites in PCR products amplified using primer sets shown in Tables 17 and 18 in a DNA methylation analysis using a mass spectrometer to be described later (Example 5). As to "AUC value", "cutoff value", "specificity", "sensitivity", and "1-specificity", see Example 5 described later.

The third method is a method based on the following principle. First, the genomic DNA is treated with bisulfite. Note that this bisulfite treatment converts unmethylated cytosine residues to uracil, but uracil is expressed as thymine in the following extension reaction (sequence reaction). Then, using the bisulfite-treated genomic DNA as a template, a DNA comprising at least one of the CpG sites is amplified. Subsequently, the amplified DNAs are dissociated into single strands. Thereafter, only one of the dissociated single stranded DNAs is separated. After that, the extension reaction is performed on each base from one near the base at the CpG site, pyrophosphoric acid generated during this is caused to enzymatically emit light, and the intensity of the luminescence is measured. The intensity of luminescence from the methylated cytosine residue (luminescence intensity of cytosine) and the intensity of luminescence from the unmethylated cytosine residue (luminescence intensity of thymine) thus obtained are compared with each other to calculate the DNA methylation level (%) at the CpG site, for example, according to the following formula.

DNA methylation level (%)=luminescence intensity of cytosine×100/(luminescence intensity of cytosine+luminescence intensity of thymine).

Examples of the third method include a pyrosequencing method (registered trademark, Pyrosequencing) (see Anal. Biochem. (2000) 10: 103-110) and the like.

The fourth method is a method based on the following principle. First, the genomic DNA is treated with bisulfite. Next, in a reaction system containing an intercalator which emits fluorescence when inserted between DNA double strands, a nucleotide comprising at least one of the CpG sites is amplified using the bisulfite-treated genomic DNA as a template. Then, the temperature of the reaction system is changed to detect a variation in the intensity of fluorescence emitted by the intercalator. A melting curve of the nucleotide comprising at least one of the CpG sites is compared with a melting curve of an amplification product obtained by using methylated/unmethylated control specimens as templates to then calculate the DNA methylation level at the CpG site.

An example of the fourth method includes a methylation-sensitive high resolution melting analysis (MS-HRM, see Wojdacz TK et al., Nat Protoc., 2008, vol. 3, pp. 1903 to 8).

The fifth method is a method based on the following principle. First, the genomic DNA is treated with bisulfite. Next, prepared are a primer set capable of amplification in the case where the CpG site is methylated, and a primer set capable of amplification in the case where the CpG site is not methylated. Then, using the bisulfite-treated genomic DNA as a template and these primer set, a nucleotide comprising at least one of the CpG sites is amplified. Subsequently, amounts of the obtained amplification products, that is, the amount of the amplification product specific to the methylated CpG site and the amount of the amplification product specific to the unmethylated CpG site, are compared with each other to calculate the DNA methylation level at the CpG site.

Further, as another embodiment of the fifth method, first, the genomic DNA is treated with bisulfite. Next, an oligonucleotide probe is prepared which has a nucleotide capable of hybridizing in the case where the CpG site is methylated, and which is labeled with a reporter fluorescent dye and a quencher fluorescent dye. In addition, an oligonucleotide probe is prepared which has a nucleotide capable of hybridizing in the case where the CpG site is not methylated, and which is labeled with a quencher fluorescent dye and a reporter fluorescent dye different from the aforementioned reporter fluorescent dye. Then, the oligonucleotide probes are hybridized to the bisulfite-treated genomic DNA. Further, using as a template the genomic DNA with the oligonucleotide probes hybridized thereto, a nucleotide comprising the CpG site is amplified. Subsequently, fluorescences emitted by the reporter fluorescent dyes through degradation of the oligonucleotide probes associated with the amplification are detected. The intensity of the fluorescence emitted by the reporter fluorescent dye specific to the methylated cytosine CpG site and the intensity of the fluorescence emitted by the reporter fluorescent dye specific to the unmethylated cytosine CpG site thus detected are compared with each other to calculate the DNA methylation level at the CpG site.

Examples of the fifth method include methylation-specific quantitative PCR (methylation-specific polymerase chain reaction (MS-PCR) using real-time quantitative PCR) such as MethyLight assay using TaqMan probe(registered trademark).

The sixth method is a method based on the following principle. First, the genomic DNA is treated with bisulfite. Next, using as a template a nucleotide comprising the bisulfite-converted CpG site, a sequencing reaction is performed directly. Then, the fluorescence intensities of the determined base sequence, that is, the fluorescence intensity from the methylated cytosine residue (fluorescence intensity of cytosine) and the fluorescence intensity from of the unmethylated cytosine residue (fluorescence intensity of thymine) are compared with each other to calculate the DNA methylation level at the CpG site.

Further, as another embodiment of the sixth method, first, the genomic DNA is treated with bisulfite. Then, a nucleotide comprising the bisulfite-converted CpG site is cloned by a PCR reaction or the like. Subsequently, the base sequence of each of multiple cloned products thus obtained is determined. The number of cloned products having a base sequence specific to the methylated cytosine CpG site and the number of cloned products having a base sequence specific to the unmethylated cytosine CpG site are compared with each other to thereby calculate the DNA methylation level at the CpG site.

Examples of the sixth method include bisulfite direct sequencing and bisulfite cloning sequencing (see Kristensen LS et al., Clin Chem, 2009, vol. 55, pp. 1471 to 83).

The seventh method is a method based on the following principle. First, the genomic DNA is treated with bisulfite. Then, using as a template a nucleotide comprising the bisulfite-converted CpG site, a region comprising the CpG site is amplified by PCR. Subsequently, the amplified DNA fragments are treated with a restriction enzyme capable of recognizing sites differing in sequence from each other in the cases where the CpG site is and is not methylated. Thereafter, band intensities of restriction enzyme fragments from the methylated CpG site and restriction enzyme fragments from the unmethylated CpG site, which are fractionated by electrophoresis, are quantitatively analyzed, so that the DNA methylation level at the CpG site can be calculated.

An example of the seventh method includes COBRA (combined bisulfite restriction enzyme analysis).

Although the methods that can be suitably used as the "method for detecting a DNA methylation level" of the present invention have been described above, the present invention is not limited thereto. Moreover, as described above, the genomic DNA prepared from a subject is further treated with bisulfite in detecting the DNA methylation level. Thus, the method for detecting an unfavorable prognostic risk of renal cell carcinoma of the present invention may be a method, wherein the step (b) is a step of treating the genomic DNA prepared in the step (a) with bisulfite and detecting a DNA methylation level of the CpG site.

Those skilled in the art can set an indicator for determining whether or not the subject is classified into an unfavorable prognosis group according to the DNA methylation level detected in the step (b) in the present invention, as appropriate in accordance with the method for detecting a DNA methylation level. For example, as described in Examples later, a receiver operating characteristic (ROC) analysis is performed on each CpG site to obtain the sensitivity (positive rate) and specificity. Further, a DNA methylation level at which the sum of the sensitivity and the specificity is the maximum can be set as the indicator (cutoff value, diagnostic threshold) . If a detected DNA methylation level is higher than the cutoff value, the subject can be classified into the unfavorable prognosis group.

Moreover, in the present invention, from the viewpoint that the sensitivity or specificity in detecting an unfavorable prognostic risk of renal cell carcinoma can be further improved, not only a DNA methylation level but also the number of CpG sites exhibiting a value higher than the cutoff value may be used as an indicator for determining whether or not the subject is classified into the unfavorable prognosis group. For example, as described in Examples later, if the number of sites satisfying the cutoff value is 15 or more among 23 CpG units according to the present invention, the subject may be classified into the unfavorable prognosis group (see Fig. 24 illustrated later).

In this manner, the present invention makes it possible to judge an unfavorable prognostic risk of renal cell carcinoma after nephrectomy, which cannot be detected by the existing classification criteria of histological observation and the like. Although nephrectomy is the first choice as a method for treating renal cell carcinoma, if metastasis/recurrence can be discovered at an early stage, an immunotherapy, molecularly-targeted therapeutic drug, or the like can be expected to be effective against the metastasis/recurrence.

Thus, the present invention can also provide a method for treating renal cell carcinoma, the method comprising: a step of administering a molecularly targeted therapeutic drug to the subject classified into the unfavorable prognosis group by the method of the present invention and/or a step of conducting an immunotherapy of the subject.

Further, in the present invention, patients classified into the unfavorable prognosis group among a large number of renal cell carcinoma cases subjected to nephrectomy are subjected to more intensive metastasis/recurrence screening. In this event, it is expected that discovering at an early stage can improve the clinical outcome; on the other hand, for patients not classified into the unfavorable prognosis group, the load of the metastasis/recurrence screening can be reduced.

The present invention provides an oligonucleotide according to any one of the following (a) and (b), which have a length of at least 12 bases, for use in the method for detecting an unfavorable prognostic risk of renal cell carcinoma:

(a) an oligonucleotide that is a pair of primers designed to flank at least one site selected from the CpG site group; and
(b) an oligonucleotide that is any one of a primer and a probe capable of hybridizing to a nucleotide comprising at least one site selected from the CpG site group.

Examples of the pair of primers according to (a) designed to flank at least one site selected from the CpG site group include primers (polymerase chain reaction (PCR) primers (forward primer and reverse primer)) capable of amplifying a DNA comprising at least one site selected from the bisulfite-converted CpG site group. The primers are primers capable of hybridizing to eachbisulfite-converted nucleotide on both sides of at least one site selected from the CpG site group.

In addition, an example of the primer according to (b) capable of hybridizing to the nucleotide comprising at least one site selected from the CpG site group includes a primer (sequencing primer) capable of performing an extension reaction on each base from one near the base at the bisulfite-converted CpG site. Further, an example of the probe according to (b) capable of hybridizing to the nucleotide comprising at least one site selected from the CpG site group includes a probe (so-called TaqMan probe) capable of hybridizing to the nucleotide comprising the bisulfite-converted CpG site.

Furthermore, the oligonucleotide of the present invention has a length of at least 12 bases, but preferably at least 15 bases, more preferably at least 20 bases.

The oligonucleotide capable of hybridizing to the particular nucleotide has a base sequence complementary to the particular nucleotide, but the base sequent does not have to be completely complementary as long as the oligonucleotide hybridizes. Those skilled in the art can design the sequences of these oligonucleotides as appropriate on the basis of the base sequence comprising the CpG site either bisulfite-converted or not converted, by a known procedure, for example, as described in Examples later, using MassARRAY primer design software EpiDesigner (http://www.epidesigner.com, manufactured by SEQUENOM, Inc.), pyrosequencing assay design software ver. 1.0 (manufactured by QIAGEN N. V.), or the like. Additionally, the phrase "comprising the CpG site" according to the present invention and similar phrases may mean not only containing all of the CpG site, that is, both of cytosine and guanine, but also containing a part thereof (cytosine, guanine, or uracil or thymine after unmethylated cytosine is converted with bisulfite).

The oligonucleotide of the present invention is preferably a primer selected from the group consisting of base sequences of SEQ ID NOs: 17 to 48 in a DNA methylation analysis method using a mass spectrometer as described in Examples later (see Tables 17 and 18). In addition, in pyrosequencing as described in Examples later, the oligonucleotide of the present invention is preferably a primer selected from the group consisting of base sequences of SEQ ID NOs: 49 to 57 (see Table 9).

Furthermore, the present invention can also provide a kit for use in the method for detecting an unfavorable prognostic risk of renal cell carcinoma, the kit comprising the oligonucleotide.

In a preparation of the oligonucleotide, the oligonucleotide may be fixed if necessary. For example, in the case of detection by an Infinium assay, a probe fixed to beads can be used. Moreover, the oligonucleotide may be labeled if necessary. For example, a biotin-labeled primer may be used in the case of detection by a pyrosequencing method, and a probe labeled with a reporter fluorescent dye and a quencher fluorescent dye may be used in the case of detection by a TaqMan probe method.

The kit of the present invention can comprise a preparation other than the preparation of the oligonucleotide. Such a preparation includes reagents required for bisulfate conversion (for example, a solution of sodium bisulfite and the like), reagents required for PCR reaction (for example, deoxyribonucleotides, thermostable DNA polymerases, and the like), reagents required for Infinium assay (for example, nucleotides labeled with a fluorescent substance), reagents required for MassARRAY (for example, RNAses for base-specific cleavage reaction), reagents required for pyrosequencing (for example, ATP-sulfurylase, adenosine-5'-phosphosulfate, luciferases, andluciferins for detection of pyrophosphoric acid; streptavidin for separation of single stranded DNAs; and the like), reagents required for MS-HRM (for example, intercalators which emit fluorescence when inserted between DNA double strands, and the like). Moreover, the examples include reagents required for detection of the labels (for example, substrates and enzymes, positive controls and negative controls, buffer solutions used for dilution or washing of samples (genomic DNA derived from kidney tissues of subjects, and the like), or the like) . The kit may further comprise an instruction thereof.

[Examples]

Hereinafter, the present invention will be more specifically described on the basis of Examples. However, the present invention is not limited to the following Examples. Note that the samples and methods used in Examples are as follows.

<Patients and Tissue Samples>

From materials surgically resected from 110 patients with primary clear cell renal cell carcinomas, 109 tumor tissue (T) samples and corresponding 107 non-cancerous renal cortex tissue (N) samples were obtained. The N samples showed no remarkable histological changes.

Note that these patients did not receive preoperative treatment but underwent nephrectomy at the National Cancer Center Hospital, Tokyo, Japan. The patients included 79 men and 31 women with a mean age of 62.8 ± 10.3 (mean ± standard deviation, 36 to 85 years old).

Moreover, histological diagnosis was made on the samples in accordance with the WHO classification (see Eble, J. N. et al., "Renal cell carcinoma. WHO classification of tumours. Pathology and genetics. Tumours of the urinary system and male genital organs", 2004, IARC Press, Lyon, pp. 10 to 43, Fig. 1).

Further, the histological grade of all the tumors was evaluated in accordance with the criteria described in "Fuhrman, S. A. et al., Am. J. Surg. Pathol., 1982, vol. 6, pp. 655 to 663" and classified according to the TNM classification in "Sobin, L. H. et al., International Union Against Cancer (UICC), TNM Classification Of Malignant Tumors, 6th edition, 2002, Wiley-Liss, New York, pp. 193 to 195".

In addition, the criteria for macroscopic configuration of renal cell carcinoma followed the criteria established for hepatocellular carcinoma (HCC) (see NPLs 4 to 6). Note that type 3 (contiguous multinodular type) HCCs show poorer histological differentiation and a higher incidence of intrahepatic metastasis than type 1 (single nodular type) and type 2 (single nodular type with extranodular growth) HCCs (see Kanai, T. et al., Cancer, 1987, vol. 60, pp. 810 to 819).

The presence or absence of vascular involvement was examined microscopically on slides stained with hematoxylin-eosin and elastica van Gieson.

The presence or absence of tumor thrombi in the main trunk of the renal vein was examined macroscopically. Note that renal cell carcinoma is usually enclosed by a fibrous capsule and well demarcated. Moreover, renal cell carcinoma hardly ever contains fibrous stroma between cancer cells. Hence, cancer cells were successfully obtained from the surgical specimens, avoiding contamination with both non-cancerous epithelial cells and stromal cells.

Furthermore, for comparison with the RCC patients, 29 samples of normal renal cortex tissues (C1 to C29) were obtained from materials that had been surgically resected from 29 patients without any primary renal tumor. The patients without any primary renal tumor from whom the samples were obtained included 18 men and 11 women with a mean age of 61.4 ± 10.8 (mean ± standard deviation, 31 to 81 years old). Additionally, 22 of these patients were patients who had undergone nephroureterectomy for urothelial carcinomas of the renal pelvis and ureter, while 6 patients had undergone nephrectomy with resection of retroperitoneal sarcoma around the kidney. The remaining one patient had undergone paraaortic lymph node dissection for metastatic germ cell tumor, which resulted in simultaneous nephrectomy because it was difficult to preserve the renal artery.

All the patients included in this study provided written informed consent. In addition, the study was conducted with the approval of the Ethics Committee of the National Cancer Center, Tokyo, Japan.

<Infinium Assay>

High-molecular-weight DNA from fresh frozen tissue samples obtained from the patients was extracted by treatment with phenol-chloroform, followed by dialysis (see Sambrook, J. et al., Molecular Cloning: A Laboratory Manual. Third Edition, Cold Spring Harbor Laboratory Press, NY, pp. 6.14 to 6.15).

Then, 500-ng aliquots of the DNA were subjected to bisulfite conversion using an EZ DNA Methylation-Gold (TM) kit (manufactured by Zymo Research Corporation).

Subsequently, DNA methylation status at 27578 CpG sites was analyzed at single-CpG resolution using the Infinium HumanMethylation27 Bead Array (manufactured by Illumina, Inc.). This array contains CpG sites located within the proximal promoter regions of the transcription start sites of 14475 genes (consensus coding sequences) registered in the NCBI database. Moreover, on average, two sites were selected per gene, and furthermore, 3 to 20 CpG sites were selected per gene for 200 or more cancer-related and imprinted genes, and employed for the array. In addition, 40 control probes were employed for each array. These control probes included staining, hybridization, extension, and bisulfate conversion controls, as well as negative controls.

Note that an Evo robot (manufactured by Tecan Group Ltd.) was used for automated processing of the bisulfite-converted DNA. Moreover, whole-genome amplification was performed using the Infinium Assay Kit (manufactured by Illumina, Inc.) (see Bibikova, M. et al., Epigenomics, 2009, vol. 1, pp. 177 to 200).

Then, after hybridization between the DNA fragments thus amplified and the probes on the array, the specifically hybridized DNA was fluorescence-labeled by a single-base extension reaction. Subsequently, the DNA was detected using a BeadScan reader (manufactured by Illumina, Inc.) in accordance with the manufacturer's protocol. The obtained data were analyzed using GenomeStudio methylation software (manufactured by Illumina, Inc.).

Note that, at each CpG site, the ratio of the fluorescent signal was measured using a relative ratio of a methylated probe to the sum of the methylated and unmethylated probes. Specifically, the so-called β value (range: 0.00 to 1.00) reflects the methylation level at an individual CpG site.

<Statistical Analysis>

In the Infinium assay, the call proportions (P-values for detection of signals above the background <0.01) for 32 probes in all of the tissue samples analyzed were 90% or less. Since such a low call proportion may be attributable to polymorphism at the probe CpG sites, these 32 probes were excluded from the present assay. In addition, all CpG sites on chromosomes X and Y were excluded, to avoid any gender-specific methylation bias. As a result, 26454 CpG sites on the autosomal chromosomes were left as a final analysis target.

Infinium probes showing significant differences in DNA methylation levels between the 29 C samples and 107 N samples were identified by a logistic model.

Probes on which DNA methylation levels showed ordered differences from C to N and then to T samples were identified by the cumulative logit model using the 29 C, 107 N, and 109 T samples.

Differences of DNA methylation status between 104 paired samples of N and corresponding T derived from a single patient were examined by the Wilcoxon signed-rank test.

A false discovery rate (FDR) of q=0.01 was considered significant.

Unsupervised hierarchical clustering (Euclidean distance, Ward method) based on DNA methylation levels ($\Delta\beta_{T-N}$) was performed in patients with clear cell renal cell carcinomas.

Correlations between clusters of patients and clinicopathological parameters were examined by Wilcoxon rank sum test and Fisher's exact test.

Survival curves of patients belonging to each cluster were calculated by the Kaplan-Meier method. Then, the differences were compared by the Log-rank test.

The number of Infinium assay probes showing DNA hypermethylation or DNA hypomethylation in each cluster and the average DNA methylation level ($\Delta\beta_{T-N}$) of each cluster were examined using Wilcoxon rank sum test at a significance level of $P<0.05$.

The CpG sites discriminating the clusters were identified by Fisher's exact test and random forest analysis (see Breiman, L., Mach. Learn., 2001, vol. 45, pp. 5 to 32).

(Example 1)

<DNA Methylation Alternations during Renal Carcinogenesis>

First, representative CpG sites found based on the Infinium assay were verified by performing a pyrosequencing method under conditions shown in Table 9. As a result, as shown in Figs. 2 to 4, there was a high correlation in terms of the DNA methylation level of each CpG site between the analysis results of the highly quantitative pyrosequencing method (the vertical axes in Figs. 2 to 4) and the analysis results of the Infinium assay (the horizontal axes in Figs. 2 to 4).

[Table 9]

| Gene | Target ID | Primer | | PCR conditions | | | | | |
|---|---|---|---|---|---|---|---|---|---|
| ZFP42 | cg06274159 | Forward | GGAGGAGTTGATGGGTGGTTGTA | 95°C 30sec | × 50cycles | | | | |
| | | Reverse | Biotin-CCCAAACACTCTACTATTTCCAATACCA | 60°C 30sec | | | | | |
| | | Sequencing | GGGTGGTTGTAGTTTGA | 72°C 1 min | | | | | |
| ZNF154 | cg08668790 | Forward | GGAAAGTAGGTTTTTTGAGTTTTTATTGG | 95°C 30sec | × 5cycles | 95°C 30sec | × 5cycles | 95°C 30sec | × 40cycles |
| | | Reverse | Biotin -CCCTAAAACTTAAATAAACCATTTCTCAT | 59°C 30sec | | 57°C 30sec | | 55°C 30sec | |
| | | Sequencing | TGAGTTTTTATTGGTTTAGTA | 72°C 1 min | | 72°C 1 min | | 72°C 1 sec | |
| ZNF540 | cg03975694 | Forward | AGGAGTAGGGTAGGGTAGAATTAGGTTAAAG | 95°C 30sec | × 5 cycles | 95°C 30sec | ×5cycles | 95°C 30sec | ×40cycles |
| | | Reverse | Biotin-ACCCAAACAACTCCTAAAACTACTTAATTCTC | 59°C 30sec | | 57°C 30sec | | 55°C 30sec | |
| | | Sequencing | GGTAGGGTAGAATTAGGTTAAA | 72°C 1 sec | | 72°C 1 min | | 72°C 1 sec | |

This confirmed that the data on the present Infinium assay were highly reliable.

Precancerous conditions in the kidney have been rarely discussed. Nevertheless, the present inventors have suggested that non-cancerous tissues are already at precancerous stages from the viewpoint of altered DNA methylation, despite the absence of any remarkable histological changes and the lack of association with chronic inflammation and persistent infection with viruses or other pathogenic microorganisms (PLT 1 and NPLs 4 to 7) .

In this regard, the result of the present Infinium assay was analyzed by the logistic model. The result revealed that the DNA methylation levels on 4830 probes were already altered in the N samples compared to those in the C samples (FDR, q=0.01, see (a) in Table 10).

Further, in order to reveal the DNA methylation alternations inherited by renal cell carcinomas themselves, probes on which DNA methylation levels showed ordered differences from C to N and then to T samples were identified by the cumulative logit model. As a result, such ordered differences of DNA methylation level were observed on 11089 probes (FDR, q=0.01, see (b) in Table 10) .

Furthermore, in order to reveal the cancer-prone DNA methylation alternations, 104 paired samples of N and T were examined by the Wilcoxon signed-rank test. As a result, significant differences between the N samples and the corresponding renal cell carcinomas were observed on 10870 probes (FDR, q=0.01, see (c) in Table 10).

[Table 10]

| | | | |
|---|---|---|---|
| (a) | The number of probes on which DNA methylation levels were altered in non-cancerous renal cortex tissues (N) from RCC patients relative to those in normal renal cortex tissues (C) from patients without any primary renal tumor (Logistic model analysis, False discovery rate (FDR) $_q$=0.01) | DNA hypermethylation ($\beta N > \beta C$) | 4,589 |
| | | DNA hypomethylation ($\beta N < \beta C$) | 241 |
| | | Total | 4,830 |
| (b) | The number of probes on which DNA methylation levels showed ordered differences from C to N, and then to T samples (tumorous tissue) (Cumulative logit model analysis, False discovery rate (FDR) $_q$=0.01) | DNA hypermethylation ($\beta C < \beta N < \beta T$, $\beta C < \beta N \fallingdotseq \beta T$ or $\beta C \fallingdotseq \beta N < \beta T$) | 6,653 |
| | | DNA hypomethylation ($\beta C > \beta N > \beta T$, $\beta C > \beta N \fallingdotseq \beta T$ or $\beta C \fallingdotseq \beta N > \beta T$) | 4,436 |
| | | Total | 11,089 |
| (c) | The number of probes showing different DNA methylation levels between T and the corresponding N samples (Wilcoxon signed-rank test analysis, False discovery rate (FDR) $_q$=0.01) | DNA hypermethylation ($\beta_{T-N} > 0$) | 5,408 |
| | | DNA hypomethylation ($\beta_{T-N} < 0$) | 5,462 |
| | | Total | 10,870 |

The above result revealed that although DNA hypomethylation was also observed during progression to established cancer, DNA hypermethylation frequently occurred at the very early stages of renal carcinogenesis.

Moreover, 801 probes were identified which satisfied all of the criteria shown in (a), (b), and (c) in Table 10; in other words, the DNA methylation alterations thereon were already evident at the non-cancerous stages, and also these alterations were inherited by and strengthened in the renal cell carcinomas.

(Example 2)

<Epigenetic Clustering of Renal Cell Carcinomas>

The result of the unsupervised hierarchical clustering using the DNA methylation levels ($\Delta\beta_{T-N}$) on the 801 probes revealed that 104 patients with clear cell renal cell carcinomas were subclustered into Cluster A (n=90) and Cluster B (n=14) (see Fig. 5). Note that, as described above, the DNA methylation status at the 801 probes was altered at the precancerous stages, which was presumably involved in the renal carcinogenesis.

Next, the clinicopathological parameters of clear cell renal cell carcinomas belonging to Clusters A and B, and TNM stage were examined. Table 11 shows the obtained result.

[Table 11]

| Clinicopathological parameters | | Cluster A (n=90) | ClusterB (n=14) | P |
|---|---|---|---|---|
| Age | | 62.08±10.08 | 67.36±11.06 | $8.36 \times 10^{-2}$ (b) |
| Sex | Male | 63 | 11 | $5.41 \times 10^{-1}$ (c) |
| | Female | 27 | 3 | |
| Tumor diameter (cm) | | 5.10±3.19 | 8.75±2.85 | $1.07 \times 10^{-4}$ (b) |
| Macroscopic configuration | Type 1 | 37 | 1 | $6.29 \times 10^{-4}$ (c) |
| | Type 2 | 29 | 2 | |
| | Type 3 | 24 | 11 | |
| Predominant histological grades (d) | G1 | 47 | 1 | $8.33 \times 10^{-6}$ (c) |
| | G2 | 35 | 4 | |
| | G3 | 7 | 7 | |
| Highest histological grades (e) | G1 | 8 | 0 | $5.67\times10^{-4}$ (c) |
| | G2 | 43 | 1 | |
| | G3 | 24 | 4 | |
| Vascular involvement | Negative | 54 | 1 | $2.45 \times 10^{-4}$ (c) |
| | Positive | 36 | 13 | |
| Renal vein tumor thrombus formation | Negative | 69 | 5 | $3.38 \times 10^{-3}$ (c) |
| | Positive | 21 | 9 | |
| Predominant growth pattern Expansive (d) | Expansive | 84 | 7 | $1.86 \times 10^{-4}$ (c) |
| | Infiltrative | 6 | 7 | |
| Most aggressive growth pattern (e) | Expansive | 57 | 4 | $2.06 \times 10^{-3}$ (c) |
| | Infiltrative | 33 | 10 | |
| Tumor necrosis | Negative | 71 | 2 | $486 \times 10^{-6}$ (c) |
| | Positive | 19 | 12 | |
| Invasion to renal pelvis | Negative | 83 | 10 | $3.98 \times 10^{-2}$ (c) |
| | Positive | 7 | 4 | |
| Pathological TNM stage | Stage 1 | 50 | 0 | $541 \times10^{-5}$ (c) |
| | Stage 2 | 1 | 1 | |
| | Stage 3 | 23 | 9 | |
| | Stage 4 | 16 | 4 | |

Note that, among "P-values" in Table 11, "P<0.05" are underlined, the numerical values with (b) are of the Wilcoxon rank sum test, and the numerical values with (c) are of the Fisher's exact test. Moreover, regarding the clinicopathological parameter with (d), findings in the predominant area are described if the tumor showed heterogeneity. Regarding the clinicopathological parameter with (e), if the tumor showed heterogeneity, the most aggressive features of the tumor are described.

Further, the survival rates of patients belonging to these Clusters A and B were also examined. The period of the survival rate analysis was 42 to 4024 days (mean: 1821 days). Figs. 6 and 7 show the obtained results (Kaplan-Meier survival curves).

As apparent from the result shown in Table 11, Cluster B had larger (or higher) values than Cluster A in terms of: the diameter of clear cell renal cell carcinomas, incidence of single nodular type with extranodular growth(type 2) or contiguous multinodular type (type 3) according to the aforementioned macroscopic configuration, frequencies of vascular involvement, renal vein tumor thrombus formation, infiltrating growth, tumor necrosis, and renal pelvis invasion, histological grade, and pathological TNM stage. Note that it is clear as shown in Table 11 that epigenetic clustering of renal cell carcinomas was dependent on neither sex nor age of the patients.

Moreover, as apparent from the results shown in Figs. 6 and 7, the recurrence-free survival rate (cancer-free survival rate) and overall survival rate of the patients belonging to Cluster B were significantly lower than those of the patients belonging to Cluster A (the P-value of the cancer-free survival rate was $4.16\times10^{-6}$, the P-value of the overall survival rate was $1.32\times10^{-2}$).

(Example 3)

<DNA Methylation Profiles of Renal Cell Carcinoma s>

Next, the proportions of probes showing various degrees of DNA hypermethylation in T samples compared to the corresponding N samples ($\Delta\beta_{T-N}$>0.1, 0.2, 0.3, 0.4, or 0.5) for all 26454 probes were analyzed. Moreover, the proportions of probes showing various degrees of DNA hypomethylation in N samples compared to the corresponding T samples ($\Delta\beta_{T-N}$<-0.1, -0.2, -0.3, -0.4, or -0.5) for all 26454 probes were analyzed. Figs. 8 to 12 show the obtained result.

As apparent from the result shown in Figs. 8 to 12, the probes showing prominent DNA hypomethylation ($\Delta\beta_{T-N}$<-0.5) were accumulated slightly more in Cluster B than in Cluster A. However, the incidence of DNA hypomethylation in Clusters A and B did not reach a statistically significant difference ($\Delta\beta_{T-N}$<-0.1, -0.2, -0.3, or -0.4) . On the other hand, the probes showing DNA hypermethylation were markedly accumulated in Cluster B relative to Cluster A, regardless of the degree of DNA hypermethylation ($\Delta\beta_{T-N}$>0.1, 0.2, 0.3, 0.4, or 0.5).

Thus, it was revealed that renal cell carcinomas belonging to Cluster B were characterized by accumulation of DNA hypermethylation.

Further, Tables 12 and 13 shows the top 61 probes on which DNA methylation levels differed markedly between Clusters A and B. Note that, in Tables 12 and 13, "target ID" indicates the probe number for the Infinium HumanMethylation27 Bead Array all assigned by Illumina, Inc., and "chromosomal number" and "position on chromosome" indicate a position on the reference human genome sequence NCBI database Genome Build 37 (hereinafter, the same applies to headings in Tables regarding probes) . "Y" under "CpG island" indicates that the corresponding probe is located within the CpG island, while "N" indicates that the corresponding probe is not located within the CpG island (the same applies to Tables 14 and 15). Further, "gene region" indicates that the corresponding probe is located in an exon or an intron, or upstream of the transcription start site (TSS). Furthermore, "P-value" indicates a value calculated by the Wilcoxon rank sum test.

[Table 12]

| | Target ID | Chromosomal number | Position on the chromosome | Gene symbol | CpG island | Gene region | $\Delta\ \beta_{T-n}$ (mean ± SD) | | *P-value* |
|---|---|---|---|---|---|---|---|---|---|
| | | | | | | | Cluster A (n=90) | Cluster B (n=14) | |
| 1 | cg18722841 | 11 | 71,954,982 | PHOX2A | Y | Exon 1 | 0.034±0.064 | 0.258±0.120 | $3.23\times10^{-8}$ |
| 2 | cg03975694 | 19 | 38,042,472 | ZNF540 | Y | Exon 1 | 0.173±0.112 | 0.415 ±0.089 | $5.24\times10^{-8}$ |
| 3 | cg22183706 | 11 | 14,993,818 | CALCA | Y | Exon 1 | 0.064±0.073 | 0.265±0.112 | $6.49\times10^{-8}$ |
| 4 | cg12374721 | 17 | 46,799,640 | PRAC | Y | Intron 1 | 0096±0.120 | 0.427±0.160 | $7.22\times10^{-8}$ |
| 5 | cg02367951 | 6 | 27,806,562 | HIST1 H2AK | Y | 445-bp TSS | 0.053±0.053 | 0.145±0.025 | $8.02\times10^{-8}$ |
| 6 | cg20023231 | 16 | 22,825,282 | HS3ST2 | Y | 578-bp TSS | 0.034±0.058 | 0.215±0.139 | $1.04\times10^{-7}$ |
| 7 | cg08668790 | 19 | 58,220,662 | ZNF154 | Y | 83-bp TSS | 0.087±0.112 | 0.411±0.170 | $1.10\times10^{-7}$ |
| 8 | cg14859460 | 5 | 178,422,244 | GRM6 | Y | 120-bp TSS | 0.077±0.105 | 0.434±0.184 | $1.10\times10^{-7}$ |
| 9 | cg06274159 | 4 | 188,916,867 | ZFP42 | Y | 58-bp TSS | 0.078±0.112 | 0.426±0.196 | $1.35\times10^{-7}$ |
| 10 | cg01291404 | 12 | 48,397,872 | COL2A1 | Y | Intron 1 | 0.019± 0.049 | 0.157±0.104 | $1.43\times10^{-7}$ |
| 11 | cg20312228 | 3 | 126,113,707 | CCDC37 | Y | 75-bp TSS | 0.096±0.097 | 0.330±0.127 | $1.43\times10^{-7}$ |
| 12 | cg05778847 | 19 | 38,746,538 | PPP1R14A | Y | Intron 1 | -0.010±0.050 | 0.166±0.131 | $1.50\times10^{-7}$ |
| 13 | cg00848728 | 1 | 58,716,018 | DAB1 | Y | Exon 1 | 0.023±0.043 | 0.178±0.149 | $1.66\times10^{-7}$ |
| 14 | cg18555440 | 11 | 17,741,687 | MYOD1 | Y | Exon 1 | 0.096±0.106 | 0.331±0.104 | $1.75\times10^{-7}$ |
| 15 | cg27059238 | 1 | 149,783,755 | HIST2H2BF | Y | Exon 1 | 0.052± 0.052 | 0.133±0.019 | $1.75\times10^{-7}$ |
| 16 | cg05445326 | 3 | 196.065.569 | TM4SF19 | N | 311-bp) TSS | -0.153±0.129 | -0.425±0.096 | $1.85\times10^{-7}$ |
| 17 | cg24784109 | 6 | 26,200,116 | HIST1H3D | Y | 652-bp TSS | 0.034±0.039 | 0.162±0.068 | $2.04\times10^{-7}$ |
| 18 | cg06263495 | 11 | 2,292,004 | ASCL2 | Y | Exon 1 | 0.118±0.133 | 0.410±0.144 | $2.26\times10^{-7}$ |
| 19 | cg09260089 | 10 | 134,599,860 | NKX6-2 | Y | 323-bp TSS | 0.078±0.083 | 0-372±0.150 | $2.26\times10^{-7}$ |
| 20 | cg16652063 | 17 | 6,616,653 | SLC13A5 | Y | Exon 1 | 0.101±0.105 | 0.376±0.134 | $2.51\times10^{-7}$ |
| 21 | cg22040627 | 17 | 6,617,030 | SLC13A5 | Y | 290-bp TSS | 0.045±0.072 | 0.283±0.103 | $2.64\times10^{-7}$ |
| 22 | cg02919422 | 8 | 55,370,544 | SOX17 | Y | Exon 1 | 0.125±0.122 | 0.362±0.117 | $2.92\times10^{-7}$ |
| 23 | cg17162024 | 8 | 53,418,454 | FAM150A | Y | 433-bp TSS | 0.126±0.120 | 0.499±0.184 | $3.40\times10^{-7}$ |
| 24 | cg25971347 | 16 | 86,544,339 | F0XF1 | Y | Exon 1 | 0.020±0.045 | 0.183±0.153 | $3.57\times10^{-7}$ |
| 25 | cg16232126 | 2 | 108,603,005 | SLC5A7 | Y | Exon 1 | 0.116±0.129 | 0.402±0.148 | $3.76\times10^{-7}$ |
| 26 | cg26309134 | 19 | 56,879,571 | ZNF542 | Y | Exon 1 | 0.021±0.047 | 0.308±0.197 | $3.76\times10^{-7}$ |
| 27 | cg06005396 | 19 | 590,541 | HCN2 | Y | Exon 1 | 0.053 0.052 | 0.238±0.127 | $3.95\times10^{-7}$ |
| 28 | cg25668368 | 2 | 163,695,882 | KCNH7 | Y | 642-bp TSS | 0.003±0.056 | 0.161±0.131 | $4.59\times10^{-7}$ |
| 29 | cg02245378 | 2 | 223,161,771 | CCDC140 | Y | 1095-bp TSS | 0.066±0.120 | 0.309±0.149 | $4.82\times10^{-7}$ |
| 30 | cg08555612 | 3 | 71,834,640 | PROK2 | Y | 283-bp TSS | 0.017±0.073 | 0.227±0.158 | $4.82\times10^{-7}$ |

[Table 13]

| | Target ID | Chromosomal number | Position on the chromosome | Gene symbol | CpG island | Gene region | $\Delta\ \beta_{T-N}$ (mean ± SD) | | *P-value* |
|---|---|---|---|---|---|---|---|---|---|
| | | | | | | | Cluster A ($_n$=90) | Cluster B (n=14) | |
| 31 | cg05521696 | 12 | 8,025,495 | SLC2A14 | Y | Exon 1 | 0.106±0.102 | 0.332±0.127 | $5.32\times10^{-7}$ |
| 32 | cg13870866 | 7 | 35,293,130 | TBX20 | Y | Exon 1 | 0.092±0.101 | 0.312±0.115 | $5.59\times10^{-7}$ |
| 33 | cg26705553 | 16 | 3,096,711 | MMP25 | Y | Exon 1 | 0.015±0.030 | 0.154±0.121 | $5.59\times10^{-7}$ |
| 34 | cg00489401 | 5 | 180,075,875 | FLT4 | Y | Intron 1 | 0.131±0.137 | 0.451±0.167 | $5.88\times10^{-7}$ |
| 35 | cg12741420 | 6 | 392,131 | IRF4 | Y | Intron 1 | 0.024±0.046 | 0.212±0.154 | $6.17\times10^{-7}$ |
| 36 | cg12768605 | 19 | 44,324,951 | LYPD5 | Y | 143-bp TSS | 0.075±0.096 | 0.294±0.125 | $6.17\times10^{-7}$ |
| 37 | cg19064258 | 16 | 22,826,117 | HS3ST2 | Y | Exon 1 | 0.081±0.086 | 0.297±0.151 | $6.17\times10^{-7}$ |
| 38 | cg01580681) | 4 | 174,450,016 | HAND2 | Y | Exon 1 | 0.066±0.106 | 0.332±0.169 | $6.48\times10^{-7}$ |
| 39 | cg08045570 | 6 | 1,390,502 | FOXF2 | Y | Exon 1 | 0.017±0.046 | 0.205±0.184 | $6.81\times10^{-7}$ |
| 40 | eg13666729 | 1 | 32,930,473 | ZBTB8B | Y | 185-bp TSS | 0.025±0.053 | 0.170±0.152 | $6.81\times10^{-7}$ |
| 41 | cg02162069 | 19 | 57,352,134 | ZIM2 | Y | 37-bp TSS | 0.021±0.061 | 0.148±0.069 | $7.15\times10^{-7}$ |
| 42 | cg21243096 | 1 | 38,511,557 | POU3F1 | Y | Exon 1 | 0.047±0.071 | 0.199±0.093 | $7.15\times10^{-7}$ |
| 43 | cg21790626 | 19 | 58,220,494 | ZNF154 | Y | Exon 1 | 0.065±0.093 | 0.375±0.199 | $7.51\times10^{-7}$ |
| 44 | cg04457979 | 11 | 2.890.647 | KCNQ1DN | Y | 616-bp TSS | 0.071±0.09 | 0.274±0.152 | $7.89\times10^{-7}$ |
| 45 | cg05488632 | 19 | 15,343,174 | EPHX3 | Y | Intron 1 | 0.085±0.091 | 0.293±0.131 | $7,89\times10^{-7}$ |
| 46 | cg14312526 | 3 | 138,665,291 | FOXL2 | Y | Exon 1 | 0.034±0.08 | 0.234±0.150 | $7.89\times10^{-7}$ |
| 47 | cg01144286 | 20 | 9,495,596 | C20orf103 | Y | Intron 1 | 0.002±0.019 | 0.097±0.103 | $8.28\times10^{-7}$ |
| 48 | cg01401376 | 6 | 133,563,342 | EYA4 | Y | Intron 1 | 0.012±0.021 | 0.140±0.129 | $8.28\times10^{-7}$ |
| 49 | cg27553955 | 2 | 42,720,326 | KCNG3 | Y | Exon 1 | 0.084±0.096 | 0.248±0.080 | $8.28\times10^{-7}$ |
| 50 | cg03469054 | 12 | 130,387,861 | TMEM132D | Y | Exon 1 | 0.069±0.073 | 0.306±0.155 | $8.70\times10^{-7}$ |
| 51 | cg11935147 | 1 | 145,075,831 | PDE4D1P | Y | Exon 1 | 0.049±0.080 | 0.240±0.137 | $8.70\times10^{-7}$ |
| 52 | cg16428251 | 3 | 137,483,479 | SOX14 | Y | 100-bp TSS | 0.080±0.090 | 0.294±0.143 | $8.70\times10^{-7}$ |
| 53 | cg19576304 | 18 | 56,940,022 | RAX | Y | Intron 1 | 0.077±0.098 | 0.281±0.124 | $8.70\times10^{-7}$ |
| 54 | cg02844545 | 6 | 10,882,043 | GCM2 | Y | Exon 1 | 0.079±0.104 | 0.281±0.134 | $9.13\times10^{-7}$ |
| 55 | cg23130254 | 2 | 176,964,588 | HOXD12 | Y | Exon 1 | 0.062±0.091 | 0.294±0.171 | $9.13\times10^{-7}$ |
| 56 | cg27389185 | 19 | 38,042,123 | ZNF540 | Y | 185-bp TSS | 0.139±0.106 | 0.321±0.082 | $9.13\times10^{-7}$ |
| 57 | cg19817399 | 15 | 75,018,674 | CYP1A1 | Y | 797-bp TSS | 0.022±0.044 | 0.163±0.118 | $9.58\times10^{-7}$ |
| 58 | cg06277657 | 7 | 137,532,374 | DGKI | Y | 765-bp TSS | 0.073±0.136 | 0.306±0.105 | $1.01\times10^{-6}$ |
| 59 | cg16924616 | 7 | 96,653,617 | DLX5 | Y | Exon 1 | 0.041±0.065 | 0.273±0.150 | $1.01\times10^{-6}$ |
| 60 | cg00662556 | 18 | 74,963,364 | GALR1 | Y | Intron 1 | 0.151±0.128 | 0.377±0.124 | $1.06\times10^{-6}$ |
| 61 | cg04473302 | 7 | 107,301,217 | SLC26A4 | Y | Exon 1 | 0.021±0.061 | 0.175±0.16 | $1.06\times10^{-6}$ |

Although only 19246 probes, 72.8%, out of the total of 26454 probes were located within CpG islands, 60 probes, 98 . 4%, out of the 61 probes located within CpG islands showed DNA hypermethylation in renal cell carcinomas belonging to Cluster B ($\Delta\beta_{T-N}$>0.097, Tables 12 and 13). Note that the remaining one probe among the 61 probes was located within a non-CpG island and showed DNA hypomethylation ($\Delta\beta_{T-N}$>-0.425 ± 0.096 in Cluster B).

The results described in Examples 2 and 3 revealed that Cluster B was well correlated with the clinicopathological phenotype and characterized by frequent DNA hypermethylation on CpG islands.

Note that such characteristics of renal cell carcinomas belonging to Cluster B are similar to those of CpG island methylator phenotype (CIMP)-positive cancers in other well-studied organs (for example, colon and stomach) (NPLs 8 to 11). In other words, this single-CpG resolution methylome analysis identified, for the first time, CIMP-positive renal cell carcinomas as Cluster B.

(Example 4)

<Identification of Hallmark CpG Sites of CIMP-Positive Renal Cell Carcinomas>

Correlations between DNA methylation levels (β values) in renal cell carcinoma tissues (T samples) and those in non-cancerous renal tissues (N samples) from representative patients with renal cell carcinomas belonging to Clusters A and B were examined. Figs. 13 and 14 show the obtained result in scattergrams. Note that Cases: 1 to 4 shown in Fig. 13 are examples of the representative patients with renal cell carcinomas belonging to Cluster A, and Cases: 5 to 8 shown in Fig. 14 are examples of the representative patients with renal cell carcinomas belonging to Cluster B.

As apparent from the result shown in Figs. 13 and 14, probes for which the DNA methylation levels were low in the N samples and for which the degree of DNA hypermethylation in the T samples relative to the corresponding N samples was prominent were obvious only in Cluster B, and not in Cluster A.

Based on this result, in order to discriminate renal cell carcinomas belonging to Cluster B from those belonging to Cluster A, focused on were probes for which the average β value in all N samples was less than 0.2 and the incidence of more than 0.4 $\Delta\beta_{T-N}$ was markedly high in Cluster B than Cluster A ($P<1.98\times10^{-6}$, Fisher's exact test).

Then, among such probes, 16 probes (15 genes: FAM150A, GRM6, ZNF540, ZFP42, ZNF154, RIMS4, PCDHAC1, KHDRBS2, ASCL2, KCNQ1, PRAC, WNT3A, TRH, FAM78A, and ZNF671) showed more than 0.4 $\Delta\beta_{T-N}$ in 6 or more (42.8% or more) renal cell carcinomas among the 14 renal cell carcinomas belonging to Cluster B. On the other hand, the 16 probes showed more than 0.4 $\Delta\beta_{T-N}$ in 2 or fewer (2.2% or less) renal cell carcinomas among the 90 renal cell carcinomas belonging to Cluster A (see Table 14).

[Table 14]

| Target ID | Chromosomal number | Position on the chromosome | CpG island | Gene symbol | The number of tumors whose $\Delta\beta$T-N>0.4 (%) | | P |
|---|---|---|---|---|---|---|---|
| | | | | | Cluster A (n=90) | Cluster B (n=14) | |
| **cg17162024** | **8** | **53,478,454** | **Y** | **FAM150A** | **2 (2.2)** | **12 (85.7)** | **$4.60\times10^{-12}$** |
| **cg14859460** | **5** | **178,422,244** | **Y** | **GRM6** | **0 (0)** | **10 (71.4)** | **$3.84\times10^{-11}$** |
| **cg03975694** | **19** | **38,042,472** | **Y** | **ZNF540** | **2 (2.2)** | **9 (64.3)** | **$3.64\times10^{-8}$** |
| **cg06274159** | **4** | **188,916,867** | **Y** | **ZFP42** | **1 (1.1)** | **8 (57.1)** | **$9.91\times10^{-8}$** |
| **cg08668790** | **19** | **58,220,662** | **Y** | **ZNF154** | **1 (1.1)** | **8 (57.1)** | **$9.91\times10^{-8}$** |
| **cg19332710** | **20** | **43,438,865** | **Y** | **RIMS4** | **2 (2.2)** | **8 (57.1)** | **$4.68\times10^{-7}$** |
| **cg12629325** | **5** | **140,306,458** | **Y** | **PCDHAC1** | **2 (2.2)** | **7 (50)** | **$5.10\times10^{-6}$** |
| **cg18239753** | **6** | **62,995,963** | **Y** | **KHDRBS2** | **2 (2.2)** | **7 (50)** | **$5.10\times10^{-6}$** |
| **cg06263495** | **11** | **2,292,004** | **Y** | **ASCL2** | **2 (2.2)** | **7 (50)** | **$5.10\times10^{-6}$** |
| **cg17575811** | **11** | **2,466,409** | **Y** | **KCNQ1** | **1 (1.1)** | **7 (50)** | **$1.21\times10^{-6}$** |
| **cg12374721** | **17** | **46,799,640** | **Y** | **PRAC** | **2 (2.2)** | **7 (50)** | **$5.1,0\times10^{-6}$** |
| **cg21790626** | **19** | **58,220,494** | **Y** | **ZNF154** | **0 (0)** | **7 (50)** | **$1.62\times10^{-7}$** |
| **cg01322134** | **1** | **228,194,448** | **Y** | **WNT3A** | **0 (0)** | **6 (42.9)** | **$1.98\times10^{-6}$** |

(continued)

| Target ID | Chromosomal number | Position on the chromosome | CpG island | Gene symbol | The number of tumors whose Δ $\beta$T-N>0.4 (%) | | P |
|---|---|---|---|---|---|---|---|
| | | | | | Cluster A (n=90) | Cluster B (n=14) | |
| cg01009664 | 3 | 129,693,613 | Y | TRH | 0 (0) | 6 (42.9) | $1.98\times10^{-6}$ |
| cg12998491 | 9 | 134,152,531 | Y | FAM78A | 0 (0) | 6 (42.9) | $1.98\times10^{-6}$ |
| cg19246110 | 19 | 58,238,928 | Y | ZNF671 | 0 (0) | 6 (42.9) | $1.98\times10^{-6}$ |

Moreover, as apparent from the result shown in Fig • 15, the DNA methylation levels ($\Delta\beta_{T-N}$) on the 16 CpG s ites differed completely between Clusters A and B.

Further, random forest analysis was performed using 869 probes on which DNA methylation levels ($\Delta\beta_{T-N}$) differed markedly between Clusters A and B (FDR [q=0.01]) (see Figs. 16 and 17). As a result, the top 4 probes were further identified which were able to discriminate Cluster A from Cluster B (see Table 15).

[Table 15]

| Target ID | Chromosomal number | Position on the chromosome | CpG island | Gene symbol | $\Delta \beta_{T\text{-}N}$ (mean ± SD) | | P |
|---|---|---|---|---|---|---|---|
| | | | | | Cluster A (n=90) | Cluster B (n=14) | |
| cg17162024 | 8 | 53,478,454 | Y | FAM150A | 0.126±0.120 | 0.499±0.184 | $3.40\times10^{-7}$ |
| cg22040627 | 17 | 6,617,030 | Y | SLC13A5 | 0.045±0.072 | 0.283 ±0.103 | $2.64\times10^{-7}$ |
| cg14859460 | 5 | 178,422,244 | Y | GRM6 | 0.077±0.105 | 0.434±0.184 | $1.10\times10^{-7}$ |
| cg09260089 | 10 | 134,599,860 | Y | NKX6-2 | 0.078±0.083 | 0.372±0.150 | $2.26\times10^{-7}$ |

Note that 2 genes (FAM150A and GRM6) were shared by the 15 genes and the top 4 genes which were found by the random forest analysis to be able to discriminate Cluster A from Cluster B.

Thus, CpG sites of these 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2) can be considered as hallmarks of CIMP-positive renal cell carcinomas, for example, renal cell carcinomas belonging to Cluster B. In other words, it was revealed that it was possible to detect an unfavorable prognostic risk of patients with renal cell carcinomas by detecting DNA methylation levels at CpG sites of the 17 genes.

In addition, levels of these genes expressed were analyzed by quantitative RT-PCR. The result revealed that DNA hypermethylation reduced the expression of these genes (see Table 16).

[Table 16]

| Gene | Measured value | N samples (n=28) | T samples (n=28) | *P-value* |
|---|---|---|---|---|
| ZNF540 | DNA methylation level | 0.1 18 ± 0.037 | 0.352 ± 0.161 | $1.15 \times 10^{-8}$ |
| | mRNA expression level | 1.085 ± 1.166 | 0.337 ± 0.443 | $1.70 \times 10^{-4}$ |
| ZFP42 | DNA methylation level | 0.077 ± 0.039 | 0.239 ± 0.226 | $4.59 \times 10^{-4}$ |
| | mRNA expression level | 19.424 ± 16.589 | 0.1 59 ± 0.540 | $2.40 \times 10^{-11}$ |
| ZNF154 | DNA methylation level | 0.035 ± 0.012 | 0.1 70 ± 0.210 | $8.74 \times 10^{-4}$ |
| | mRNA expression level | 1.574 ± 1.107 | 0.550 ± 0.386 | $2.59 \times 10^{-6}$ |
| KCNQ1 | DNA methylation level | 0.068 ± 0.020 | 0.142 ± 0.153 | $7.87 \times 10^{-3}$ |
| | mRNA expression level | 6.892 ± 5.050 | 1.259 ± 0.670 | $1.32 \times 10^{-10}$ |
| SOX17 | DNA methylation level | 0.125 ± 0.042 | 0.285 ± 0.174 | $7.01 \times 10^{-6}$ |
| | mRNA expression level | 6.959 ± 4.334 | 4.879 ± 4.372 | $4.03 \times 10^{-2}$ |

Thus, it was demonstrated that the DNA methylation alternations occurring at the precancerous stage determined the aggressiveness of renal cell carcinomas and the prognosis of the patients through alterations of gene expression levels.

(Example 5)

<Detection of DNA Methylation Level in Renal Cell Carcinomas, using Mass Spectrometer>

The effectiveness of the DNA methylation level detection at the CpG sites of the 17 genes was verified by a MassARRAY method, a different methylated DNA detection method from the Infinium assay.

The MassARRAY method is a method for detecting a difference in molecular weight between methylated DNA fragments and unmethylated DNA fragments using a mass spectrometer after a bisulfate-treated DNA is amplified and transcribed into RNA, which is further base-specifically cleaved with an RNase.

First, MassARRAY primers were designed using EpiDesigner (manufactured bySEQUENOM, Inc., primer design software for MassARRAY) for CpG islands containing the CpG sites that are the probe site of the Infinium array.

Note that the PCR target sequence in MassARRAY is somewhat long: approximately 100 to 500 bp. Accordingly, DNA methylation levels of a large number of CpG sites around the CpG sites that are the probe site of the Infinium array can be evaluated together.

Moreover, in order to exclude the influence of a bias in PCR, a test was run in such a manner as to average combinations of three DNA polymerases with conditions of approximately four annealing temperatures per primer set, so that optimum PCR conditions for favorable quantification were determined.

Then, it was confirmed that the adopted PCR conditions were favorable in terms of the quantification for all the CpG sites contained in the PCR target sequence and to be analyzed. The MassARRAY analysis was performed on 88 specimens of CIMP-negative renal cell carcinomas and 14 specimens of CIMP-positive renal cell carcinomas.

Specifically, first, in the same manner as in the above-described Infinium assay, a genomic DNA was extracted from each sample and converted with bisulfate. Then, the resultant was amplified by PCR, and an *in vitro* transcription reaction was carried out. Subsequently, the obtained RNA was specifically cleaved at a uracil site with RNAse A, thereby forming fragments differed from one another in length according to the presence or absence of the methylation on the genomic DNA of each sample. Thereafter, the obtained RNA fragments were subjected to MALDI-TOF MAS (manufactured by SEQUENOM, Inc., MassARRAY Analyzer 4) capable of detecting a difference in mass of a single base to conduct the mass analysis. The obtained mass analysis result was aligned with a reference sequence using analysis software (EpiTYPER, manufactured by SEQUENOM, Inc.). The methylation level was calculated from a mass ratio between the RNA fragment derived from the methylated DNA and the RNA fragment derived from the unmethylated DNA.

Tables 17 and 18 and Sequence Listing show the sequences of the primers used in this analysis and the sequences of PCR products amplified using the primer sets. Figs. 18 to 23 show some of the obtained result.

[Table 17]

| Target gene name_ primer set name | Size of PCR product | Forward primer | Reverse primer | Target sequence (sequence of PCR product) |
|---|---|---|---|---|
| SLC13A5_MA_10 | 500 | aggaagagagGAAGGATTTGAATTTGGAGATATAGTTT | cagtaatacgactcactatagggagaaggctAAAAAACCCAAAAACCTACAAAAAA | SEQ ID NO: 1 |
| SLC13A5_MA_13 | 463 | aggaagagagTTTTTTTGGGTTTTGAAGGGTT | cagtaatacgactcactatagggagaaggctTTATATCCCTTCCTCTCTAAAACTCC | SEQ ID NO: 2 |
| SLC13A5_MA_15 | 384 | aggaagagagTTTTTTTTGTTTTAGGGGTTGT | cagtaatacgactcactatagggagaaggctCCACCAACATAAATAAAACTCCCC | SEQ ID NO: 3 |
| FAM150A_MA_14 | 455 | aggaagagagGGGAGGATTTAGTAGGGTAATTGT | cagtaatacgactcactatagggagaaggctTTTCACCTAAAAAAACACTAAAACC | SEQ ID NO: 4 |
| GRM6_MA_8 | 188 | aggaagagagGGTTTAGGATAAGTTTGTGATAGATG | cagtaatacgactcactatagggagaaggctAAAACAAAAAAACAAACCCAAAAAT | SEQ ID NO: 5 |
| ZFP42_MA_2 | 196 | aggaagagagGAGTTGATGGGTGGTTGTAGTTT | cagtaatacgactcactatagggagaaggctCCCATTTAAAAAAATTCCATAAAACAAA | SEQ ID NO: 6 |
| ZNF154_ MA_5 5 | 279 | aggaagagagGGTGAATATATTTTAGAGAAGTTAAAATGG | cagtaatacgactcactatagggagaaggctTCCCTCCACTACCCTAAAACTTAAA | SEQ ID NO: 7 |
| RIMS4_MA_9 | 402 | aggaagagagGGAGTTTTAGTTTATGAGGGAAGGA | cagtaatacgactcactatagggagaaggctAAACCCCAAAATCTCCAAAATAC | SEQ ID NO: 8 |

[Table 18]

| Target gene name_ primer set name | Size of PCR product | Forward primer | Reverse primer | Target sequence (sequence of PCR product) |
|---|---|---|---|---|
| TRH_MA_8 | 414 | aggaagagagAATAGATTTTTAGAGGTGGTGTAGAAA | cagtaatacgactcactatagggagaaggctAAAAAACTCCCTTTCCAATACTCC | SEQ ID NO:9 |
| ZNF540_MA_17 | 463 | aggaagagagGGGTAGGGTAGAATTAGGTTAAAGAAA | cagtaatacgactcactatagggagaaggctACTAAAATCAATAACCCCCAAAAAA | SEQ ID NO:10 |
| PCDHAC1_ MA_5 | 362 | aggaagagagTGGTAGTTTTTGGGATATAAGAGGG | cagtaatacgactcactatagggagaaggctAAACTACCCAAATCTTAACCTCCAC | SEQ ID NO:11 |
| PRAC_MA_2 | 264 | aggaagagagGGTGAAAGTTTGTTGTTTATTTTTTTT | cagtaatacgactcactatagggagaaggctCAAACTAAATTCTAATCCCCACCTT | SEQ ID NO:1 2 |
| ZNF671_MA_8 | 428 | aggaagagagTGGGATATAGGGGTTGTAGGTATTT | cagtaatacgactcactatagggagaaggctATAAAAACCACACTCTACCCACAAA | SEQ ID NO:1 3 |
| WNT3A_MA_9 | 348 | aggaagagagGTTTATTTGGTAATGAGGGGTTGTT | cagtaatacgactcactatagggagaaggctTTCCTCAATCTTAAACATCTCAAAA | SEQ ID NO:1 4 |
| KHDRBS2_MA_19 (rev) | 422 | aggaagagagTTTGGTATTATTATTAATGAGTGGTTGG | cagtaatacgactcactatagggagaaggctAACAAATCCTACCTTCTACCAAAAAA | SEQ ID NO:1 5 |

(continued)

| Target gene name_ primer set name | Size of PCR product | Forward primer | Reverse primer | Target sequence (sequence of PCR product) |
|---|---|---|---|---|
| ASCL2_MA_8 | 339 | aggaagagagGTTAATAAAGTTGGGTTTTTGTTGG | cagtaatacgactcactatagggagaaggctAATACAAACCTCCAAACCCTCC | SEQ ID NO:1 6 |

As apparent from the results shown in Figs. 18 to 23, it was verified similarly to the analysis result using the Infinium array above that it was possible to distinguish between renal cell carcinomas belonging to Cluster B (CIMP-positive group) of unfavorable prognosis and renal cell carcinomas belonging to Cluster A (CIMP-negative group) of favorable prognosis by detecting DNA methylation levels of the CpG sites, in all the regions of the MassARRAY analysis target. Further, the MassARRAY analysis revealed that the hypermethylation status in the CIMP-positive group continued not only at one CpG site but also in all the region of the CpG island containing the same (for example, a region of around 1500 bp of the Infinium-probe CpG site).

Thus, it was revealed that one CpG site in a region where strong silencing occurred by the hypermethylation status of all the promoter region had been identified in Example 4; in other words, it was revealed that detecting a DNA methylation level of not only the aforementioned 18 CpG sites but also at least one CpG site located on CpG islands of the 17 genes made it possible to detect an unfavorable prognostic risk of renal cell carcinoma.

Further, the DNA methylation levels at 312 CpG sites of 14 genes in the 14 cases already classified into the CIMP-positive group by the above-described Infinium assay and of the 88 CIMP-negative cases were quantified by the MassARRAY method. Then, based on the result, a receiver operating characteristic (ROC) analysis was performed, and "sensitivity (positive rate)", "specificity", and "1-specificity (false-positive rate)" were obtained which are used when the CIMP-positive group is distinguished from the CIMP-negative group on the basis of each CpG site alone. Further, a ROC curve was created from the obtained values of these, and an AUC (area under the curve, the area under the ROC curve) was calculated. Moreover, a cutoff value (diagnostic threshold) at which"sensitivity+specificity" was the maximum was set for each CpG site. Tables 19 to 27 show the obtained results of the CpG sites quantitatively analyzed by the MassARRAY analysis. Note that, in Tables 19 to 27, multiple CpG sites which are close to each other, and whose DNA methylation levels are measured together due to the feature of the MassARRAY method, are collectively shown as a single unit. Additionally, in these tables, "target gene name_primer set name_CpG site" indicates the order of CpG sites in PCR products amplified using the primer sets shown in Tables 17 and 18. Note that, in Table 23, SLC13A5_10_CpG_44 and SLC13A5_13_CpG_1 respectively indicate the 44th CpG site and the 1st CpG site in the region amplified by different primer sets, but their positions on the genome (positions on NCBI database Genome Build 37) are at the same CpG site: position 6617077 on chromosome 17.

[Table 19]

| Target gene name_primer set name | CpG unit | AUC value | Cutoff value | Sensitivity | Specificity | 1-specificity |
|---|---|---|---|---|---|---|
| FAM150A_MA_14 | CpG_8 | 0.936 | 0.108 | 0.833 | 0.941 | 0.059 |
| | CpG_9.10 | 0.947 | 0.074 | 0.917 | 0.838 | 0.162 |
| | CpG_ 13.14.15 | 0.912 | 0.108 | 0.833 | 0.853 | 0.147 |
| | CpG_16 | 0.898 | 0.098 | 0.833 | 0.897 | 0.103 |
| | CpG_18.19 | 0.945 | 0.183 | 1000 | 0.838 | 0.162 |
| | CpG_20 | 0.667 | 0.508 | 0.667 | 0.721 | 0.279 |
| | CpG_21.22 | 0.934 | 0.338 | 0.917 | 0.912 | 0.088 |
| | CpG_26 | 0.968 | 0.307 | 0.833 | 0.985 | 0.015 |
| | CpG_21.28 | 0.939 | 0.255 | 0.917 | 0.941 | 0.059 |
| | CpG_29 | 0.911 | 0.055 | 0.917 | 0.926 | 0.074 |
| | CpG_30 | 0.968 | 0.301 | 0.833 | 0.985 | 0.015 |
| | CpG_31 | 0.925 | 0.072 | 0.833 | 0.941 | 0.059 |

(continued)

| Target gene name_primer set name | CpG unit | AUC value | Cutoff value | Sensitivity | Specificity | 1-specificity |
|---|---|---|---|---|---|---|
| | CpG_32 | 0.895 | 0.223 | 0.833 | 0.956 | 0.044 |
| | CpG_37.38.39 | 0.912 | 0.227 | 0.750 | 0.971 | 0.029 |
| | CpG_40 | 0.892 | 0.265 | 0.917 | 0.868 | 0.132 |
| | CpG_41.42 | 0.939 | 0.255 | 0.917 | 0.941 | 0.059 |
| | CpG_43 | 0.881 | 0.195 | 0.667 | 0.971 | 0.029 |
| GRM6_MA_8 | CpG_1.2 | 0.903 | 0.232 | 0.786 | 0.932 | 0.068 |
| | CpG_45 | 0.931 | 0.115 | 0.929 | 0.830 | 0.170 |
| ZFP42_MA_2 | CpG_1.2 | 0.871 | 0.295 | 0.714 | 0.955 | 0.045 |
| | CpG_3 | 0.917 | 0.202 | 0.786 | 0.943 | 0.057 |
| | CpG_4 | 0.933 | 0.135 | 0.929 | 0.841 | 0.159 |
| | CpG_5 | 0.928 | 0.133 | 0.929 | 0.886 | 0.114 |
| | CpG_6 | 0.888 | 0.408 | 0.786 | 0.898 | 0.102 |
| | CpG_78 | 0.932 | 0.345 | 0.857 | 0.909 | 0.091 |

[Table 20]

| Target gene name_primer set name | CpG unit | AUC value | Cutoff value | Sensitivity | Specificity | 1 -specificity |
|---|---|---|---|---|---|---|
| ZNF540_MA_17 | CpG_1 | 0.875 | 0.415 | 0.833 | 0.828 | 0.172 |
| | CpG_2 | 0.882 | 0.509 | 1.000 | 0.707 | 0.293 |
| | CpG_34 | 0.928 | 0.222 | 0.833 | 0.897 | 0.103 |
| | CpG_5 | 0.882 | 0.415 | 0.833 | 0.828 | 0.172 |
| | CpG_6 | 0.983 | 0.410 | 1.000 | 0.983 | 0.017 |
| | CpG_7.8 | 0.897 | 0.304 | 0.833 | 0.931 | 0.069 |
| | CpG_9 | 0.960 | 0.357 | 1.000 | 0.931 | 0.069 |
| | CpG_10.11 | 0.991 | 0.364 | 1.000 | 0.966 | 0.034 |
| | CpG_12.13 | 0.927 | 0.477 | 1.000 | 0.810 | 0.190 |
| | CpG_14 | 0.848 | 0.344 | 0.833 | 0.914 | 0.086 |
| | CpG_15 | 0.920 | 0.282 | 1.000 | 0.810 | 0.190 |
| | CpG_16.17 | 0.733 | 0.452 | 0.833 | 0.690 | 0.310 |
| | CpG_18 | 0.797 | 0.342 | 0.833 | 0.810 | 0.190 |
| | CpG_20.21 | 0.878 | 0.384 | 0.833 | 0.931 | 0.069 |
| | CpG_22.23 | 0.859 | 0.325 | 0.833 | 0.879 | 0.121 |
| | CpG_24.25 | 0.941 | 0.502 | 0.833 | 0.966 | 0.034 |

(continued)

| Target gene name_primer set name | CpG unit | AUC value | Cutoff value | Sensitivity | Specificity | 1 -specificity |
|---|---|---|---|---|---|---|
| | CpG_26 | 0.928 | 0.378 | 0.833 | 0.897 | 0.103 |
| ZNF154_MA_5 | CpG_1 | 0.956 | 0.133 | 0.929 | 0.909 | 0.091 |
| | CpG_4 | 0.966 | 0.148 | 0.857 | 0.955 | 0.045 |
| | CpG_5.6 | 0.959 | 0.222 | 0.929 | 0.955 | 0.045 |
| | CpG_8 | 0.912 | 0.118 | 1.000 | 0.750 | 0.250 |
| | CpG_9 | 0.825 | 0.162 | 0.929 | 0.682 | 0.318 |
| | CpG_ 11.12 | 0.917 | 0.368 | 0.929 | 0.784 | 0.216 |

[Table 21]

| Target gene name_primer set name | CpG unit | AUC value | Cutoff value | Sensitivity | Specificity | 1-specificity |
|---|---|---|---|---|---|---|
| RIMS4_MA_9 | CpG_1 | 0.779 | 0.102 | 0.833 | 0.728 | 0.272 |
| | CpG_23 | 0.800 | 0.150 | 1.000 | 0.531 | 0.469 |
| | CpG_45 | 0.866 | 0.465 | 0.750 | 0.889 | 0.111 |
| | CpG_6.7.8.9 | 0.846 | 0.307 | 0.833 | 0.765 | 0.235 |
| | CpG_10 | 0.826 | 0.202 | 0.833 | 0.753 | 0.247 |
| | CpG_11 | 0.860 | 0.102 | 0.833 | 0.753 | 0.247 |
| | CpG_13.14 | 0.820 | 0.132 | 0.667 | 0.951 | 0.049 |
| | CpG_15 | 0.913 | 0.102 | 0.833 | 0.877 | 0.123 |
| | CpG_16 | 0.860 | 0.173 | 0.833 | 0.118 | 0.222 |
| | CpG_17 | 0.914 | 0.135 | 0.833 | 0.864 | 0.136 |
| | CpG_18 | 0.737 | 0.248 | 0.750 | 0.815 | 0.185 |
| PCDHAC1_MA_5 | CpG_1 | 0.821 | 0.195 | 0.857 | 0.716 | 0.284 |
| | CpG_2.3 | 0.718 | 0.225 | 0.643 | 0.841 | 0.159 |
| | CpG_4.5 | 0.718 | 0.225 | 0.643 | 0.841 | 0.159 |
| | CpG_6 | 0.899 | 0.135 | 0.929 | 0.716 | 0.284 |
| | CpG_8 | 0.862 | 0.109 | 0.857 | 0.773 | 0.227 |
| | CpG_9 | 0.821 | 0.195 | 0.857 | 0.716 | 0.284 |
| | CpG_16 | 0.821 | 0.079 | 0.929 | 0.614 | 0.386 |
| | CpG_ 17.18.19 | 0.818 | 0.265 | 0.714 | 0.761 | 0.239 |
| | CpG_20.21 | 0.806 | 0.199 | 0.643 | 0.875 | 0.125 |
| | CpG_22.23 | 0.781 | 0.142 | 0.714 | 0.830 | 0.170 |
| | CpG_24 | 0.797 | 0.106 | 0.929 | 0.580 | 0.420 |
| | CpG_ 25.26.27 | 0.821 | 0.227 | 0.643 | 0.898 | 0.102 |
| | CpG_28 | 0.760 | 0.214 | 0.714 | 0.784 | 0.216 |
| | CpG_29 | 0.845 | 0.165 | 1.000 | 0.636 | 0.364 |

[Table 22]

| Target gene name_primer set name | CpG unit | AUC value | Cutoff value | Sensitivity Specificity | | 1-specificity |
|---|---|---|---|---|---|---|
| PRAC_MA_2 | CpG_2.3 | 0.943 | 0.415 | 0.857 | 0.943 | 0.057 |
| | CpG_4 | 0.915 | 0.393 | 0.786 | 0.932 | 0.068 |
| | CpG_6 | 0.888 | 0.233 | 0.857 | 0.818 | 0.182 |
| | CpG_7 | 0.944 | 0.350 | 0.929 | 0.864 | 0.136 |
| | CpG_8 | 0.957 | 0.407 | 0.929 | 0.898 | 0.102 |
| TRH_MA_8 | CpG_2.3.4.5 | 0.903 | 0.158 | 0.846 | 0.795 | 0.205 |
| | CpG_6 | 0.857 | 0.278 | 0.846 | 0.784 | 0.216 |
| | CpG_11.12 | 0.973 | 0.308 | 1.000 | 0.886 | 0.114 |
| | CpG_13 | 0.917 | 0.172 | 0.846 | 0.841 | 0.159 |
| | CpG_25 | 0.902 | 0.210 | 0.846 | 0.898 | 0.102 |
| | CpG_26 | 0.810 | 0.107 | 0.692 | 0.852 | 0.148 |
| | CpG_27.28.29 | 0.950 | 0.258 | 0.846 | 0.932 | 0.068 |
| | CpG_3031 | 0.943 | 0.175 | 0.923 | 0.909 | 0.091 |
| | CpG_32 | 0.902 | 0.175 | 0.846 | 0.932 | 0.068 |
| | CpG_33.34 | 0.935 | 0.173 | 0.923 | 0.852 | 0.148 |
| | CpG_35 | 0.952 | 0.110 | 0.923 | 0.920 | 0.080 |
| | CpG_36 | 0.917 | 0.172 | 0.846 | 0.841 | 0.159 |
| | CpG_37 | 0.921 | 0.055 | 1.000 | 0.761 | 0.239 |
| | CpG_38 | 0.872 | 0.292 | 0.846 | 0.818 | 0.182 |
| | CpG_39 | 0.943 | 0.115 | 1.000 | 0.886 | 0.114 |
| | CpG_40 | 0.967 | 0.066 | 1.000 | 0.875 | 0.125 |
| | CpG_41 | 0.925 | 0.187 | 0.846 | 0.920 | 0.080 |
| | CpG_42 | 0.858 | 0.402 | 0.769 | 0.943 | 0.057 |
| | CpG_43.44 | 0.867 | 0.110 | 0.769 | 0.898 | 0.102 |

[Table 23]

| Target gene name_primer set name | CpG unit | AUC value | Cutoff value | Sensitivity | Specificity | 1-specificity |
|---|---|---|---|---|---|---|
| SLC13A5_MA_10 | CpG_1.2 | 0.877 | 0.147 | 0.786 | 0.898 | 0.1 02 |
| | CpG_3.4.5 | 0.940 | 0.243 | 0.929 | 0.830 | 0.170 |
| | CpG_6.7 | 0.791 | 0.222 | 0.786 | 0.795 | 0.205 |
| | CpG_9.10.11 | 0.906 | 0145 | 0.857 | 0.875 | 0.125 |
| | CpG_12 | 0.983 | 0.075 | 0.929 | 0.966 | 0.034 |
| | CpG_13 | 0.928 | 0.040 | 0.929 | 0.875 | 0.125 |
| | CpG_14.15 | 0.946 | 0.205 | 0.857 | 0.898 | 0.102 |
| | CpG_21 | 0.983 | 0.185 | 1.000 | 0.943 | 0.057 |
| | CpG_22.23 | 0.951 | 0.233 | 1.000 | 0.886 | 0.114 |

(continued)

| Target gene name_primer set name | CpG unit | AUC value | Cutoff value | Sensitivity | Specificity | 1-specificity |
|---|---|---|---|---|---|---|
| | CpG 24.25.26 | 0.954 | 0.148 | 1.000 | 0.875 | 0.25 |
| | CpG_27 | 0.896 | 0.087 | 0.857 | 0.807 | 0.193 |
| | CpG_28.29 | 0.900 | 0.178 | 0.929 | 0.864 | 0.136 |
| | CpG_30.31 | 0.951 | 0.233 | 1.000 | 0.886 | 0.114 |
| | CpG_32.33 | 0.834 | 0.312 | 0.857 | 0.761 | 0.239 |
| | CpG_34.35 | 0.927 | 0.144 | 0.929 | 0.818 | 0.182 |
| | CpG_36.31 | 0.841 | 0.275 | 0.857 | 0.830 | 0.170 |
| | CpG_ 40.41.42.43 | 0.942 | 0.258 | 1.000 | 0.830 | 0.170 |
| | CpG_44 | 0.949 | 0.138 | 0.857 | 0.955 | 0.045 |
| SLC13A5_MA_13 | CpG_1 | 0.927 | 0.155 | 0.800 | 0.977 | 0.023 |
| | CpG_2 | 0.930 | 0.318 | 1.000 | 0.864 | 0.136 |
| | CpG_6.7.8.9 | 0.864 | 0.343 | 0.900 | 0.761 | 0.239 |
| | CpG_15.16 | 0.916 | 0.278 | 0.800 | 0.898 | 0.102 |
| | CpG_17.18 | 0.931 | 0.267 | 1.000 | 0.795 | 0.205 |

[Table 24]

| Target gene name_primer set name | CpG unit | AUC value | Cutoff value | Sensitivity | Specificity | 1-specificity |
|---|---|---|---|---|---|---|
| SLC1 3A5_MA_13 | CpG_19.20 | 0.930 | 0.328 | 1.000 | 0.864 | 0.136 |
| | CpG_21 | 0.886 | 0.312 | 0.900 | 0.841 | 0.159 |
| | CpG_22 | 0.780 | 0.202 | 0.800 | 0.750 | 0.250 |
| | CpG_24.25 | 0.869 | 0.185 | 1.000 | 0.693 | 0.307 |
| | CpG_26 | 0.944 | 0.228 | 1.000 | 0.852 | 0.148 |
| | CpG_27 | 0.893 | 0.202 | 1.000 | 0.727 | 0.273 |
| | CpG_ 28.29.30 | 0.877 | 0.295 | 0.800 | 0.943 | 0.057 |
| | CpG_31 | 0.893 | 0.407 | 1.000 | 0.818 | 0.182 |
| | CpG_32.33 | 0.914 | 0.288 | 1.000 | 0.739 | 0.261 |
| | CpG_35 | 0.913 | 0.392 | 0.900 | 0.898 | 0.102 |
| | CpG_36.37 | 0.934 | 0.238 | 1.000 | 0.773 | 0.227 |

(continued)

| Target gene name_primer set name | CpG unit | AUC value | Cutoff value | Sensitivity | Specificity | 1-specificity |
|---|---|---|---|---|---|---|
| SLC13A5_MA_15 | CpG_1.2 | 0.879 | 0.243 | 1.000 | 0.672 | 0.328 |
| | CpG_3 | 0.942 | 0.222 | 1.000 | 0.866 | 0.134 |
| | CpG_4 | 0.875 | 0.278 | 0.778 | 0.910 | 0.090 |
| | CpG_5.6.7 | 0.936 | 0.300 | 0.778 | 1.000 | 0.000 |
| | CpG_8 | 0.908 | 0.388 | 0.889 | 0.896 | 0.104 |
| | CpG_9.10 | 0.927 | 0.377 | 0.889 | 0.896 | 0.104 |
| | CpG_12 | 0.885 | 0.247 | 1.000 | 0.746 | 0.254 |
| | CpG_13.14 | 0.935 | 0.284 | 0.889 | 0.896 | 0.104 |
| | CpG_16 | 0.680 | 0.820 | 0.556 | 0.806 | 0.194 |
| | CpG_17 | 0.681 | 0.463 | 0.778 | 0.567 | 0.433 |
| | CpG_18 | 0.681 | 0.463 | 0.778 | 0.567 | 0.433 |
| | CpG_19 | 0.774 | 0.543 | 1.000 | 0.627 | 0.373 |
| | CpG_20.21 | 0.942 | 0.685 | 0.889 | 0.881 | 0.119 |

[Table 25]

| Target gene name_primer set name | CpG unit | AUC value | Cutoff value | Sensitivity | Specificity | 1-specificity |
|---|---|---|---|---|---|---|
| ZNF671_ MA_8 | CpG_2 | 0.892 | 0.157 | 0.643 | 0.989 | 0.011 |
| | CpG_3 | 0.871 | 0.128 | 0.857 | 0.724 | 0.276 |
| | CpG_4 | 0.906 | 0.048 | 0.929 | 0.713 | 0.287 |
| | CpG_6.7.8 | 0.893 | 0.058 | 0.857 | 0.736 | 0.264 |
| | CpG_9 | 0.888 | 0.055 | 0.857 | 0.736 | 0.264 |
| | CpG_10 | 0.954 | 0.152 | 0.857 | 0.897 | 0.103 |
| | CpG_11.12.13 | 0.835 | 0.050 | 0.857 | 0.690 | 0.310 |
| | CpG_14 | 0.926 | 0.062 | 1.000 | 0.747 | 0.253 |
| | CpG_15 | 0.871 | 0.128 | 0.857 | 0.724 | 0.276 |
| | OpG_16.17 | 0.893 | 0.080 | 0.643 | 0.977 | 0.023 |
| | CpG_18 | 0.895 | 0.082 | 0.786 | 0.816 | 0.184 |
| | CpG_20 | 0.927 | 0.105 | 0.929 | 0.759 | 0.241 |
| | CpG_21.22.23 | 0.812 | 0.165 | 0.786 | 0.920 | 0.080 |
| | CpG_24.25 | 0.898 | 0.228 | 0.714 | 0.920 | 0.080 |
| | CpG_26 | 0.965 | 0.105 | 1.000 | 0.885 | 0.115 |
| | CpG_27 | 0.892 | 0.157 | 0.643 | 0.989 | 0.011 |
| | CpG_28 | 0.954 | 0.152 | 0.857 | 0.897 | 0.103 |
| | CpG_29 | 0.954 | 0.152 | 0.857 | 0.897 | 0.103 |
| | CpG_30 | 0.871 | 0.128 | 0.857 | 0.724 | 0.276 |
| | CpG_31 | 0.951 | 0.105 | 0.857 | 0.920 | 0.080 |

(continued)

| Target gene name_primer set name | CpG unit | AUC value | Cutoff value | Sensitivity | Specificity | 1-specificity |
|---|---|---|---|---|---|---|
| | CpG_33 | 0.910 | 0.110 | 0.786 | 0.920 | 0.080 |

[Table 26]

| Target gene name_primer set name | CpG unit | AUC value | Cutoff value | Sensitivity | Specificity | 1-specificity |
|---|---|---|---|---|---|---|
| WNT3A_MA_9 | CpG_1 | 0.770 | 0.035 | 0.571 | 0.943 | 0.057 |
| | CpG_2.3 | 0.838 | 0.328 | 0.786 | 0.864 | 0.136 |
| | CpG_4.5.6 | 0.736 | 0.178 | 0.786 | 0.636 | 0.364 |
| | CpG_7 | 0.943 | 0.225 | 0.857 | 0.886 | 0.114 |
| | CpG_8 | 0.943 | 0.225 | 0.857 | 0.886 | 0.114 |
| | CpG_9 | 0.943 | 0.225 | 0.857 | 0.886 | 0.114 |
| | CpG_10 | 0.869 | 0.158 | 0.857 | 0.807 | 0.193 |
| | CpG_11 | 0.831 | 0.128 | 0.929 | 0.784 | 0.216 |
| | CpG_12 | 0.849 | 0.127 | 0.857 | 0.818 | 0.182 |
| KHDRBS2_MA_19(rev) | CpG_1 | 0.824 | 0.115 | 0.786 | 0.810 | 0.190 |
| | CpG_5 | 0.767 | 0.185 | 0.857 | 0.655 | 0.345 |
| | CpG_6.7.8 | 0.789 | 0.265 | 0.714 | 0.738 | 0.262 |
| | CpG_12 | 0.797 | 0.195 | 0.786 | 0.750 | 0.250 |
| | CpG_13.14 | 0.721 | 0.265 | 0.857 | 0.619 | 0.381 |
| | CpG_16 | 0.762 | 0.265 | 0.714 | 0.786 | 0.214 |
| | CpG_17.18 | 0.824 | 0.215 | 0.857 | 0.786 | 0.214 |
| | CpG_19 | 0.762 | 0.265 | 0.714 | 0.786 | 0.214 |
| | CpG_ 21.22.23.24 | 0.654 | 0.275 | 0.571 | 0.702 | 0.298 |
| | CpG_25.26 | 0.824 | 0.215 | 0.857 | 0.786 | 0.214 |
| | CpG_27 | 0.836 | 0.195 | 0.857 | 0.714 | 0.286 |
| | CpG_28.29 | 0.759 | 0.265 | 0.714 | 0.750 | 0.250 |
| | CpG_ 32.33.34.35 | 0.701 | 0.225 | 0.786 | 0.643 | 0.357 |
| | CpG_36 | 0.668 | 0.195 | 0.643 | 0.643 | 0.357 |
| | CpG_37.38 | 0.773 | 0.150 | 0.929 | 0.583 | 0.417 |
| | CpG_ 39.40.41 | 0.673 | 0.195 | 0.857 | 0.488 | 0.512 |

[Table 27]

| Target gene name_primer set name | CpG unit | AUC value | Cutoff value | Sensitivity | Specificity | 1-specificity |
|---|---|---|---|---|---|---|
| ASCL2_MA_8 | CpG_7 | 0.724 | 0.210 | 0.714 | 0.821 | 0.179 |
| | CpG_8 | 0.886 | 0.230 | 0.929 | 0.869 | 0.131 |
| | CpG_9.10 | 0.907 | 0.300 | 0.929 | 0.821 | 0.179 |
| | CpG_11 | 0.849 | 0.235 | 0.857 | 0.857 | 0.143 |
| | CpG_12 | 0.811 | 0.325 | 0.857 | 0.821 | 0.179 |
| | CpG_13 | 0.857 | 0.245 | 0.857 | 0.857 | 0.143 |
| | CpG_14 | 0.759 | 0.045 | 0.643 | 0.905 | 0.095 |
| | CpG_15 | 0.827 | 0.085 | 0.857 | 0.881 | 0.119 |
| | CpG_ 16.17 | 0.866 | 0.255 | 0.857 | 0.869 | 0.131 |
| | CpG_ 21.22 | 0.888 | 0.435 | 0.929 | 0.845 | 0.155 |
| | CpG_26 | 0.502 | 0.495 | 0.429 | 0.690 | 0.310 |
| | CpG_27 | 0.697 | 0.255 | 0.857 | 0.560 | 0.440 |

As apparent from the results shown in Tables 19 to 27, it was found that a large number of CpG sites havi ng a high diagnostic ability existed in each CpG island besides the Infinium-probe CpG sites. Specifically, t he number of CpG sites having an AUC > 0.9 was 141 site s, and the number of CpG sites having an AUC > 0.95 was 32 sites.

Moreover, in the MassARRAY method, one measurement value is obtained from consecutive CpG sites such as CGCGCG, for example, "FAM150A_14_CpG_13.14.15", as a whole. Accordingly, the 141 sites having an AUC > 0.9 correspond to 90 measurement values (units) based on the AUC calculation. Similarly, the 32 sites having an AUC > 0.95 correspond to 23 measurement values (units) in terms of the measurement value based on the AUC calculation.

Furthermore, as apparent from the result shown in Fig. 24, it was possible to clearly discriminate the CIMP-positive group from the CIMP-negative group by using the 23 CpG units (23 measurement values) having an AUC larger than 0.95 as the indicator.

[Industrial Applicability]

As has been described above, the present invention makes it possible to clearly classify renal cell carcinomas of unfavorable prognosis (CIMP-positive renal cell carcinomas) and relatively favorable renal cell carcinomas by detecting a DNA methylation level at at least one CpG site of the 17 genes (FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2).

Since the difference in the DNA methylation level between the unfavorable prognosis group and the favorable group is large, such a difference can be easily detected by a PCR method and the like (for example, methylation-specific quantitative PCR, COBRA) already widespread in examination rooms in hospitals and other places. Moreover, a genomic DNA for prognosis can be abundantly extracted from specimens resulting from renal cell carcinoma surgeries without involving unnecessary invasion to patients. Thus, the method for detecting an unfavorable prognostic risk of renal cell carcinoma of the present invention is useful in the clinical field as the method directed to improve the clinical outcome.

[Sequence Listing Free Text]

SEQ ID NO: 17
<223> Artificially synthesized primer sequence (SLC13A5_MA_10 forward primer used for MassARRAY assay)
SEQ ID NO: 18
<223> Artificially synthesized primer sequence (SLC13A5_MA_10 reverse primer used for MassARRAY assay)
SEQ ID NO: 19
<223> Artificially synthesized primer sequence (SLC13A5_MA_13 forward primer used for MassARRAY assay)

SEQ ID NO: 20
<223> Artificially synthesized primer sequence (SLC13A5_MA_13 reverse primer used for MassARRAY assay)
SEQ ID NO: 21
<223> Artificially synthesized primer sequence (SLC13A5_MA_15 forward primer used for MassARRAY assay)
SEQ ID NO: 22
<223> Artificially synthesized primer sequence (SLC13A5_MA_15 reverse primer used for MassARRAY assay)
SEQ ID NO: 23
<223> Artificially synthesized primer sequence (FAM150A_MA_14 forward primer used for MassARRAY assay)
SEQ ID NO: 24
<223> Artificially synthesized primer sequence (FAM150A_MA_14 reverse primer used for MassARRAY assay)
SEQ ID NO: 25
<223> Artificially synthesized primer sequence (GRM6_MA_8 forward primer used for MassARRAY assay)
SEQ ID NO: 26
<223> Artificially synthesized primer sequence (GRM6_MA_8 reverse primer used for MassARRAY assay)
SEQ ID NO: 27
<223> Artificially synthesized primer sequence (ZFP42_MA_2 forward primer used for MassARRAY assay)
SEQ ID NO: 28
<223> Artificially synthesized primer sequence (ZFP42_MA_2 reverse primer used for MassARRAY assay)
SEQ ID NO: 29
<223> Artificially synthesized primer sequence (ZFP42_ MA_5 forward primer used for MassARRAY assay)
SEQ ID NO: 30
<223> Artificially synthesized primer sequence (ZFP42_MA_5 reverse primer used for MassARRAY assay)
SEQ ID NO: 31
<223> Artificially synthesized primer sequence (RIMS4_MA_9 forward primer used for MassARRAY assay)
SEQ ID NO: 32
<223> Artificially synthesized primer sequence (RIMS4_MA_9 reverse primer used for MassARRAY assay)
SEQ ID NO: 33
<223> Artificially synthesized primer sequence (TRH_MA_8 forward primer used for MassARRAY assay)
SEQ ID NO: 34
<223> Artificially synthesized primer sequence (TRH_MA_8 reverse primer used for MassARRAY assay)
SEQ ID NO: 35
<223> Artificially synthesized primer sequence (ZNF540_MA_17 forward primer used for MassARRAY assay)
SEQ ID NO: 36
<223> Artificially synthesized primer sequence (ZNF540_MA_17 reverse primer used for MassARRAY assay)
SEQ ID NO: 37
<223> Artificially synthesized primer sequence (PCDHAC1_MA_5 forward primer used for MassARRAY assay)
SEQ ID NO: 38
<223> Artificially synthesized primer sequence (PCDHAC1_MA_5 reverse primer used for MassARRAY assay)
SEQ ID NO: 39
<223> Artificially synthesized primer sequence (PRAC_MA_2 forward primer used for MassARRAY assay)
SEQ ID NO: 40
<223> Artificially synthesized primer sequence (PRAC_MA_2 reverse primer used for MassARRAY assay)
SEQ ID NO: 41
<223> Artificially synthesized primer sequence (ZNF671_MA_8 forward primer used for MassARRAY assay)
SEQ ID NO: 42
<223> Artificially synthesized primer sequence (ZNF671_MA_8 reverse primer used for MassARRAY assay)
SEQ ID NO: 43
<223> Artificially synthesized primer sequence (WNT3A_MA_9 forward primer used for MassARRAY assay)
SEQ ID NO: 44
<223> Artificially synthesized primer sequence (WNT3A_MA_9 reverse primer used for MassARRAY assay)
SEQ ID NO: 45
<223> Artificially synthesized primer sequence (KHDRBS2_MA_19(rev) forward primer used for MassARRAY assay)
SEQ ID NO: 46
<223> Artificially synthesized primer sequence (KHDRBS2_MA_19(rev) reverse primer used for MassARRAY assay)
SEQ ID NO: 47
<223> Artificially synthesized primer sequence (ASCL2_MA_8 forward primer used for MassARRAY assay)

SEQ ID NO: 48
<223> Artificially synthesized primer sequence (ASCL2_MA_8 reverse primer used for MassARRAY assay)
SEQ ID NO: 49
<223> Artificially synthesized primer sequence (ZFP42 forward primer for pyrosequencing)
SEQ ID NO: 50
<223> Artificially synthesized primer sequence (ZFP42 reverse primer for pyrosequencing)
SEQ ID NO: 51
<223> Artificially synthesized primer sequence (ZFP42 sequencing primer for pyrosequencing)
SEQ ID NO: 52
<223> Artificially synthesized primer sequence (ZFP154 forward primer for pyrosequencing)
SEQ ID NO: 53
<223> Artificially synthesized primer sequence (ZFP154 reverse primer for pyrosequencing)
SEQ ID NO: 54
<223> Artificially synthesized primer sequence (ZFP154 sequencing primer for pyrosequencing)
SEQ ID NO: 55
<223> Artificially synthesized primer sequence (ZFP540 forward primer for pyrosequencing)
SEQ ID NO: 56
<223> Artificially synthesized primer sequence (ZFP540 reverse primer for pyrosequencing)
SEQ ID NO: 57
<223> Artificially synthesized primer sequence (ZFP540 sequencing primer for pyrosequencing)

## Claims

**1.** A method for detecting an unfavorable prognostic risk of renal cell carcinoma, the method comprising the following steps (a) to (c):

(a) a step of preparing a genomic DNA derived from a kidney tissue of a subject;
(b) a step of detecting a DNA methylation level of at least one CpG site of a gene selected from the gene group consisting of FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5, and NKX6-2 in the genomic DNA prepared in the step (a); and
(c) a step of determining whether or not the subject is classified into an unfavorable prognosis group according to the DNA methylation level detected in the step (b).

**2.** The method according to claim 1, wherein the step (b) is a step of treating the genomic DNA prepared in the step (a) with bisulfite and detecting a DNA methylation level of the CpG site.

**3.** The method according to claim 1 or 2 wherein the detection comprises using an oligonucleotide according to any one of the following (a) and (b), which has a length of at least 12 bases:

(a) an oligonucleotide that is a pair of primers designed to flank at least one CpG site of a gene selected from the gene group; and
(b) an oligonucleotide that is any one of a primer and a probe capable of hybridizing to a nucleotide comprising at least one CpG site of a gene selected from the gene group.

## Patentansprüche

**1.** Verfahren zum Nachweis von einem ungünstigen prognostischen Risiko eines Nierenzellkarzinoms, wobei das Verfahren die folgenden Schritte (a) bis (c) umfasst:

(a) ein Schritt der Herstellung von genomischer DNA, die von einem Nierengewebe eines Subjekts stammt;
(b) ein Schritt des Nachweises eines DNA-Methylierungsniveaus von mindestens einem CpG-Ort eines Gens, ausgewählt aus der Gengruppe bestehend aus FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5 und NKX6-2 in der genomischen DNA, die in dem Schritt (a) hergestellt wurde; und
(c) ein Schritt des Bestimmens, ob das Subjekt einer Gruppe ungünstigen prognostischen Risikos zugeordnet ist oder nicht, gemäß dem DNA-Methylierungsniveau, das in Schritt (b) nachgewiesen wurde.

2. Verfahren nach Anspruch 1, wobei der Schritt (b) ein Schritt des Behandelns der genomischen DNA, die in dem Schritt (a) hergestellt wurde, mit Bisulfit und des Nachweises eines DNA-Methylierungsniveaus des CpG-Orts ist.

3. Verfahren nach Anspruch 1 oder 2, wobei der Nachweis das Verwenden eines Oligonukleotids gemäß einem der folgenden Punkte (a) und (b) umfasst, das eine Länge von mindestens 12 Basen hat:

(a) ein Oligonukleotid, das ein Paar von Primern ist, das ausgebildet ist, mindestens einen CpG-Ort eines Gens ausgewählt aus der Gengruppe zu flankieren; und
(b) ein Oligonukleotid, das eines eines Primers und einer Sonde ist und mit einem Nukleotid hybridisieren kann, das mindestens einen CpG-Ort eines Gens ausgewählt aus der Gengruppe umfasst.

## Revendications

1. Procédé de détection d'un risque de pronostic défavorable de carcinome à cellules rénales, le procédé comprenant les étapes suivantes (a) à (c) :

(a) une étape de préparation d'un ADN génomique dérivé du tissu rénal d'un sujet ;
(b) une étape de détection d'un niveau de méthylation d'ADN d'au moins un site CpG d'un gène sélectionné dans le groupe génique constitué par FAM150A, GRM6, ZNF540, ZFP42, ZNF154, RIMS4, PCDHAC1, KHDRBS2, ASCL2, KCNQ1, PRAC, WNT3A, TRH, FAM78A, ZNF671, SLC13A5 et NKX6-2 dans l'ADN génomique préparé dans l'étape (a) ; et
(c) une étape de détermination pour savoir si le sujet est classifié, ou non, dans un groupe de pronostic défavorable selon le niveau de méthylation d'ADN détecté dans l'étape (b).

2. Procédé selon la revendication 1, dans lequel l'étape (b) est une étape de traitement de l'ADN génomique préparé dans l'étape (a) avec du bisulfite et de détection d'un niveau de méthylation d'ADN du site CpG.

3. Procédé selon la revendication 1 ou 2, dans lequel la détection comprend l'utilisation d'un oligonucléotide selon l'un quelconque de (a) et (b) suivants, qui présente une longueur d'au moins 12 bases :

(a) un oligonucléotide qui comporte une paire d'amorces destinées à encadrer au moins un site CpG d'un gène choisi dans le groupe génique ; et
(b) un oligonucléotide qui est soit une amorce soit une sonde capable de s'hybrider à un nucléotide comprenant au moins un site CpG d'un gène choisi dans le groupe génique.

Fig. 1

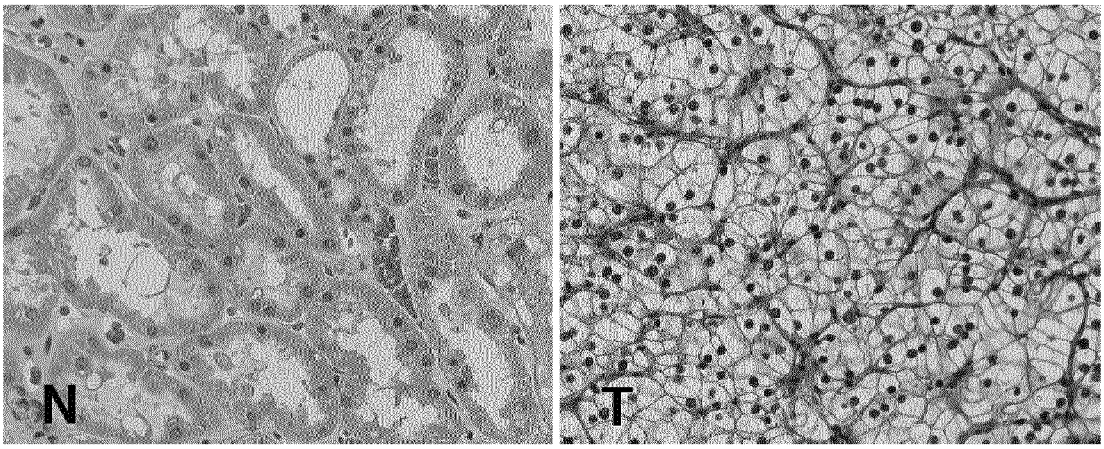
N
T

Fig. 2

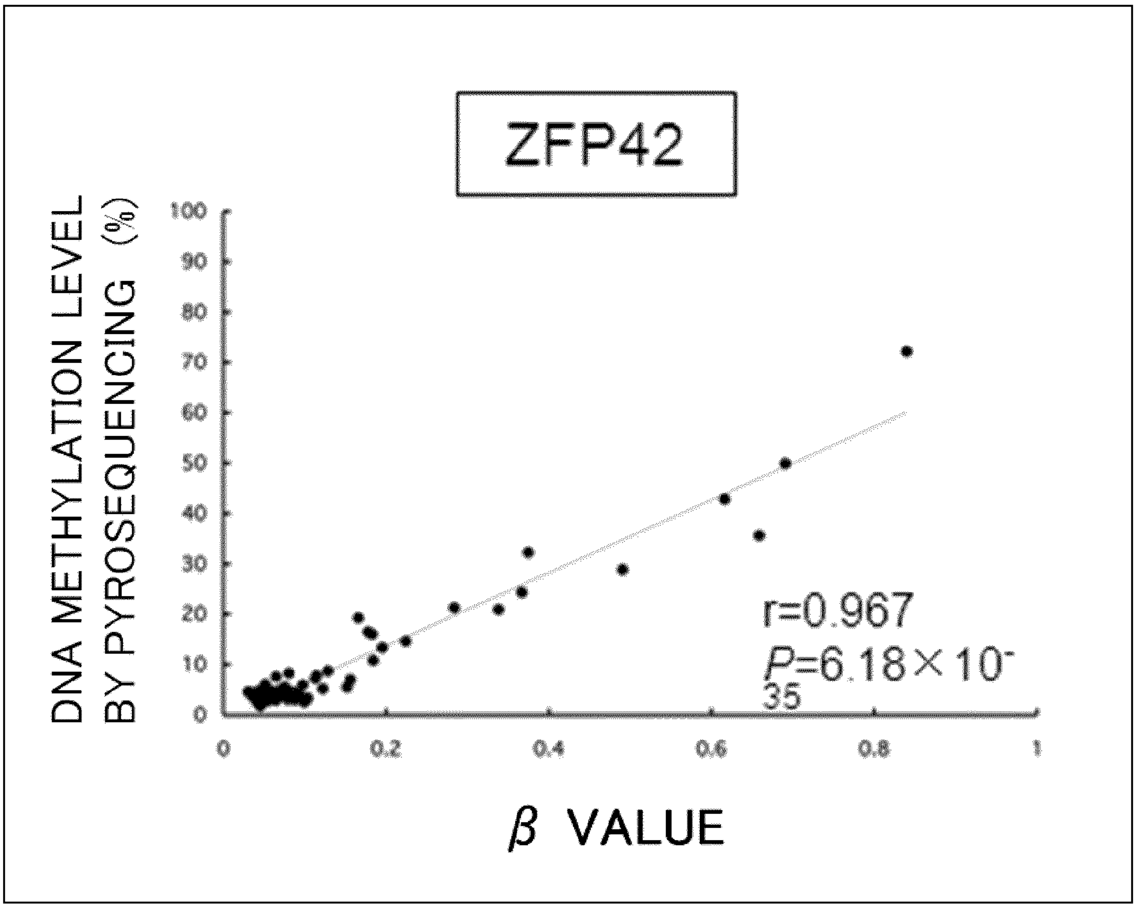
ZFP42
DNA METHYLATION LEVEL BY PYROSEQUENCING (%)
100
90
80
70
60
50
40
30
20
10
0
0
0.2
0.4
0.6
0.8
1
β VALUE
r=0.967
P=6.18×10-35

Fig. 3

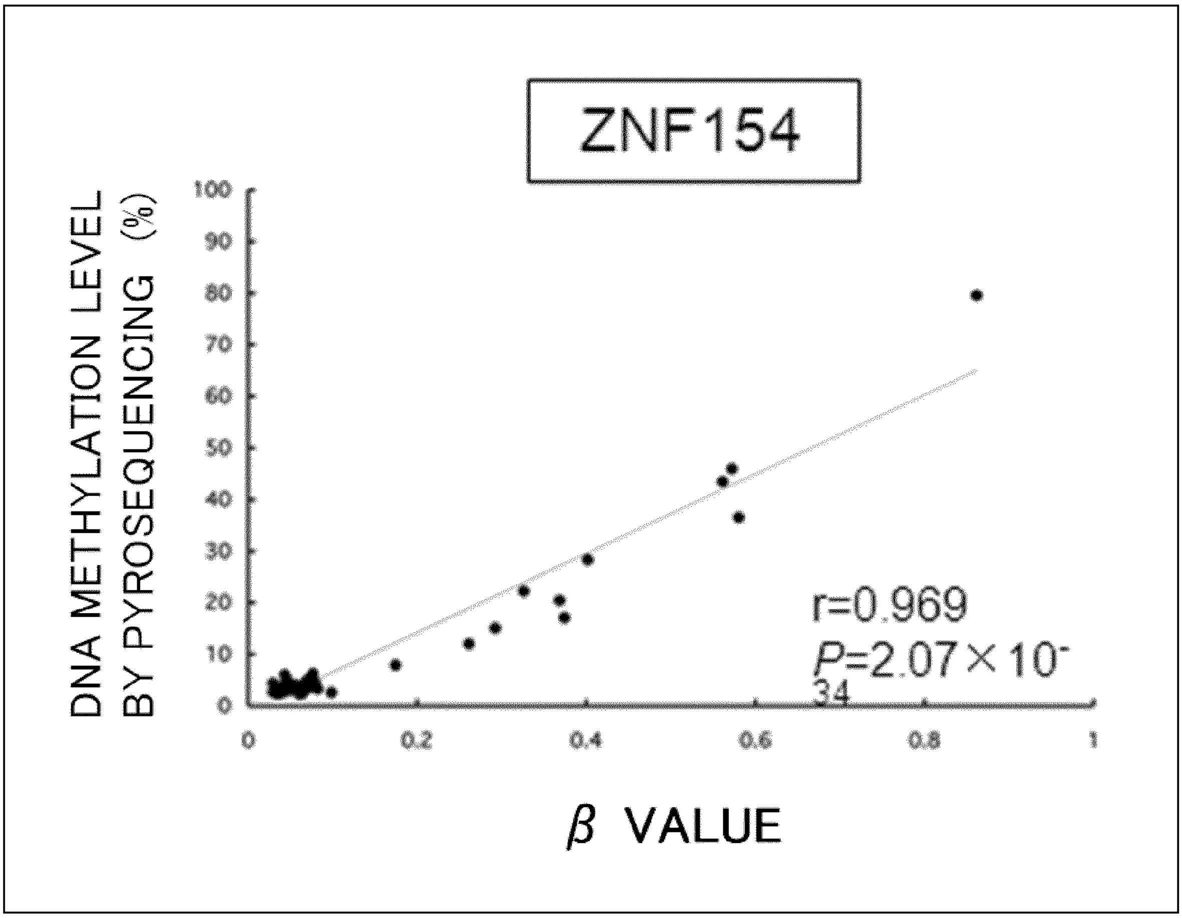
ZNF154
DNA METHYLATION LEVEL BY PYROSEQUENCING (%)
100
90
80
70
60
50
40
30
20
10
0
0
0.2
0.4
0.6
0.8
1
β VALUE
r=0.969
P=2.07×10-34

Fig. 4

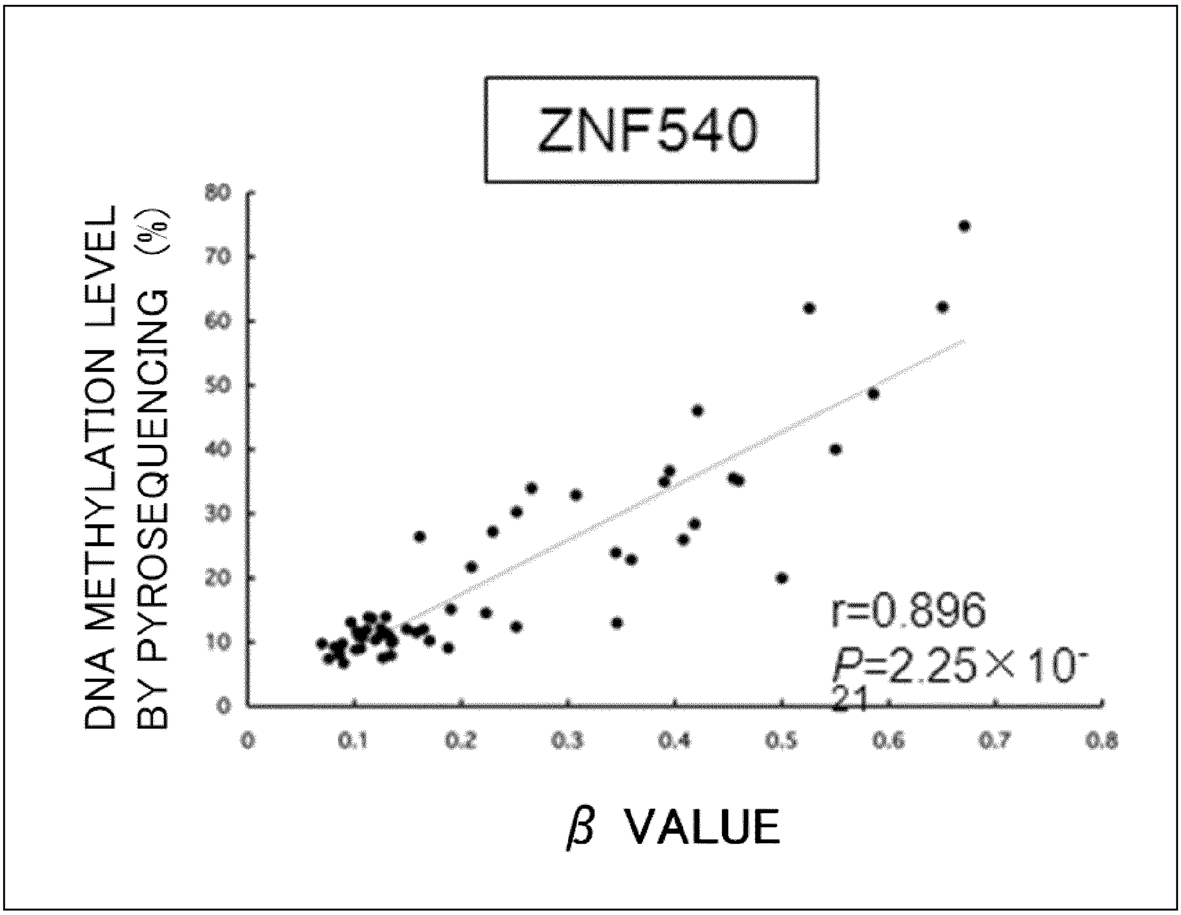
ZNF540
DNA METHYLATION LEVEL BY PYROSEQUENCING (%)
80
70
60
50
40
30
20
10
0
0
0.1
0.2
0.3
0.4
0.5
0.6
0.7
0.8
r=0.896
P=2.25×10-21
β VALUE

Fig. 5

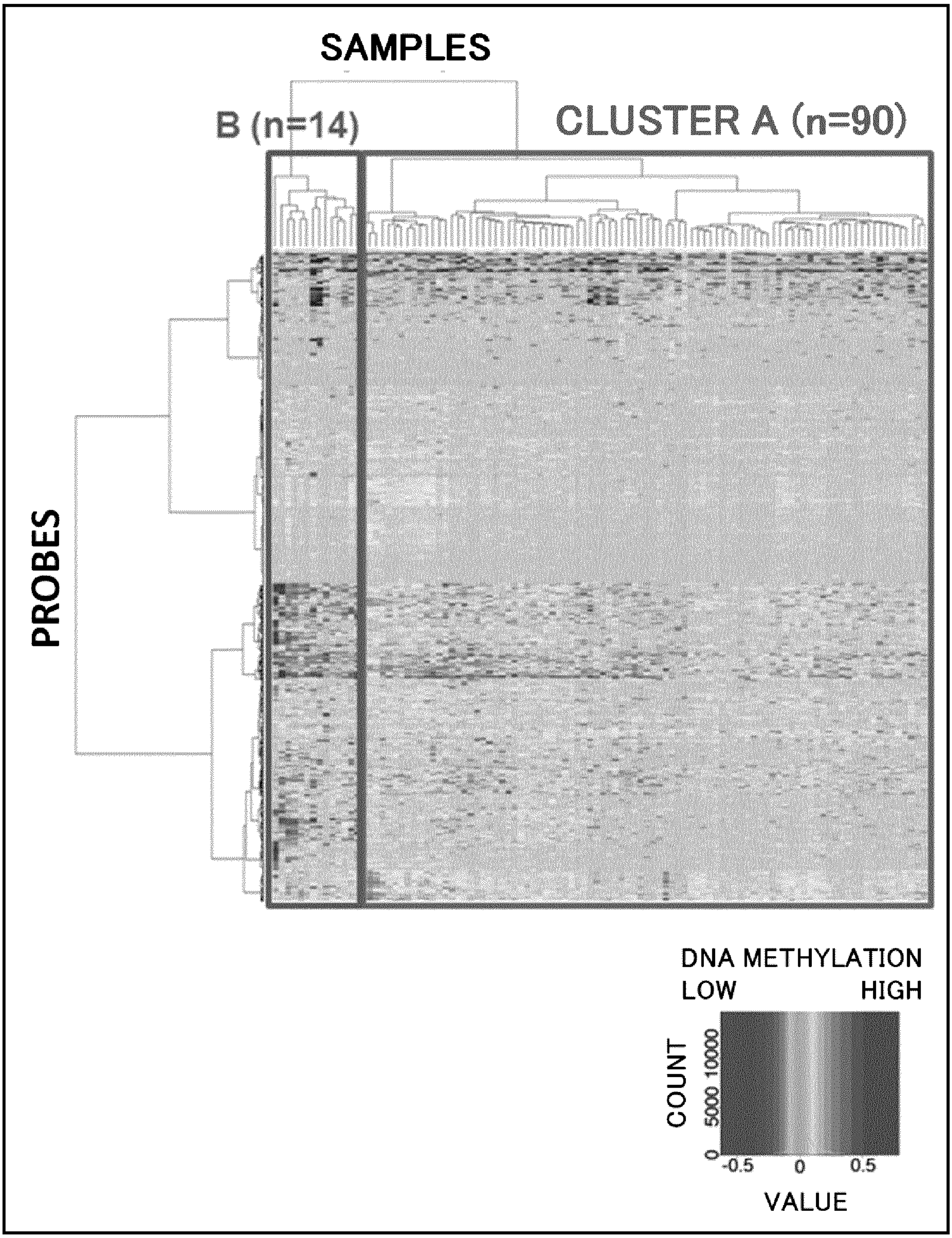
SAMPLES
B (n=14)
CLUSTER A (n=90)
PROBES
DNA METHYLATION
LOW
HIGH
COUNT
0
5000
10000
-0.5
0
0.5
VALUE

Fig. 6

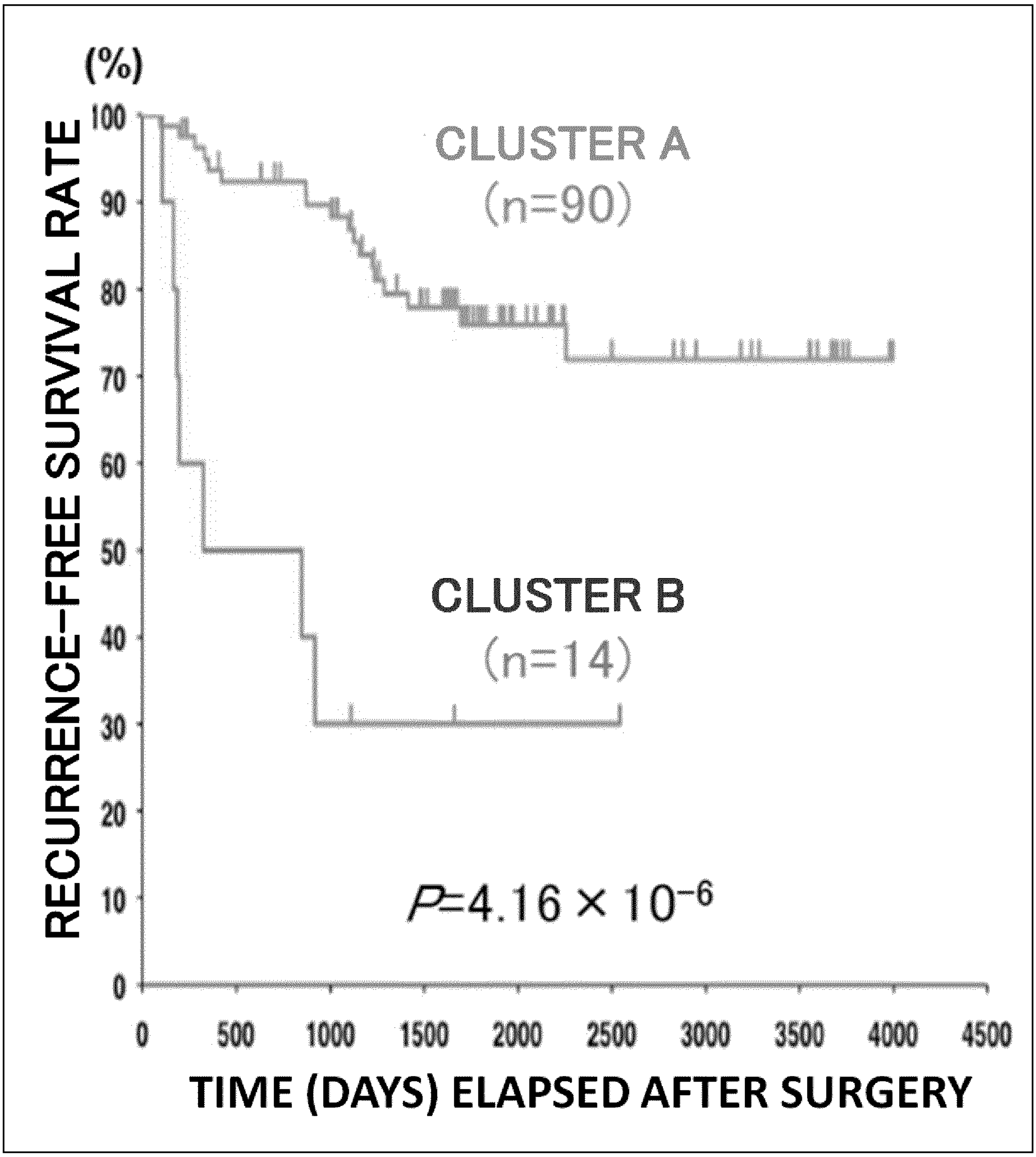
(%)
RECURRENCE-FREE SURVIVAL RATE
100
90
80
70
60
50
40
30
20
10
0
CLUSTER A
(n=90)
CLUSTER B
(n=14)
P=4.16 × 10^-6
0
500
1000
1500
2000
2500
3000
3500
4000
4500
TIME (DAYS) ELAPSED AFTER SURGERY

Fig. 7

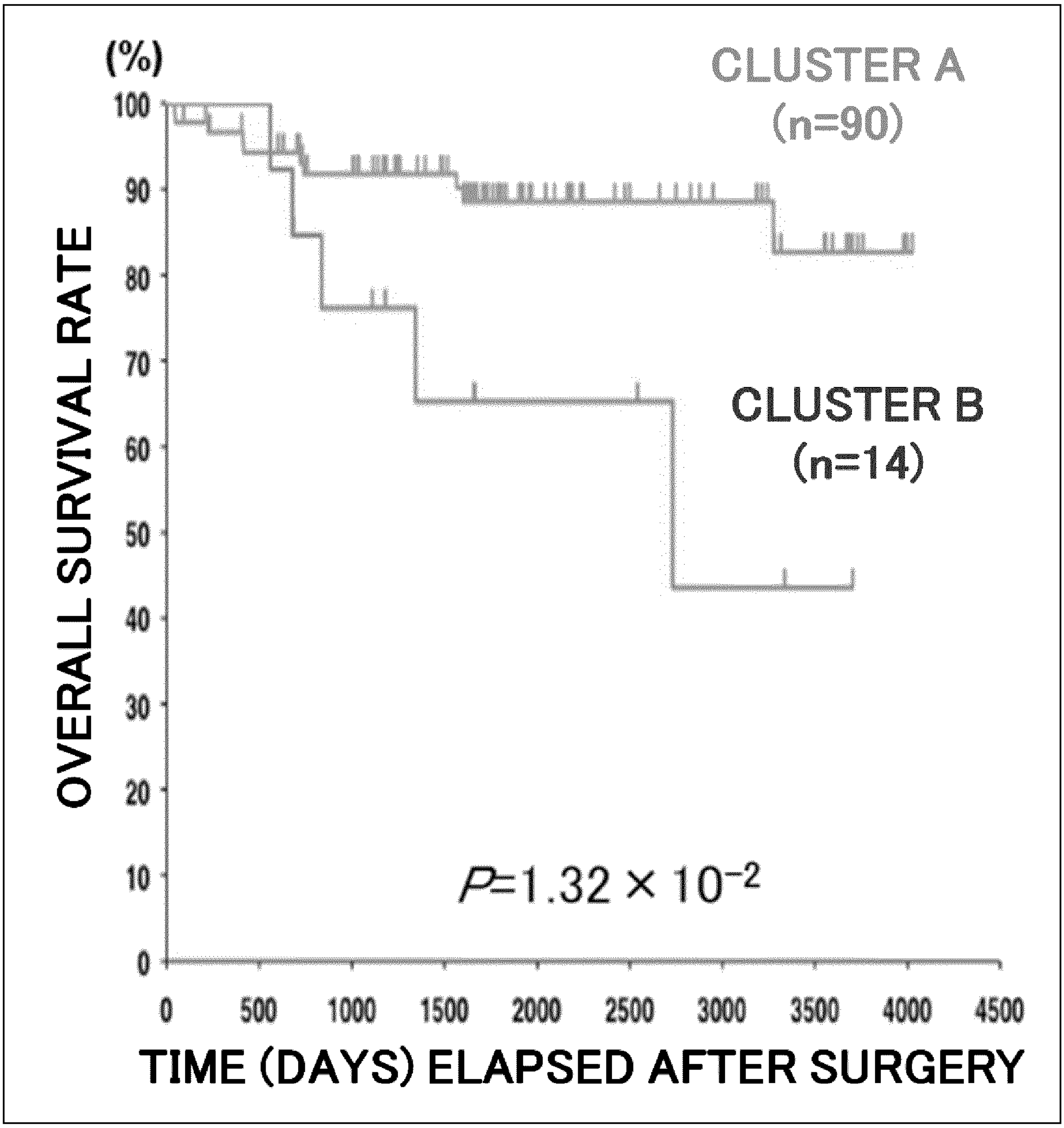
(%)
CLUSTER A
(n=90)
CLUSTER B
(n=14)
OVERALL SURVIVAL RATE
100
90
80
70
60
50
40
30
20
10
0
$P$=1.32 × 10$^{-2}$
0
500
1000
1500
2000
2500
3000
3500
4000
4500
TIME (DAYS) ELAPSED AFTER SURGERY

Fig. 8

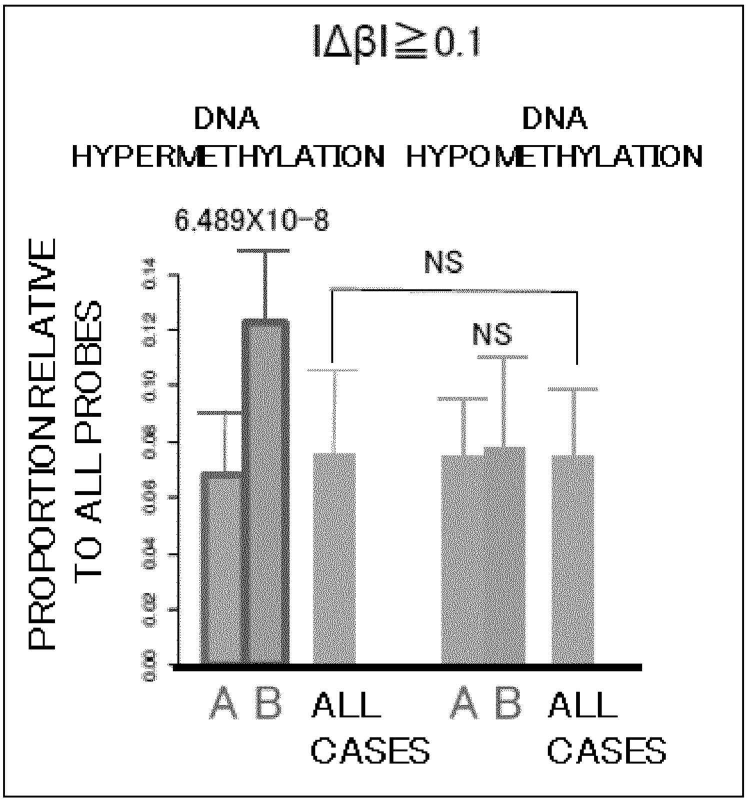
|Δβ|≧0.1
DNA HYPERMETHYLATION
DNA HYPOMETHYLATION
6.489X10-8
NS
NS
PROPORTION RELATIVE TO ALL PROBES
0.00
0.02
0.04
0.06
0.08
0.10
0.12
0.14
A B ALL CASES
A B ALL CASES

Fig. 9

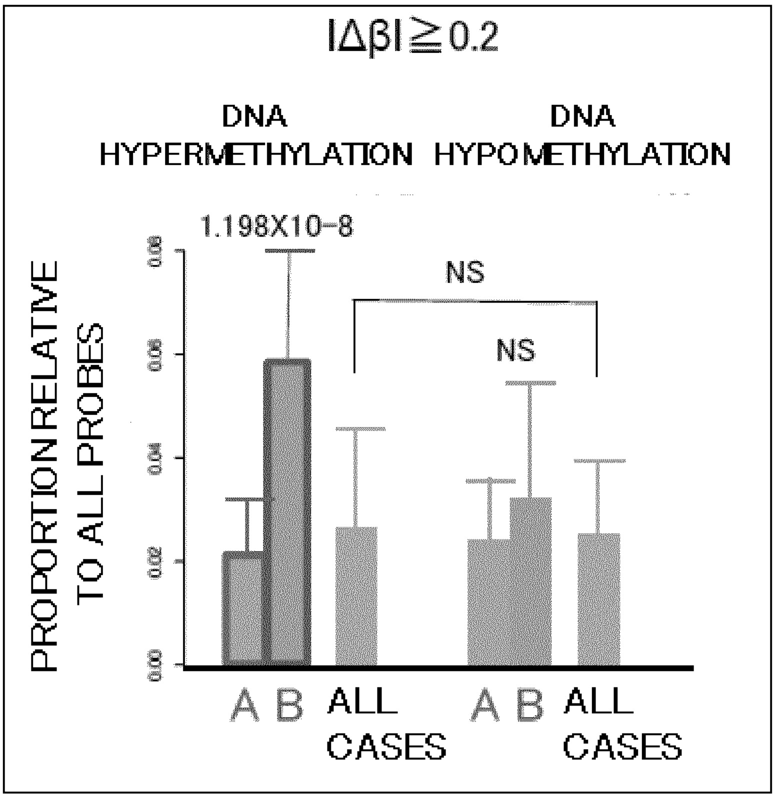
|Δβ|≧0.2
DNA HYPERMETHYLATION
DNA HYPOMETHYLATION
1.198X10-8
NS
NS
PROPORTION RELATIVE TO ALL PROBES
0.08
0.06
0.04
0.02
0.00
A
B
ALL CASES
A
B
ALL CASES

Fig. 10

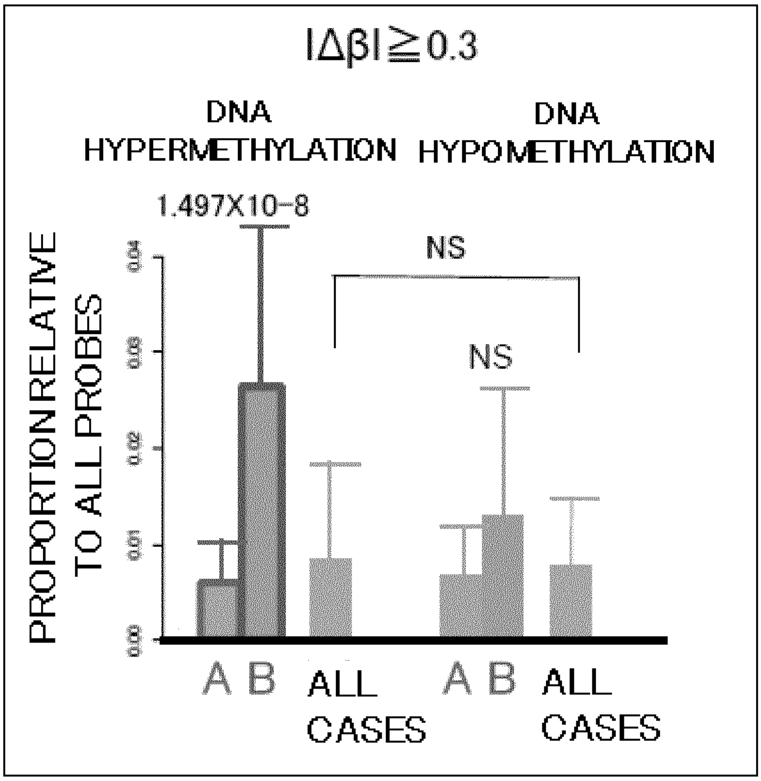
|Δβ|≧0.3
DNA HYPERMETHYLATION
DNA HYPOMETHYLATION
1.497X10-8
NS
NS
PROPORTION RELATIVE TO ALL PROBES
A B
ALL CASES
A B
ALL CASES

Fig. 11

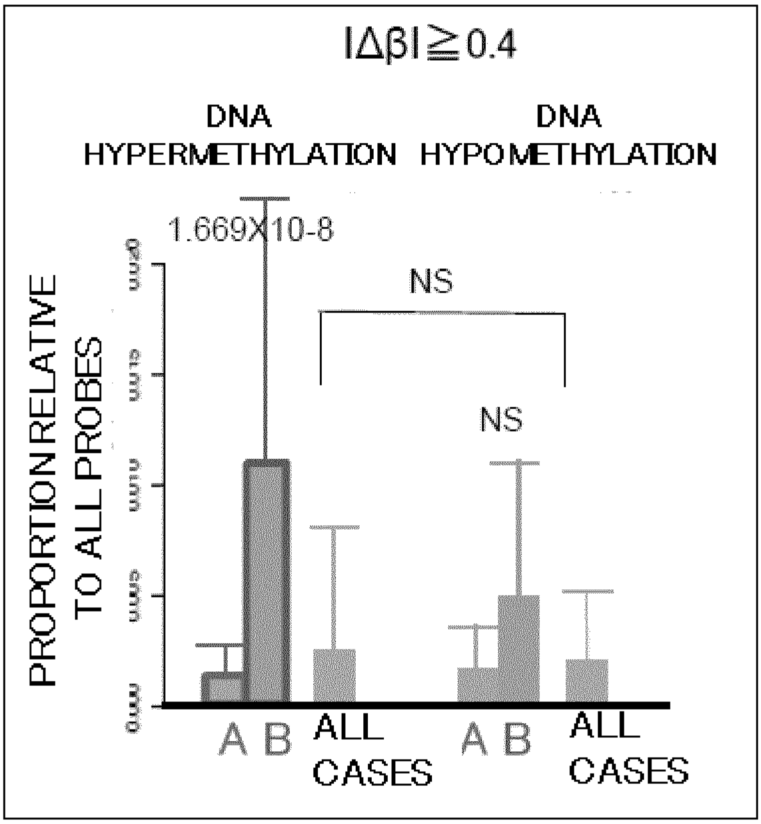
IΔβI≧0.4
DNA HYPERMETHYLATION
DNA HYPOMETHYLATION
1.669X10-8
NS
NS
PROPORTION RELATIVE TO ALL PROBES
A B
ALL CASES
A B
ALL CASES

Fig. 12

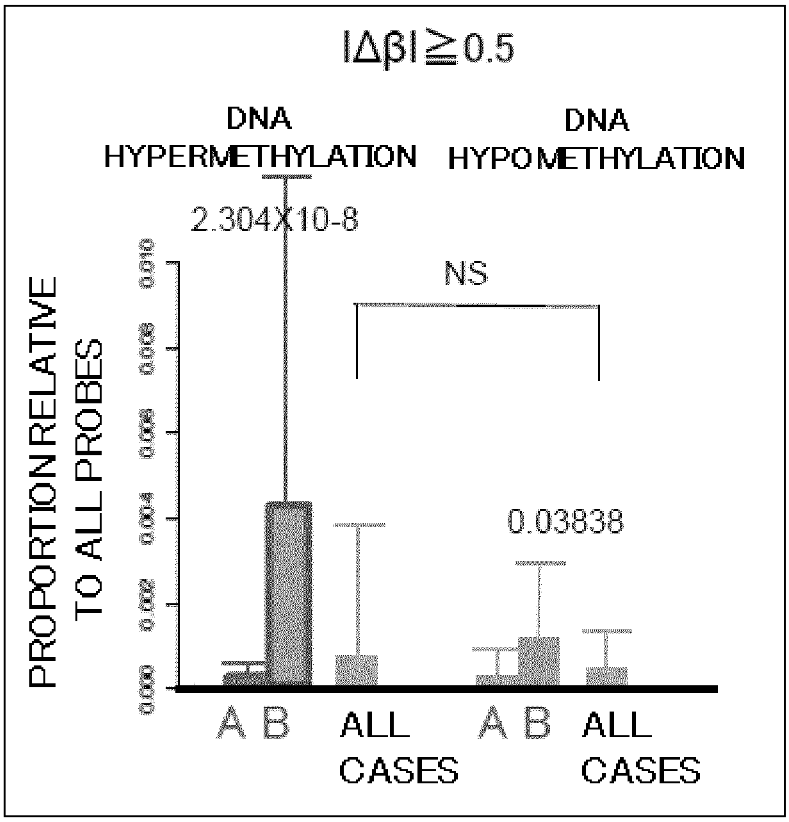
|Δβ|≧0.5
DNA HYPERMETHYLATION
DNA HYPOMETHYLATION
2.304X10-8
NS
0.03838
PROPORTION RELATIVE TO ALL PROBES
0.000
0.002
0.004
0.006
0.008
0.010
A B
ALL CASES
A B
ALL CASES

Fig. 13

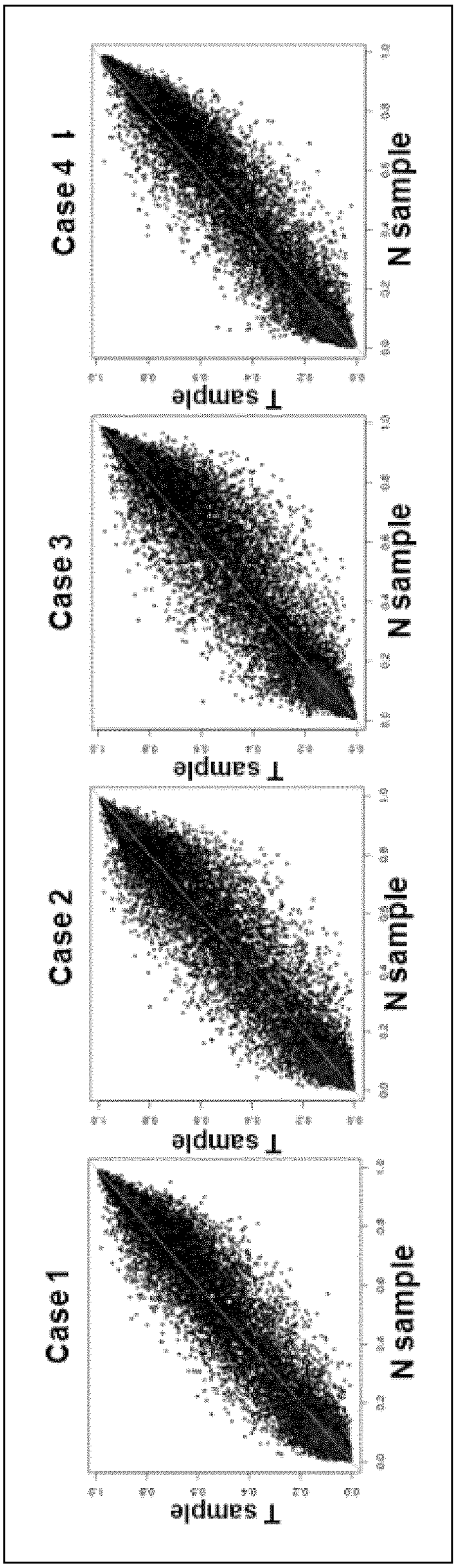

Fig. 14

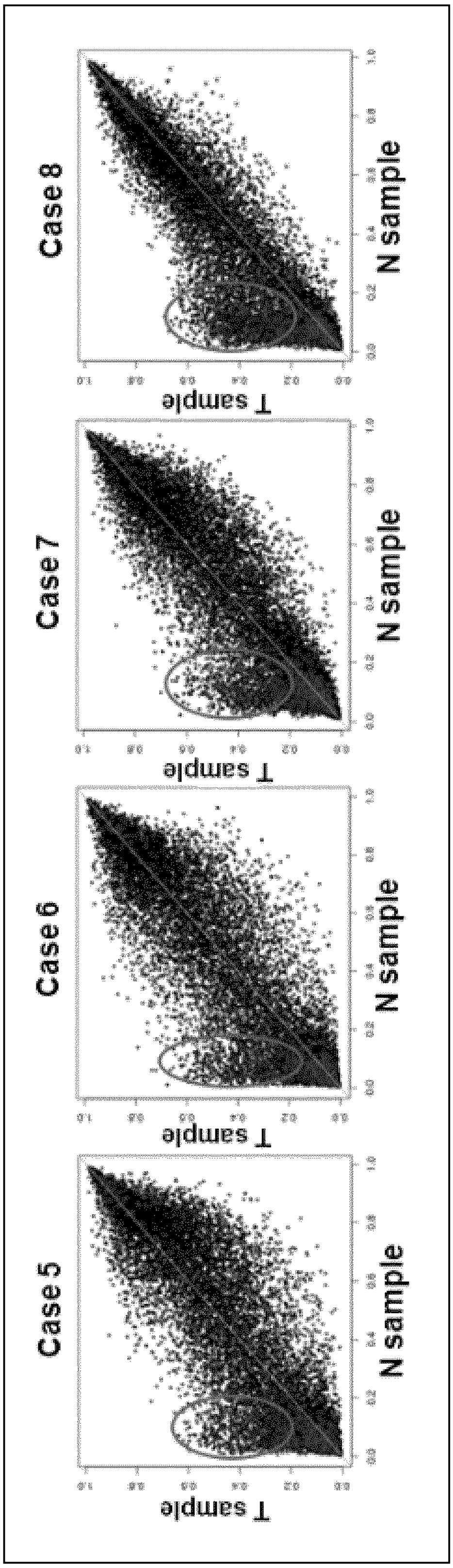

Fig. 15

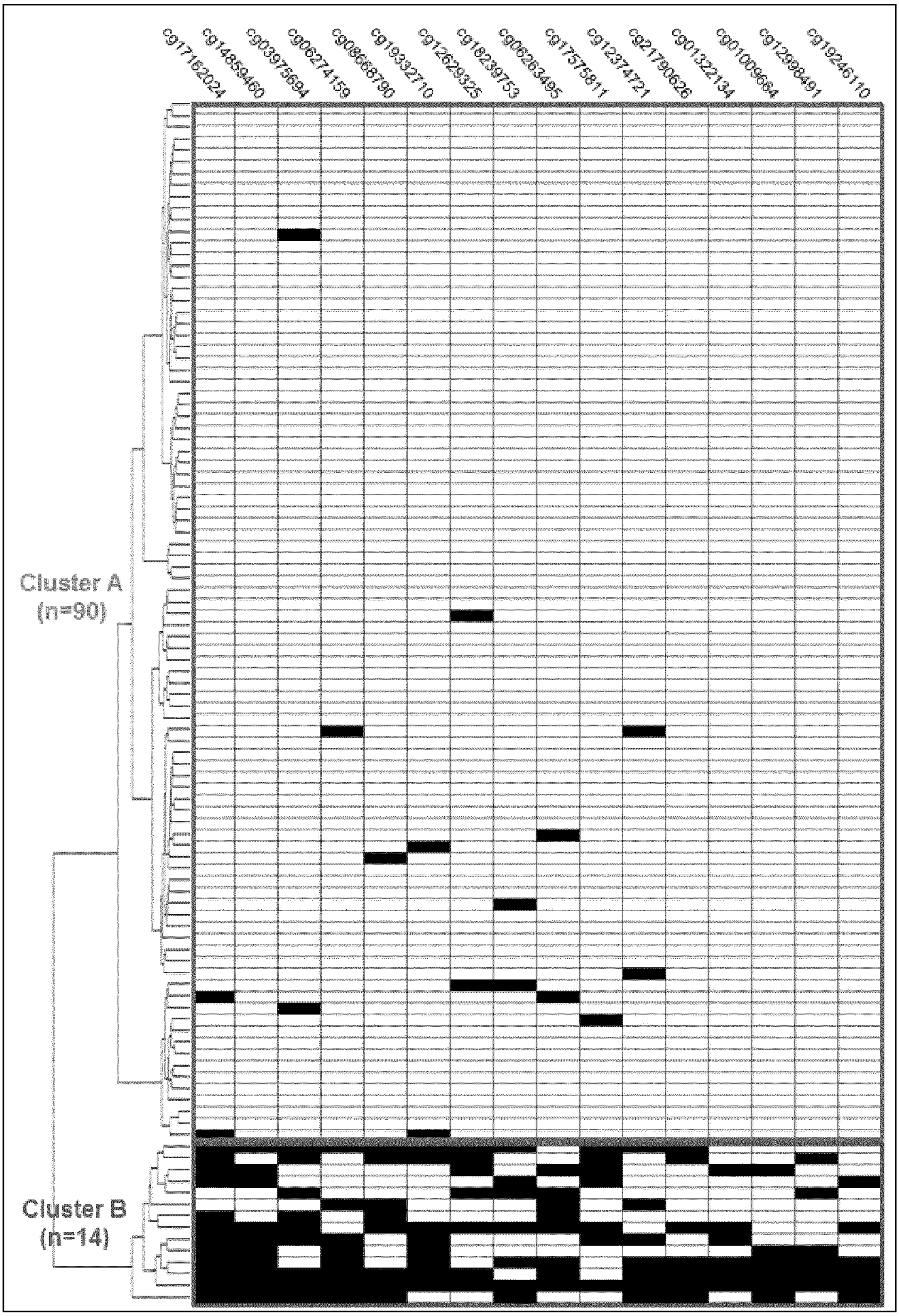
cg17162024
cg14859460
cg03975694
cg06274159
cg08668790
cg19332710
cg12629325
cg18239753
cg06263495
cg17575811
cg12374721
cg21790626
cg01322134
cg01009664
cg12998491
cg19246110
Cluster A
(n=90)
Cluster B
(n=14)

Fig. 16

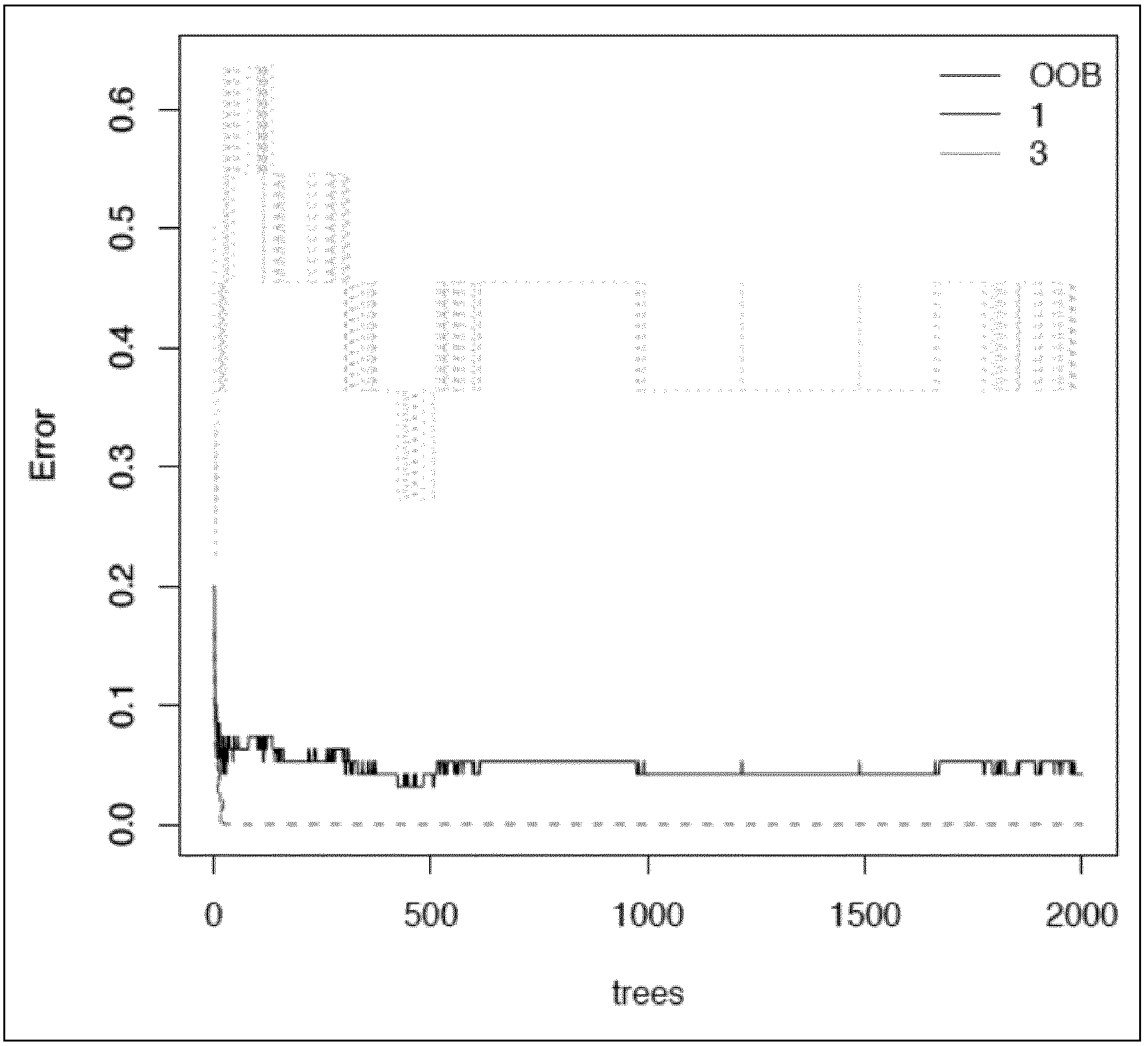

Fig. 17

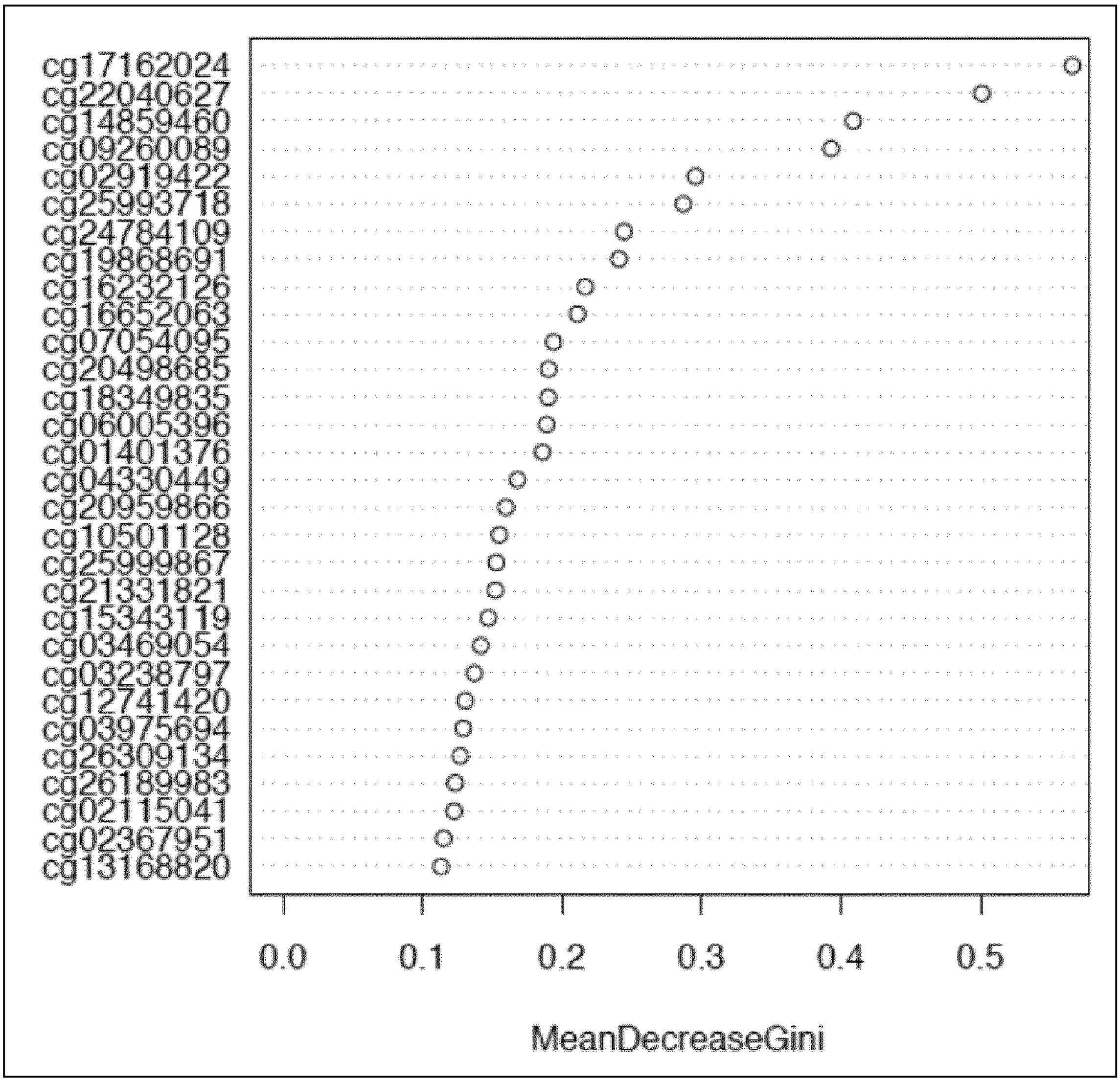
cg17162024
cg22040627
cg14859460
cg09260089
cg02919422
cg25993718
cg24784109
cg19868691
cg16232126
cg16652063
cg07054095
cg20498685
cg18349835
cg06005396
cg01401376
cg04330449
cg20959866
cg10501128
cg25999867
cg21331821
cg15343119
cg03469054
cg03238797
cg12741420
cg03975694
cg26309134
cg26189983
cg02115041
cg02367951
cg13168820
0.0
0.1
0.2
0.3
0.4
0.5
MeanDecreaseGini

Fig. 18

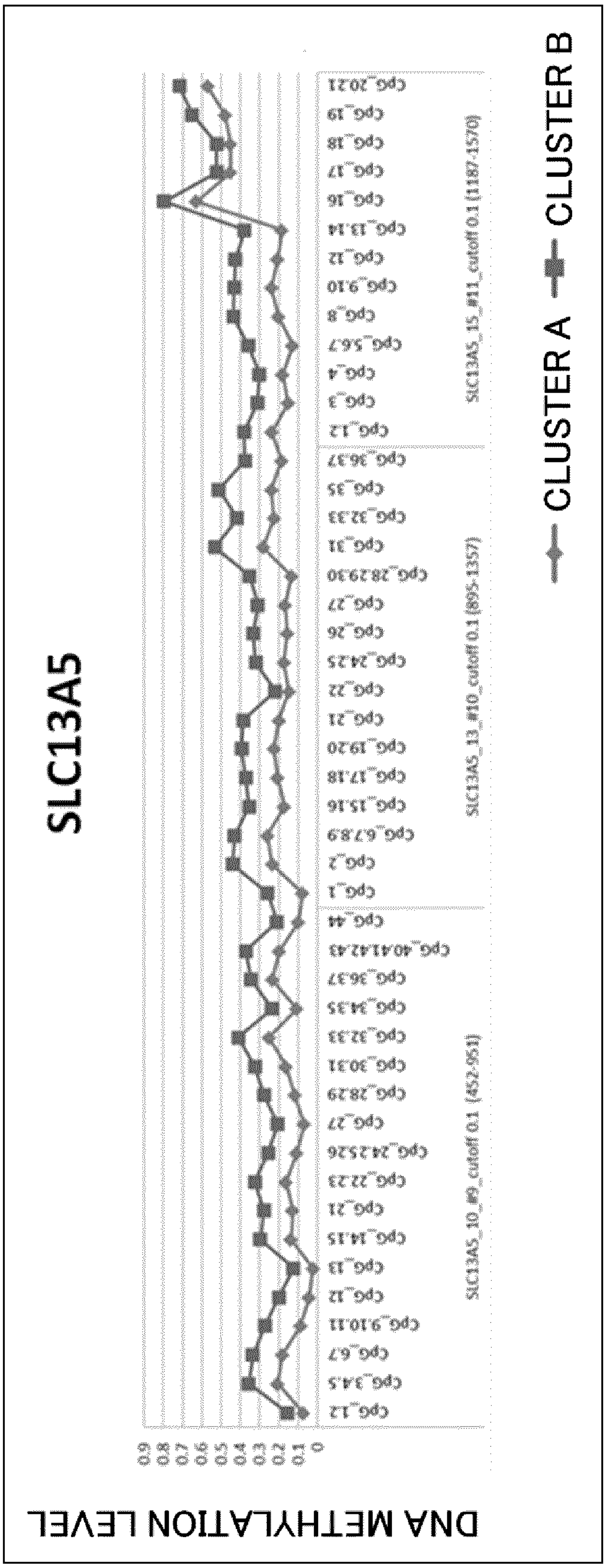
SLC13A5
DNA METHYLATION LEVEL
CLUSTER A
CLUSTER B
SLC13A5_10_#9_cutoff 0.1 (452-951)
SLC13A5_13_#10_cutoff 0.1 (895-1357)
SLC13A5_15_#11_cutoff 0.1 (1187-1570)

Fig. 19

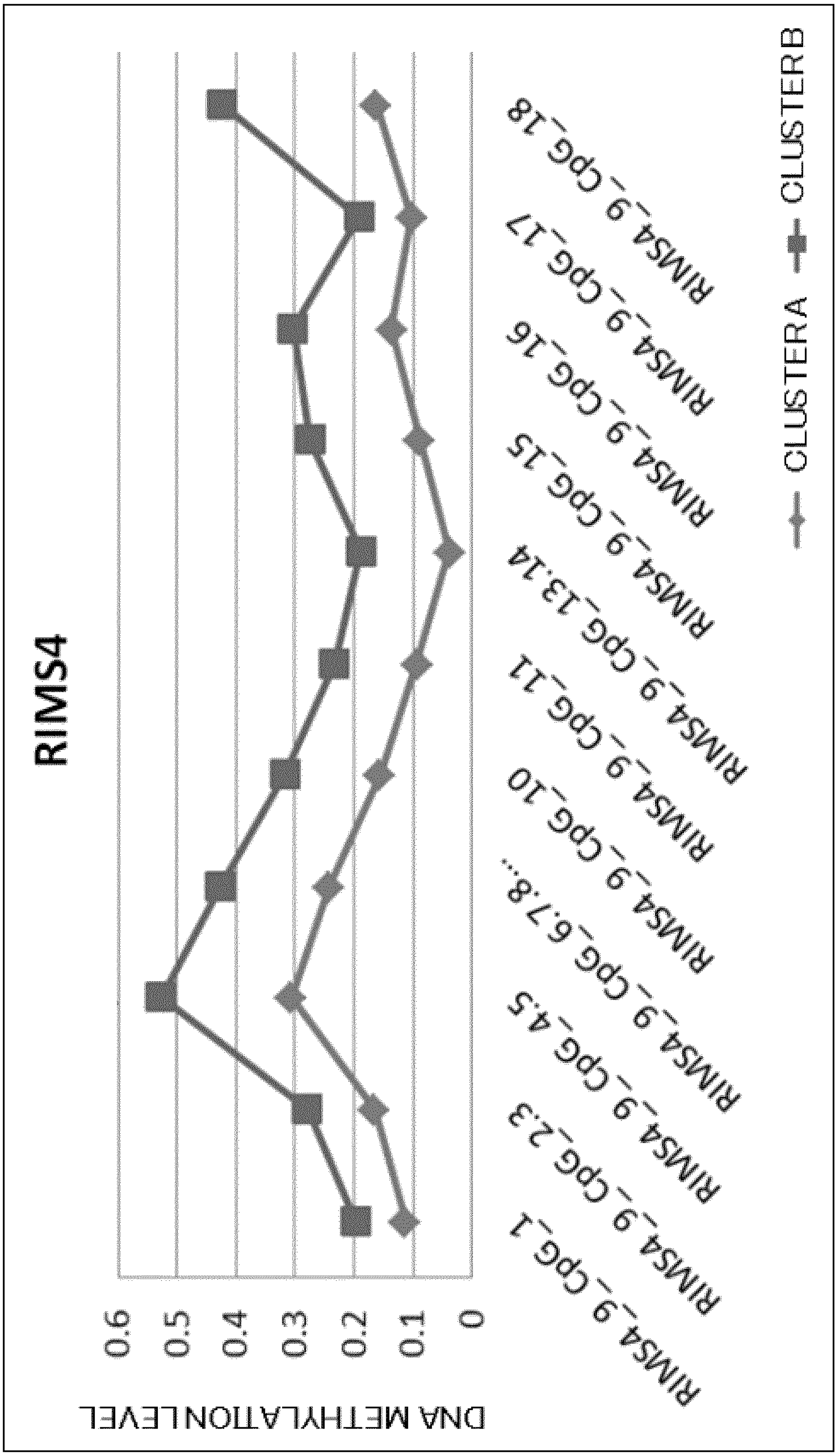
RIMS4
DNA METHYLATION LEVEL
0.6
0.5
0.4
0.3
0.2
0.1
0
RIMS4_9_CpG_1
RIMS4_9_CpG_2.3
RIMS4_9_CpG_4.5
RIMS4_9_CpG_6.7.8...
RIMS4_9_CpG_10
RIMS4_9_CpG_11
RIMS4_9_CpG_13.14
RIMS4_9_CpG_15
RIMS4_9_CpG_16
RIMS4_9_CpG_17
RIMS4_9_CpG_18
CLUSTER A
CLUSTER B

Fig. 20

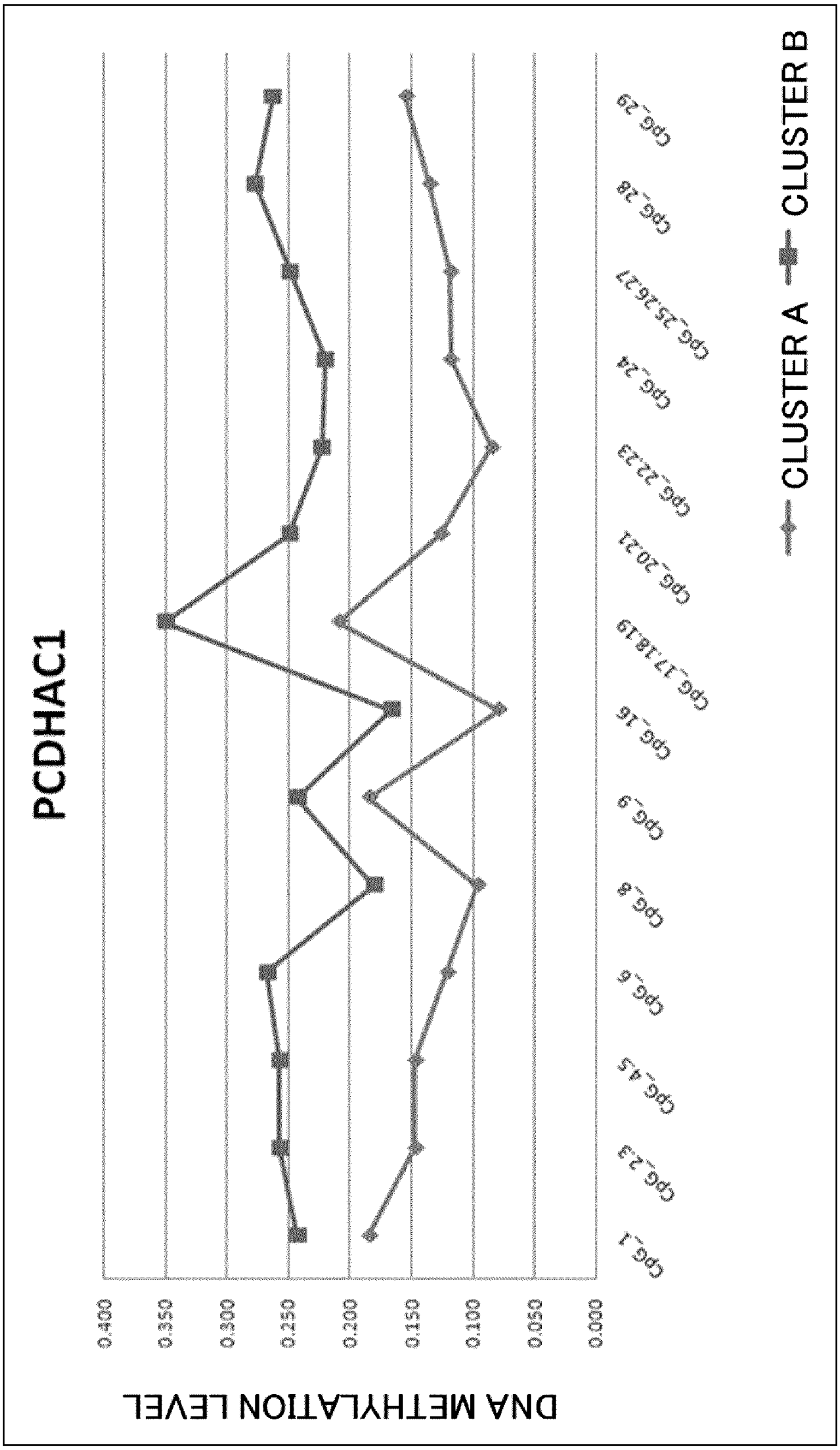
PCDHAC1
CLUSTER A
CLUSTER B
DNA METHYLATION LEVEL
0.400
0.350
0.300
0.250
0.200
0.150
0.100
0.050
0.000
CpG_1
CpG_2.3
CpG_4.5
CpG_6
CpG_8
CpG_9
CpG_16
CpG_17.18.19
CpG_20.21
CpG_22.23
CpG_24
CpG_25.26.27
CpG_28
CpG_29

Fig. 21

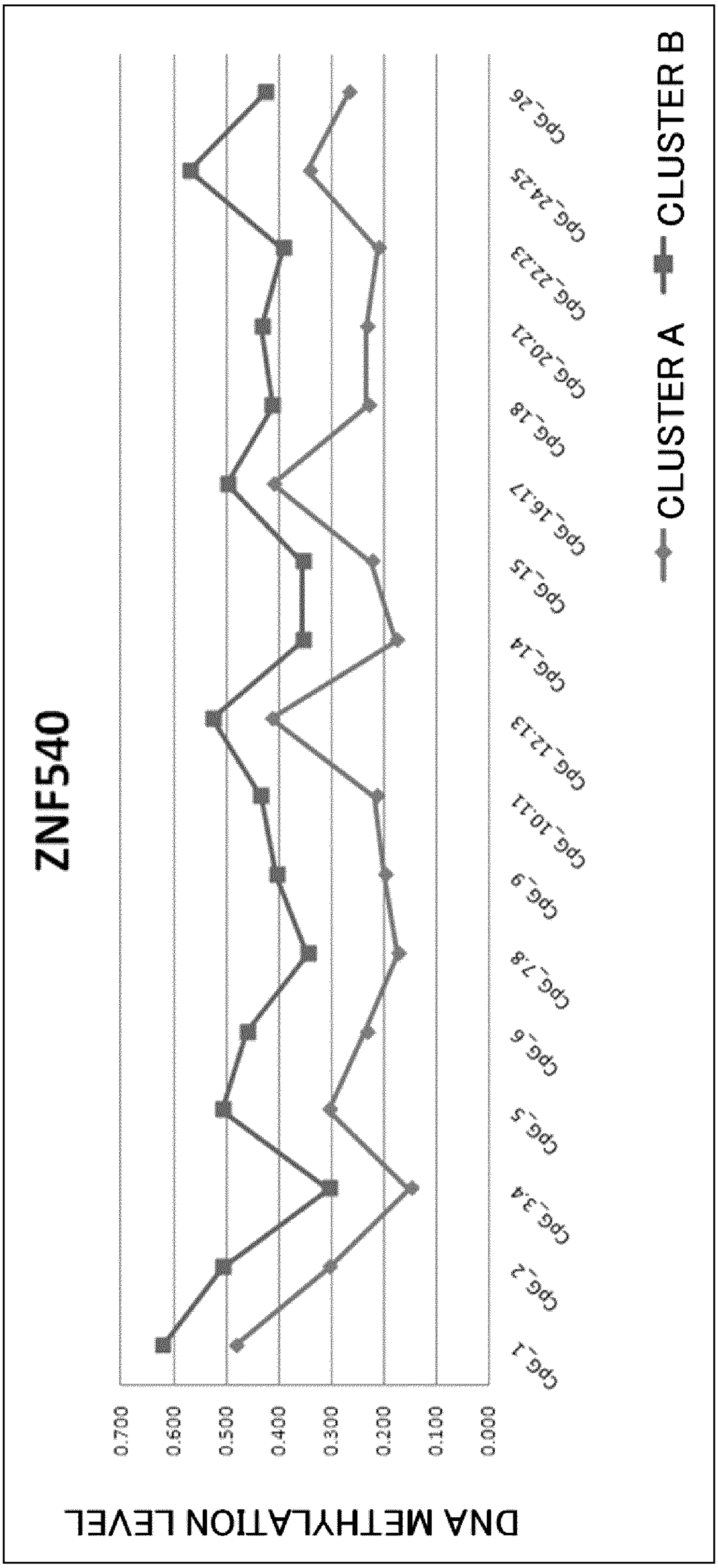
ZNF540
DNA METHYLATION LEVEL
0.700
0.600
0.500
0.400
0.300
0.200
0.100
0.000
CpG_1
CpG_2
CpG_3,4
CpG_5
CpG_6
CpG_7,8
CpG_9
CpG_10,11
CpG_12,13
CpG_14
CpG_15
CpG_16,17
CpG_18
CpG_20,21
CpG_22,23
CpG_24,25
CpG_26
CLUSTER A
CLUSTER B

Fig. 22

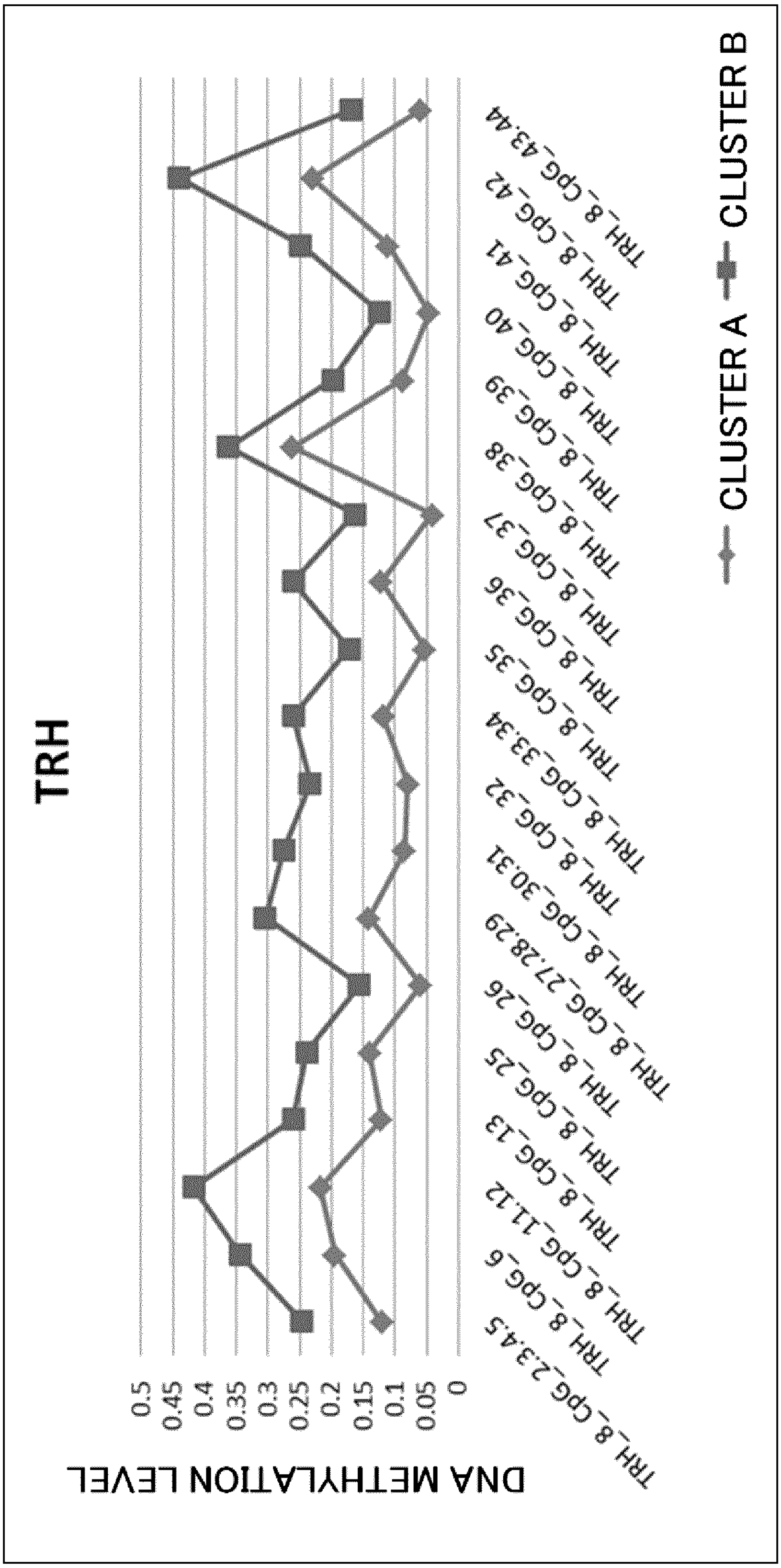
TRH
DNA METHYLATION LEVEL
0.5
0.45
0.4
0.35
0.3
0.25
0.2
0.15
0.1
0.05
0
TRH_8_CpG_2.3.4.5
TRH_8_CpG_6
TRH_8_CpG_11.12
TRH_8_CpG_13
TRH_8_CpG_25
TRH_8_CpG_26
TRH_8_CpG_27.28.29
TRH_8_CpG_30.31
TRH_8_CpG_32
TRH_8_CpG_33.34
TRH_8_CpG_35
TRH_8_CpG_36
TRH_8_CpG_37
TRH_8_CpG_38
TRH_8_CpG_39
TRH_8_CpG_40
TRH_8_CpG_41
TRH_8_CpG_42
TRH_8_CpG_43.44
CLUSTER A
CLUSTER B

Fig. 23

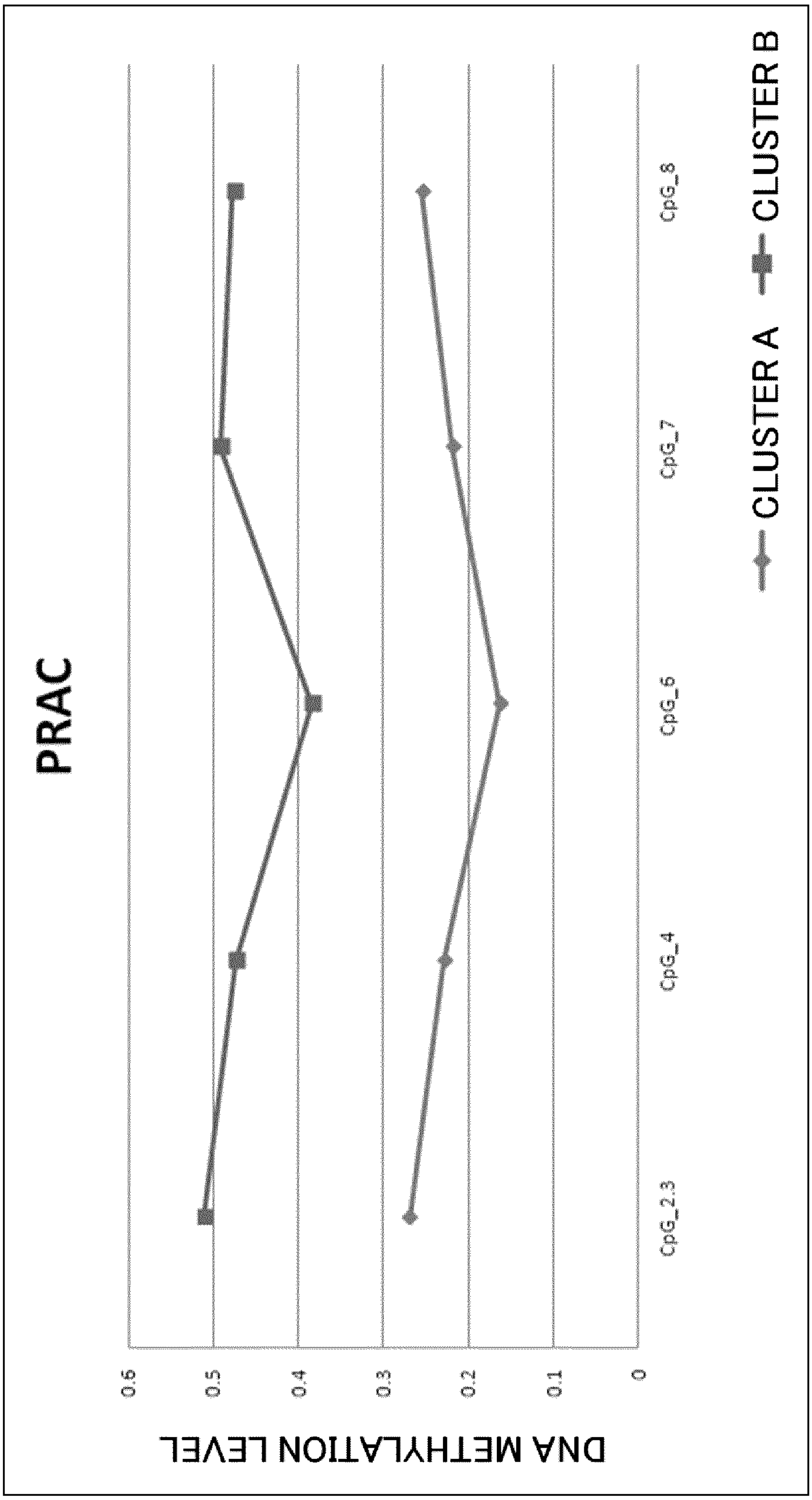
PRAC
DNA METHYLATION LEVEL
0.6
0.5
0.4
0.3
0.2
0.1
0
CpG_2.3
CpG_4
CpG_6
CpG_7
CpG_8
CLUSTER A
CLUSTER B

Fig. 24

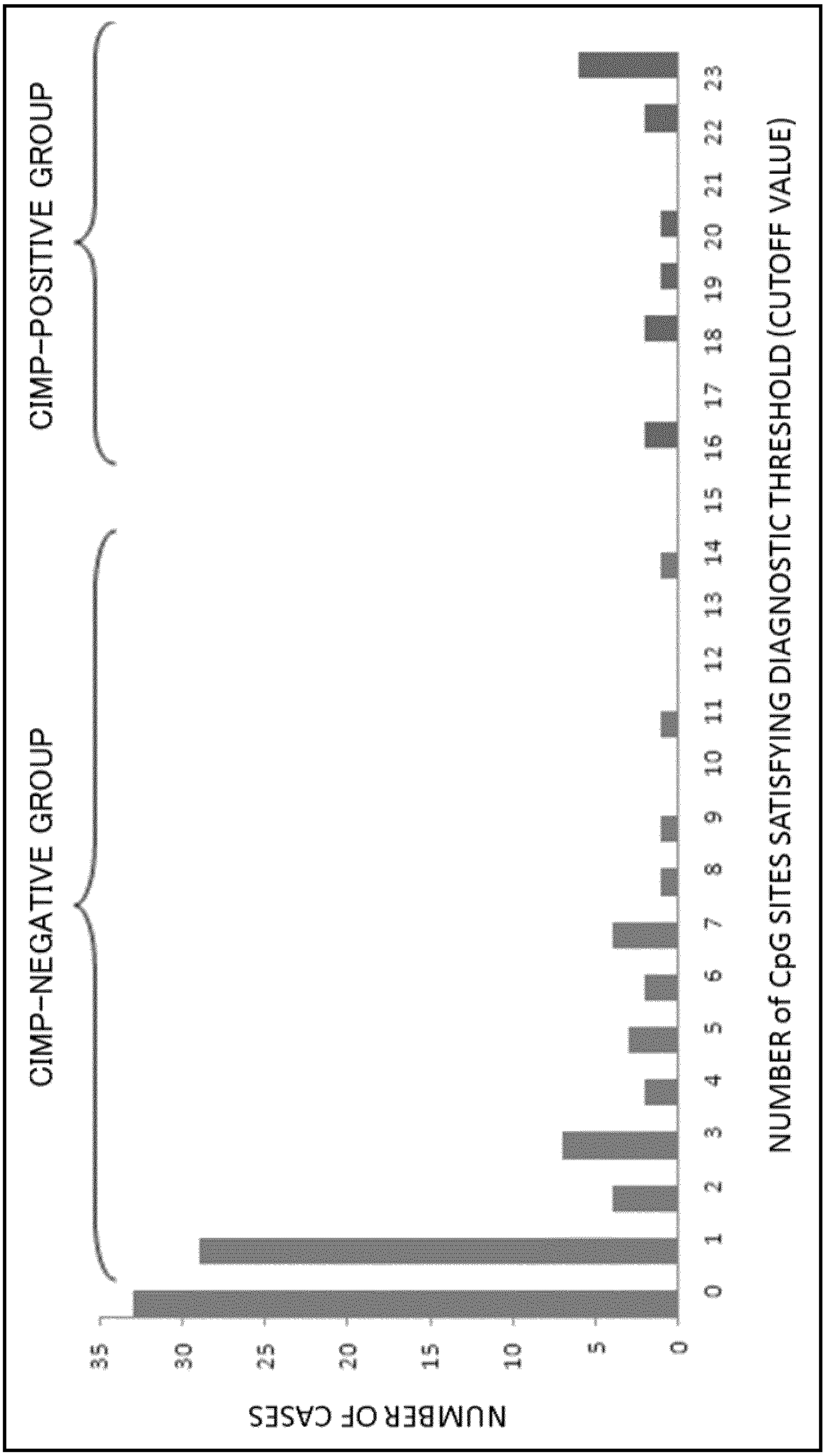
CIMP–NEGATIVE GROUP
CIMP–POSITIVE GROUP
NUMBER OF CASES
35
30
25
20
15
10
5
0
0 1 2 3 4 5 6 7 8 9 10 11 12 13 14 15 16 17 18 19 20 21 22 23
NUMBER of CpG SITES SATISFYING DIAGNOSTIC THRESHOLD (CUTOFF VALUE)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010063413 A
- US 61646044 B


### Non-patent literature cited in the description


- **DALGLIESH, G. L. et al.** *Nature,* 2010, vol. 463, 360-363
- **VAN HAAFTEN, G. et al.** *Nat. Genet.,* 2009, vol. 41, 521-523
- **VARELA, I. et al.** *Nature,* 2011, vol. 469, 539-542
- **ARAI, E. et al.** *Clin. Cancer Res.,* 2008, vol. 14, 5531-5539
- **ARAI, E. et al.** *Int. J. Cancer,* 2006, vol. 119, 288-296
- **ARAI, E. et al.** *Carcinogenesis,* 2009, vol. 3 (0), 214-221
- **ARAI, E. et al.** *Pathobiology,* 2011, vol. 78, 1-9
- **ISSA, J. P.** *Nat. Rev. Cancer,* 2004, vol. 4, 988-993
- **TOYOTA, M. et al.** *Proc. Natl. Acad. Sci. USA,* 1999, vol. 96, 8681-8686
- **SHEN, L. et al.** *Proc. Natl. Acad. Sci. USA,* 2007, vol. 104, 18654-18659
- **TOYOTA, M. et al.** *Cancer Res.,* 1999, vol. 5 (9), 5438-5442
- **MORRIS, M. R. et al.** *Genome Med.,* 2010, vol. 2 (9), 59
- **MCRONALD, F. E. et al.** *Mol. Cancer,* 2009, 8
- **MORRIS, M.R. et al.** *Oncogene,* 2010, vol. 29, 2104-2117
- **CLARK SJ et al.** *Nucleic Acids Res,* 1994, vol. 22, 2990-7
- **JURINKE C et al.** *Mutat Res,* 2005, vol. 573, 83-95
- *Anal. Biochem.,* 2000, vol. 10, 103-110
- **WOJDACZ TK et al.** *Nat Protoc.,* 2008, vol. 3, 1903-8
- **KRISTENSEN LS et al.** *Clin Chem,* 2009, vol. 55, 1471-83
- **EBLE, J. N. et al.** Renal cell carcinoma. WHO classification of tumours. Pathology and genetics. Tumours of the urinary system and male genital organs. IARC Press, 2004, 10-43
- **FUHRMAN, S. A. et al.** *Am. J. Surg. Pathol.,* 1982, vol. 6, 655-663
- **SOBIN, L. H. et al.** International Union Against Cancer (UICC), TNM Classification Of Malignant Tumors. Wiley-Liss, 2002, 193-195
- **KANAI, T. et al.** *Cancer,* 1987, vol. 60, 810-819
- **SAMBROOK, J. et al.** Molecular Cloning: A Laboratory Manual. Cold Spring Harbor Laboratory Press, 6.14-6.15
- **BIBIKOVA, M. et al.** *Epigenomics,* 2009, vol. 1, 177-200
- **BREIMAN, L.** *Mach. Learn.,* 2001, vol. 45, 5-32

SEQUENCE LISTING

<110> National Cancer Center
<120> \220t\215×\226E\212à\202Ì\227\\214ã\227\\221ª\225û\226@
<130> IBPF12-517WO
<150> US 61/646044
<151> 2012-05-11
<160> 57
<170> PatentIn version 3.1

<210> 1
<211> 500
<212> DNA
<213> Homo sapiens
<220>
<223> SLC13A5_MA_10
<400> 1

```
gaaggacttg aacttggaga catagctcag cgccgaggcc atcgcgcggg agggagactg     60
gcgggcgaga cgagtgaggg gcagctagag gcgccgcggg cttaagaagg ggccacagtc    120
cccggggatt ggggaggggg cggtgacaac tccgccccgc acgggggcgc ctccccgcgg    180
ccctggggcg gggccacccc tcggggtctg tgggacgcgc ctgcccccaa ttctgccacc    240
cggcggcggt gggaggcgtc tttggactcc aacgcttcgg gccagccctc taggggcagc    300
ctgggcccta gcatctcgcg ctgtccaagc ctctcctgcg ctgccgaggc agaggtgcgt    360
cccggggctg ccaagcgggg cgtgttttgg tcactggtgc tgcccgcttt ggcgtaaggc    420
gccctcccgc gtccgcatct gctctttcct gggctctgaa gggtcccgga tgaaactctc    480
tgcaggcctc tgggtctctc                                                500
```

<210> 2
<211> 463
<212> DNA
<213> Homo sapiens
<220>
<223> SLC13A5_MA_13
<400> 2

```
ctttcctggg ctctgaaggg tcccggatga aactctctgc aggcctctgg gtctctcagg      60
tctatctccc cgatctccct ctcctttcca tctccttact tccgcccctc cggtgtctct     120
ccgagaggtg tccccccacg ccccgcgccc tccgcaccgc gggcctcgct tcccggtccc     180
ccctggcttc ctcgccacgt ccgccccact ctaggtgcag gacccctttt ccccgctcgc     240
actctccggc ccggagctcc tgggcgatcg cacagggaag cgaggccact gtcctcctct     300
gtcccagggg ctgtcgcgct ccagtggacg ctgcaccccg cagacgcccg gcgggcagat     360
gcggacacgc gtcttggagg ggccccaccg agcctcagca gccgcagctg cccgcccgac     420
ccaggtcaga gggaaacgga gctctagaga ggaagggaca caa                       463
```

<210> 3
<211> 384
<212> DNA
<213> Homo sapiens
<220>
<223> SLC13A5_MA_15
<400> 3

```
cctcctctgt cccaggggct gtcgcgctcc agtggacgct gcaccccgca gacgcccggc      60
gggcagatgc ggacacgcgt cttggagggg ccccaccgag cctcagcagc cgcagctgcc     120
cgcccgaccc aggtcagagg gaaacggagc tctagagagg aagggacaca actaaggcga     180
cactgagaca gtcgcccatg tattcattca gcccgccagg caacagacag gtgccgagca     240
cctcttctcc gcgaggccct gttttgggca ctggagacac acggatgcaa agacatcccc     300
acctctgtga ttttcttctt tcctctcctc tgcctgcctc tcattctgca gcttcctttt     360
ggggagtctc atccatgctg gtgg                                            384
```

<210> 4
<211> 455
<212> DNA
<213> Homo sapiens
<220>
<223> FAM150A_MA_14
<400> 4

```
gggaggaccc agtagggtaa ctgccgcgtc gccccggcgg ttctccctgg gctctgtctc      60
ccgccgcctc caccccccga gcctcggggt ccgtcacggc ttcccctggc tggcggggtc     120
agtagaaccc gcggcgccta ggtccggacg gaaaaaagca gggccggggt gcggcctgga     180
tgagcggaga tctccgcgcc ttgggctcaa aggtgcgggg tgcgctctgc tgccgagccc     240
ctgctcgctc aggaacactg gccacgccgt cacgccagcc gcccctgccc caggtctgga     300
ggcccgacct gctctcctag gcgcagcacc gcgttctctt ccgcgtgggg gagcggcggg     360
cggaagaggt ctggggctgg gcaccgggga cacgcgccca gctcccctgg cctccctggg     420
gggagtggcc ggtttcagtg cttccccagg tgaaa                                455
```

<210> 5
<211> 188
<212> DNA
<213> Homo sapiens
<220>
<223> GRM6_MA_8
<400> 5

```
ggttcaggac aagtctgtga cagatgcggc cgaggccctg agcgagagag gatttaagga      60
ttctagggag ggatgagaga ccgctccgag ggtggagacc cctcctgagt gtgggggtg     120
gcggtgctgg ctctccccgc atctccttcc cctccctctc ccaatctctg ggtctgtttt     180
cctgtttt                                                              188
```

<210> 6
<211> 196
<212> DNA

<213> Homo sapiens
<220>
<223> ZFP42_MA_2
<400> 6

```
gagctgatgg gtggctgtag cctgattaga ccgcgtcagt ccggagggtg ggtcttggga     60
gggggcgcag ggcagtccac gtttccactg cagtttctcc tttgttttac gtttgggagg    120
aggtggcatt ggaaatagca gagtgcttcg cggtaacagg ggtgagtctt gtttcatgga    180
actttttttca aatggg                                                    196
```

<210> 7
<211> 279
<212> DNA
<213> Homo sapiens
<220>
<223> ZNF154_MA_5
<400> 7

```
ggtgaacaca cctcagagaa gctaaaatgg ccgccacgaa gaggcccccc caaaagtccc     60
gtcctttctt tttgtgactc tcaaggaaag tcggttttct gagctcttac tggcttagta    120
gcgtggcgtt caacgcagag cattctaggt aatgtagttt tcatagatcc cgaggtgggt    180
gccggggacc ctttgcacca acctcttgga gtaaaagcga agctccaggg cgctgggcga    240
tgagaaatgg cttatccaag tcctagggca gtggaggga                           279
```

<210> 8
<211> 402
<212> DNA
<213> Homo sapiens
<220>
<223> RIMS4_MA_9
<400> 8

```
ggagccccag cccatgaggg aaggagagga gagataaatg ggggcgctca aggcctgggg     60
cgccgggcag gggtcttggg cagggatcct ctggatgtgg ccaagacaaa gatggagagg    120
taaggtctgc gcgccacctc caatggcggg gggcgcgtcg gagccccagg ggtgggacgg    180
ccaaagccca gggcttgaag agtgggcaca ttcaggagac tcagggaggg tggcaggtcg    240
gctccaggga cgaggcaagg ggcctccaat aggcgcgggt gaggagggag atgggtcctg    300
gcgacccaaa gggcccacct gcgggaaagg tgaatgcaga caatctcggg gtccctgggg    360
gagaaggcca gaaggtagcg catcctggag accctggggt cc                       402
```

<210> 9
<211> 414
<212> DNA
<213> Homo sapiens
<220>
<223> TRH_MA_8
<400> 9

```
aacagatctc cagaggtggt gcagaaacga ccccgcgccg gcgccccatc ctgcggccag     60
tgcctccgcg ccccggctcc ggtccccacc gtccccgccc cagatttccg gaggagcagg    120
cgggcggggt cccgcggggc cggctgccgt cagcgcccct tcccggcggc cgcgacccct    180
ccccgctgac ctcactcgag ccgccgcctg gcgcagatat aagcggcggc ccatctgaag    240
agggctcggc aggcgcccgg ggtcctcagc gctgcagact cctgacctgc cgactgcgga    300
tcccgagtcc ccggatcccg gacccatcct gtggagccca ctcctggcag gtaaccgccc    360
caacccctct ccttccgcag acggtgtccg ggagcactgg aaagggagcc ctct          414
```

<210> 10
<211> 463

<212> DNA
<213> Homo sapiens
<220>
<223> ZNF540_MA_17
<400> 10

```
gggcagggca gaaccaggct aaagaaacgt ccagcgtagc ttcaaggatg caccgcgtga     60
tccctatcgg atctccccga cgcgtcaggc ctgcctagac ggtgctggga gcgcgtctcc    120
ttgaacgttg tcccgcctgg gattgcgagg taggtaccgc ctgcctgtgt gtaccggggc    180
tgctgtctcc ggggaggggc ttctggcgga caggagaacc aagcagcctc aggagctgcc    240
tgggtgtgtg tgtttctgtg cgagtgttgc atatctctgt gtgtgtttct gtgcaagtgt    300
tgcatgtctg tgtgtgtggg gggggtggtg tctggtgaaa agaatgtgtc tcgtgcggtg    360
gagcgccgtt tctctgtagt ccgcgggctc tcttatgcgc cctcttgtgg tcccaagtgt    420
gcttttcttg tttttcgctt tcttgggggt cattgatttc agc                      463
```

<210> 11
<211> 362
<212> DNA
<213> Homo sapiens
<220>
<223> PCDHAC1_MA_5
<400> 11

```
tggcagctcc tgggatacaa gagggtgcag gacagacttc aacccgcagc aggatccagc     60
gcggaaagct ctgcagcagg atccagcgcg gaaagccccc cgcagcactt ctttcggggg    120
gctcctgttt ccttaagcct agaaggtgtg gtcgctcacg ttcaccgtcc cgcctctcgc    180
cgcctccgct cggcagctcc acgctgagtc ccgccctctc cgccggagag gtgcgccggg    240
gtcagagcgc cgggacccga cgcgcggctc ccaaagggcg gcaggaagag cccagctggg    300
ctcagccaca gttatcagca atctgcgggc agaggatgtg gaggttaaga tctgggcagc    360
ct                                                                   362
```

<210> 12
<211> 264
<212> DNA
<213> Homo sapiens
<220>
<223> PRAC_MA_2
<400> 12

```
ggtgaaagcc tgctgctcac ccttcccttg tttcccaaaa cttctgaagg ctcccaaatt     60
cctgggagac cctctcccag ggcctcctga tgcagctacc atactgagcg atccgtcgat    120
aacgcccttg gcccaccgat cagtttacct tattagagag aaaagcactc ttggaggtag    180
taagatgggc cggtccttga tctgagaaat gggcgcacaa catcgctgtt ctctctgcaa    240
aggtggggac cagaatccag cttg                                           264
```

<210> 13
<211> 428
<212> DNA
<213> Homo sapiens
<220>
<223> ZNF671_MA_8
<400> 13

```
tgggacacag gggctgcagg cactttacga ttcggagtcg gagaaagggt gactgagggc     60
ccggaggacg cagcacccac ccgcgcggag tccgttagct ccgccatagg accgtgggcg    120
cggacagctg ccgggagcgg caggcgtctc gatcggggac gcaggcactt ccgtccctgc    180
agagcatcag acgcgtctcg ggacactggg gacaacatct cctccgcgct ttcccaacac    240
ctccacctgc ggcccacaca agcgttacag aaccccggcc agggacagcc tgacagaaac    300
aaaatgtccg ctacaaggag gagccggaag tcccgcccac gcaccccccg caggcactga    360
aacacccctc tcctgggccc tcattgggta tgcaacgtat aggtttgtgg gtagagtgtg    420
gttcccac                                                             428
```

<210> 14
<211> 348
<212> DNA
<213> Homo sapiens
<220>
<223> WNT3A_MA_9
<400> 14

```
gtccacctgg caatgagggg ctgctgtaga gagagttaag ggtgagttaa gcacggggtg     60
tgaggggctc caggaccctc aatcagaaag cgctgtgctg cgccctccac accagaaaag    120
gcgcgttccg tgagaccctc cccagcctgg cgatggaagt gcagataaac caaaggaagg    180
gtcccgaaag gtctctctca ggcctcccac ctccactgca catatcctgt gggaggggga    240
acggtggcca cactttcgcc agggcttgtg atccctcaga gccctcacca agcaaggatc    300
accccagttc cgaattaagg gcgctctgag atgcccaaga ttgaggaa                 348
```

<210> 15
<211> 422
<212> DNA
<213> Homo sapiens
<220>
<223> KHDRBS2_MA_19(rev)
<400> 15

```
cctggcacca ccaccaatga gtggttggcg gaggtgggcc gcgtcctgtt cccgcctctc     60
cagttaaggc cgctggtgtg agccggggct ctgcgcgagc gagggacgac ggaagggacg    120
ggcaggtgtg ggcgcggggc cacgcagccc gacggcggga gtcgcaggtg ctgggtgcat    180
gggccagtga ggacgcacag agatccctcg ccgcgcggag gaggagcagc gcgggagcca    240
ggcgctgccc caagaccctg cctgcgtccg agcgagcgga acctcgcgct tcgcccgggg    300
acaatccgaa gtccgcgcta tggaagagga gaaatatttg cctgagctga tggcagagaa    360
agatagcctg gatccatctt ttgtgcatgc gtcgcgcctt ttggcagaag gtaggacttg    420
cc                                                                   422
```

<210> 16
<211> 339
<212> DNA
<213> Homo sapiens
<220>
<223> ASCL2_MA_8
<400> 16

```
gctaacaaag ctgggttcct gctgggcccc gccctgctcc tcgcccccgc gactgggctg     60
ggcgcgctgt cccctagcgc agctatgtcc cgagcgcgcc cccacctgtg cgttaatcta    120
ctgggaatgg gggtggactg cgccttacct ggggcggggt ggggcttaag gagtggtcga    180
gactgaggcg gggtgggagg ttcaggttcc cggggcgcct tccccaaccc gccccgcttt    240
ccccgtccct ccacgcgcac cctgcctgtg gtttccgtgc gcccccggcc tgagggctct    300
gggcggcacc ttaacccgga gggcctggag gtctgcacc                           339
```

<210> 17
<211> 38

<212> DNA
<213> Artificial
<220>
<223> Artificially synthesized primer sequence (SLC13A5_MA_10 forward p rimer for using MassARRAY assay)
<400> 17
aggaagagag gaaggatttg aatttggaga tatagttt 38

<210> 18
<211> 56
<212> DNA
<213> Artificial
<220>
<223> Artificially synthesized primer sequence (SLC13A5_MA_10 reverse p rimer for using MassARRAY assay)
<400> 18
cagtaatacg actcactata gggagaaggc taaaaaaccc aaaaacctac aaaaaa 56

<210> 19
<211> 32
<212> DNA
<213> Artificial
<220>
<223> Artificially synthesized primer sequence (SLC13A5_MA_13 forward p rimer for using MassARRAY assay)
<400> 19
aggaagagag ttttttggg ttttgaaggg tt 32

<210> 20
<211> 57
<212> DNA
<213> Artificial
<220>
<223> Artificially synthesized primer sequence (SLC13A5_MA_13 reverse p rimer for using MassARRAY assay)
<400> 20
cagtaatacg actcactata gggagaaggc tttatatccc ttcctctcta aaactcc 57

<210> 21
<211> 32
<212> DNA
<213> Artificial
<220>
<223> Artificially synthesized primer sequence (SLC13A5_MA_15 forward p rimer for using MassARRAY assay)
<400> 21
aggaagagag tttttttgt tttaggggtt gt 32

<210> 22
<211> 55
<212> DNA
<213> Artificial
<220>
<223> Artificially synthesized primer sequence (SLC13A5_MA_15 reverse p rimer for using MassARRAY assay)
<400> 22
cagtaatacg actcactata gggagaaggc tccaccaaca taaataaaac tcccc 55

<210> 23
<211> 34
<212> DNA
<213> Artificial
<220>
<223> Artificially synthesized primer sequence (FAM150A_MA_14 forward p rimer for using MassARRAY assay)

<400> 23
aggaagagag gggaggattt agtagggtaa ttgt 34

<210> 24
<211> 56
<212> DNA
<213> Artificial
<220>
<223> Artificially synthesized primer sequence (FAM150A_MA_14 reverse p rimer for using MassARRAY assay)
<400> 24
cagtaatacg actcactata gggagaaggc ttttcaccta aaaaaacact aaaacc 56

<210> 25
<211> 36
<212> DNA
<213> Artificial
<220>
<223> Artificially synthesized primer sequence (GRM6_MA_8 forward prime r for using MassARRAY assay)
<400> 25
aggaagagag ggtttaggat aagtttgtga tagatg 36

<210> 26
<211> 56
<212> DNA
<213> Artificial
<220>
<223> Artificially synthesized primer sequence (GRM6_MA_8 reverse prime r for using MassARRAY assay)
<400> 26
cagtaatacg actcactata gggagaaggc taaaacaaaa aaacaaaccc aaaaat 56

<210> 27
<211> 33
<212> DNA
<213> Artificial
<220>
<223> Artificially synthesized primer sequence (ZFP42_MA_2 forward prim er for using MassARRAY assay)
<400> 27
aggaagagag gagttgatgg gtggttgtag ttt 33

<210> 28
<211> 60
<212> DNA
<213> Artificial
<220>
<223> Artificially synthesized primer sequence (ZNF154_MA_2 reverse pri mer for using MassARRAY assay)
<400> 28
cagtaatacg actcactata gggagaaggc tcccatttaa aaaaaattcc ataaaacaaa 60

<210> 29
<211> 40
<212> DNA
<213> Artificial
<220>
<223> Artificially synthesized primer sequence (ZNF154_MA_5 forward pri mer for using MassARRAY assay)
<400> 29
aggaagagag ggtgaatata ttttagagaa gttaaaatgg 40

<210> 30

<211> 56
<212> DNA
<213> Artificial

<220>
<223> Artificially synthesized primer sequence (ZNF154_MA_5 reverse pri mer for using MassARRAY assay)
<400> 30
cagtaatacg actcactata gggagaaggc ttccctccac taccctaaaa cttaaa 56

<210> 31
<211> 35
<212> DNA
<213> Artificial
<220>
<223> Artificially synthesized primer sequence (RIMS4_MA_9 forward prim er for using MassARRAY assay)
<400> 31
aggaagagag ggagttttag tttatgaggg aagga 35

<210> 32
<211> 54
<212> DNA
<213> Artificial
<220>
<223> Artificially synthesized primer sequence (RIMS4_MA_9 reverse prim er for using MassARRAY assay)
<400> 32
cagtaatacg actcactata gggagaaggc taaaccccaa aatctccaaa atac 54

<210> 33
<211> 37
<212> DNA
<213> Artificial
<220>
<223> Artificially synthesized primer sequence (TRH_MA_8 forward primer for using MassARRAY assay)
<400> 33
aggaagagag aatagatttt tagaggtggt gtagaaa 37

<210> 34
<211> 55
<212> DNA
<213> Artificial
<220>
<223> Artificially synthesized primer sequence (TRH_MA_8 reverse primer for using MassARRAY assay)
<400> 34
cagtaatacg actcactata gggagaaggc taaaaaactc cctttccaat actcc 55

<210> 35
<211> 37
<212> DNA
<213> Artificial
<220>
<223> Artificially synthesized primer sequence (ZNF540_MA_17 forward pr imer for using MassARRAY assay)
<400> 35
aggaagagag gggtagggta gaattaggtt aaagaaa 37

<210> 36
<211> 56
<212> DNA
<213> Artificial

<220>
<223> Artificially synthesized primer sequence (ZNF540_MA_17 reverse pr imer for using MassARRAY assay)
<400> 36
cagtaatacg actcactata gggagaaggc tactaaaatc aataaccccc aaaaaa 56

<210> 37
<211> 35
<212> DNA
<213> Artificial
<220>
<223> Artificially synthesized primer sequence (PCDHAC1_MA_5 forward pr imer for using MassARRAY assay)
<400> 37
aggaagagag tggtagtttt tgggatataa gaggg 35

<210> 38
<211> 56
<212> DNA
<213> Artificial
<220>
<223> Artificially synthesized primer sequence (PCDHAC1_MA_5 reverse pr imer for using MassARRAY assay)
<400> 38
cagtaatacg actcactata gggagaaggc taaactaccc aaatcttaac ctccac 56

<210> 39
<211> 37
<212> DNA
<213> Artificial
<220>
<223> Artificially synthesized primer sequence (PRAC_MA_2 forward prime r for using MassARRAY assay)
<400> 39
aggaagagag ggtgaaagtt tgttgtttat tttttt 37

<210> 40
<211> 56
<212> DNA
<213> Artificial
<220>
<223> Artificially synthesized primer sequence (PRAC_MA_2 reverse prime r for using MassARRAY assay)
<400> 40
cagtaatacg actcactata gggagaaggc tcaaactaaa ttctaatccc cacctt 56

<210> 41
<211> 35
<212> DNA
<213> Artificial
<220>
<223> Artificially synthesized primer sequence (ZNF671_MA_8 forward pri mer for using MassARRAY assay)
<400> 41
aggaagagag tgggatatag gggttgtagg tattt 35

<210> 42
<211> 56
<212> DNA
<213> Artificial
<220>
<223> Artificially synthesized primer sequence (ZNF671_MA_8 reverse pri mer for using MassARRAY assay)
<400> 42
cagtaatacg actcactata gggagaaggc tataaaaacc acactctacc cacaaa 56

<210> 43
<211> 35
<212> DNA
<213> Artificial
<220>
<223> Artificially synthesized primer sequence (WNT3A_MA_9 forward prim er for using MassARRAY assay)
<400> 43
aggaagagag gtttatttgg taatgagggg ttgtt 35

<210> 44
<211> 56
<212> DNA
<213> Artificial
<220>
<223> Artificially synthesized primer sequence (WNT3A_MA_9 reverse prim er for using MassARRAY assay)
<400> 44
cagtaatacg actcactata gggagaaggc tttcctcaat cttaaacatc tcaaaa 56

<210> 45
<211> 38
<212> DNA
<213> Artificial
<220>
<223> Artificially synthesized primer sequence (KHDRBS2_MA_19(rev) forw ard primer for using MassARRAY assay)
<400> 45
aggaagagag tttggtatta ttattaatga gtggttgg 38

<210> 46
<211> 57
<212> DNA
<213> Artificial
<220>
<223> Artificially synthesized primer sequence (KHDRBS2_MA_19(rev) reve rse primer for using MassARRAY assay)
<400> 46
cagtaatacg actcactata gggagaaggc taacaaatcc taccttctac caaaaaa 57

<210> 47
<211> 35
<212> DNA
<213> Artificial
<220>
<223> Artificially synthesized primer sequence (ASCL2_MA_8 forward prim er for using MassARRAY assay)
<400> 47
aggaagagag gttaataaag ttgggttttt gttgg 35

<210> 48
<211> 53
<212> DNA
<213> Artificial
<220>
<223> Artificially synthesized primer sequence (ASCL2_MA_8 reverse prim er for using MassARRAY assay)
<400> 48
cagtaatacg actcactata gggagaaggc taatacaaac ctccaaaccc tcc 53

<210> 49
<211> 23

<212> DNA
<213> Artificial
<220>
<223> Artificially synthesized primer sequence (ZFP42 forward primer fo r Pyroseqencing)
<400> 49
ggaggagttg atgggtggtt gta 23

<210> 50
<211> 28
<212> DNA
<213> Artificial
<220>
<223> Artificially synthesized primer sequence (ZFP42 reverse primer fo r Pyroseqencing)
<400> 50
cccaaacact ctactatttc caatacca 28

<210> 51
<211> 17
<212> DNA
<213> Artificial
<220>
<223> Artificially synthesized primer sequence (ZFP42 sequencing primer for Pyroseqencing)
<400> 51
gggtggttgt agtttga 17

<210> 52
<211> 29
<212> DNA
<213> Artificial
<220>
<223> Artificially synthesized primer sequence (ZNF154 forward primer f or Pyroseqencing)
<400> 52
ggaaagtagg ttttttgagt ttttattgg 29

<210> 53
<211> 29
<212> DNA
<213> Artificial
<220>
<223> Artificially synthesized primer sequence (ZNF154 reverse primer f or Pyroseqencing)
<400> 53
ccctaaaact taaataaacc atttctcat 29

<210> 54
<211> 21
<212> DNA
<213> Artificial
<220>
<223> Artificially synthesized primer sequence (ZNF154 sequencing prime r for Pyroseqencing)
<400> 54
tgagttttta ttggtttagt a 21

<210> 55
<211> 31
<212> DNA
<213> Artificial
<220>
<223> Artificially synthesized primer sequence (ZNF540 forward primer f or Pyroseqencing)

<400> 55
aggagtaggg tagggtagaa ttaggttaaa g 31

<210> 56
<211> 32
<212> DNA
<213> Artificial
<220>
<223> Artificially synthesized primer sequence (ZNF540 reverse primer f or Pyroseqencing)
<400> 56
acccaaacaa ctcctaaaac tacttaattc tc 32

<210> 57
<211> 22
<212> DNA
<213> Artificial
<220>
<223> Artificially synthesized primer sequence (ZNF540 sequencing prime r for Pyroseqencing)
<400> 57
ggtagggtag aattaggtta aa 22